# EUROPEAN PATENT APPLICATION

(11) **EP 4 640 697 A1**
(43) Date of publication of application: **29.10.2025**
(21) Application number: 23906072.6
(22) Date of filing: 22.12.2023
(51) Int. Cl.: C07K 14/47, C12N 15/62, A61K 38/00

(54) **COMPLEX AND USE THEREOF**

(30) Priority: 23.12.2022 CN 202211663018; 02.11.2023 CN 202311453875
(71) Applicant: Epigenic Therapeutics Pte. Ltd., Singapore 068908 (SG)
(72) Inventor: MAO, Shaoshuai, Shanghai 200131 (CN); ZANG, Ying, Shanghai 200131 (CN); WEI, Xiang, Shanghai 200131 (CN); LUO, Hao, Shanghai 200131 (CN)
(74) Representative: Murgitroyd & Company
(86) International application number: PCT/CN2023/140808
(87) International publication number: WO 2024/131917

(57) **Abstract**

A complex, comprising a first fusion and a second fusion, wherein one of the first fusion and the second fusion comprises a DNA methylation domain and at least one recruitment domain A, and the other fusion comprises a transcriptional repressor domain and at least one recruitment domain A'; and the recruitment domain A and the recruitment domain A' interact with each other, so that one of the first fusion and the second fusion or a part thereof is recruited to the vicinity of the other fusion. Also provided are use of the complex in the preparation of a product for inhibiting target gene expression and the preparation of a drug.

## Description

### TECHNICAL FIELD

The present application relates to the field of biomedicine, and specifically to a complex for gene editing and uses thereof.

### BACKGROUND

Abnormalities in genomic epigenetic modifications are closely related to the occurrence and development of many diseases, such as metabolic disorders, cardiovascular diseases and cancer that are common in life. Gene epigenetic editing tools can achieve the purpose of corresponding gene transcriptional regulation without changing the gene sequence. This process will not cause permanent DNA damage, nor will it produce deleterious mutations and off-target effects; Moreover, epigenetic therapy can provide better therapeutic effects by regulating the activities of multiple genes at the same time, compensate for the shortcomings of gene therapy and provide new hope for treating such diseases. On the other hand, the development of gene site-directed modification technology makes it possible to carry out targeted epigenome editing and transcriptional regulation in a natural chromatin environment, especially the development of CRISPR/Cas9 technology.

Currently, the design of epigenetic modification and transcriptional regulation is mainly based on the combination of nucleases, among which the most effective is the combination of engineered dead nuclease (dCas9). Its design principle is to fuse various epigenetic regulatory effectors to dCas9, and realize the editing of the epigenome at specific genomic sites through the targeting of the nuclease in combination with the characteristics of the target DNA. These trans-regulatory domains and proteins function by hindering RNA polymerase binding to the dCas9 targeting sites in the promoter region or recruiting endogenous transcription complexes. A recent breakthrough in this field is to use a dCas-SunTag system to fuse multiple copies of a transcriptional activating or inhibitory protein to achieve the regulation of target gene transcription. For example, Hatada Izuho et al. (PCT/JP2021/006498) used a dCas9-GCN4 fusion to recruit fusions containing epigenetic modifiers (such as methyltransferase and histone acetyltransferase), transcriptional inhibitory regulators (such as ZIM3) and antibodies, thereby inhibiting the expression of the target gene regulated by it. Currently, the researches on the in vivo treatment of diseases caused by epigenetic abnormalities using transcriptional regulation and epigenetic site-directed modification technologies are still limited, and there are certain problems with the existing editing tools, such as the unsatisfactory recruitment effect of SunTag system, the low efficiency of transcriptional regulation, and the limited range of modification of target genes (such as methylation modification).

### SUMMARY

The present application provides a complex for gene editing and its encoding nucleic acid, vector, composition, cell and other forms. Compared with the existing gene editing methods based on SunTag recruitment strategy, the complex can achieve higher regulation efficiency and wider modification and regulation range of target genes. In addition, the complex of the present application can be used in the manufacture of a product for inhibiting expression of a target gene and a medicament.

In one aspect, the present application provides a complex comprising a first fusion and a second fusion, wherein the fusion of one of the first fusion and the second fusion comprises a DNA methylation domain and at least one recruitment domain A, and wherein the other fusion comprises a transcriptional repressor domain and at least one recruitment domain A'; wherein the recruitment domain A and the recruitment domain A' are capable of interacting, such that the fusion of one of the first fusion and the second fusion or a portion thereof is capable of being recruited to the vicinity of the other fusion.

In some embodiments, the first fusion or the second fusion comprises a nucleic acid binding domain.

In some embodiments, the first fusion comprises a DNA methylation domain, a nucleic acid binding domain and at least one recruitment domain A, and the second fusion comprises a transcriptional repressor domain and at least one recruitment domain A'.

In some embodiments, the first fusion comprises, in order from N-terminus to C-terminus, a DNA methylation domain, a nucleic acid binding domain, and a recruitment domain A.

In some embodiments, the second fusion comprises, in order from N-terminus to C-terminus, a transcriptional repressor domain and a recruitment domain A', or comprises, in order from N-terminus to C-terminus, a recruitment domain A' and a transcriptional repressor domain.

In some embodiments, the first fusion comprises a transcriptional repressor domain, a nucleic acid binding domain and at least one recruitment domain A, and the second fusion comprises a DNA methylation domain and at least one recruitment domain A'.

In some embodiments, the first fusion comprises, in order from N-terminus to C-terminus, a recruitment domain A, a nucleic acid binding domain and a transcriptional repressor domain.

In some embodiments, the second fusion comprises, in order from N-terminus to C-terminus, a DNA methylation domain and a recruitment domain A', or comprises, in order from N-terminus to C-terminus, a recruitment domain A' and a DNA methylation domain.

In some embodiments, the complex is characterized in that: 1) the first fusion comprises, in order from N-terminus to C-terminus, a DNA methylation domain, a nucleic acid binding domain and a recruitment domain A, and the second fusion comprises, in order from N-terminus to C-terminus, a transcriptional repressor domain and a recruitment domain A'; or 2) the first fusion comprises, in order from N-terminus to C-terminus, a DNA methylation domain, a nucleic acid binding domain and a recruitment domain A, and the second fusion comprises, in order from N-terminus to C-terminus, a recruitment domain A' and a transcriptional repressor domain; or 3) the first fusion comprises, in order from N-terminus to C-terminus, a recruitment domain A, a nucleic acid binding domain and a transcriptional repressor domain, and the second fusion comprises, in order from N-terminus to C-terminus, a DNA methylation domain and a recruitment domain A'; or 4) the first fusion comprises, in order from N-terminus to C-terminus, a recruitment domain A, a nucleic acid binding domain and a transcriptional repressor domain, and the second fusion comprises, in order from N-terminus to C-terminus, a recruitment domain A' and a DNA methylation domain.

In some embodiments, the nucleic acid binding domain is a DNA binding domain.

In some embodiments, the DNA binding domain is selected from: a TALE domain, a zinc finger domain, a tetR domain, a meganuclease, a Cas protein, an Argonaute (Ago) protein, and a homolog, a modified form or a variant thereof.

In some embodiments, the DNA binding domain is capable of binding to a target sequence of a target locus.

In some embodiments, the DNA binding domain is capable of binding to a guide RNA.

In some embodiments, the guide RNA is capable of specifically recognizing and hybridizing to the target sequence of the target locus.

In some embodiments, the DNA binding domain is a Cas protein, and the Cas protein is a class II Cas nuclease.

In some embodiments, the Cas protein is selected from a class II type II Cas nuclease and a class II type V Cas nuclease.

In some embodiments, the Cas protein is a Cas9 or Cas12 protein.

In some embodiments, the Cas protein is a dead Cas9 (dCas9) protein or a dead Cas12 (dCas12) protein.

In some embodiments, the DNA binding domain comprises an amino acid sequence as set forth in any one of SEQ ID NOs: 1-9, 343 and 344.

In some embodiments, the recruitment domain A is selected from any one of one of the following two sets of domains, and the recruitment domain A' is selected from any one of the other of the following two sets of domains: 1) a general control nonderepressible protein 4 (GCN4), a GFP11 fragment derived from split green fluorescent protein (GFP), and a GVKESLV polypeptide; and 2) a single chain antibody (scFv), a GFP1-10 fragment derived from split green fluorescent protein (GFP) and a PDZ protein domain.

In some embodiments, in the complex: 1) the domain of one of the recruitment domain A and the recruitment domain A' is a GCN4, and wherein the other domain is a scFv; or 2) the domain of one of the recruitment domain A and the recruitment domain A' is a GFP11 fragment, and wherein the other domain is a GFP1-10; or 3) the domain of one of the recruitment domain A and the recruitment domain A' is a GVKESLV, and wherein the other domain is a PDZ protein domain.

In some embodiments, the DNA methylation domain comprises at least one DNA methyltransferase or a functionally active fragment thereof.

In some embodiments, the DNA methyltransferase is selected from DNMT3A, DNMT3B, DNMT3c, DNMT1, DNMT2 and DNMT3L.

In some embodiments, the DNA methylation domain comprises at least one DNMT3A and at least one DNMT3L.

In some embodiments, the DNA methyltransferase comprises an amino acid sequence as set forth in any one of SEQ ID NOs: 19-24.

In some embodiments, the DNA methylation domain comprises a DNMT3A-DNMT3L domain or a DNMT3L-DNMT3A domain; wherein, - indicates that the domains at both ends thereof are directly or indirectly linked in order from N-terminus to C-terminus.

In some embodiments, the transcriptional repressor is selected from one or more of the following domains: KRAB, ZIM3, ZNF680, ZNF554, ZNF264, ZNF582, ZNF324, ZNF669, ZNF354A, ZNF82, ZNF595, ZNF419, ZNF566, ZIM2, EHMT2, SUV39H1, ZFPM1, TRIM28, EZH2, MXD1, SID, LSD1, HP1a, HDAC3, HDAC1, PRMT1, SETDB1, hSIRT1, ZNF436, ZNF257, ZNF675, ZNF490, ZNF320, ZNF331, ZNF816, ZNF41, ZNF189, ZNF528, ZNF543, ZNF140, ZNF610, ZNF350, ZNF8, ZNF30, ZNF98, ZNF677, ZNF596, ZNF214, ZNF37A, ZNF34, ZNF250, ZNF547, ZNF273, ZFP82, ZNF224, ZNF33A, ZNF45, ZNF175, ZNF184, ZFP28-1, ZFP28-2, ZNF18, ZNF213, ZNF394, ZFP1, ZFP14, ZNF416, ZNF557, ZNF729, ZNF254, ZNF764, ZNF785, ZNF10, CBX5, RYBP, YAF2, MGA, CBX1, SCMH1, MPP8, SUMO3, HERC2, BIN1, PCGF2, TOX, FOXA1, FOXA2, IRF2BP1, IRF2BP2, IRF2BPL IRF-2BP1_2 N-terminal domain, HOXA13, HOXB13, HOXC13, HOXA11, HOXC11, HOXC10, HOXA10, HOXB9, HOXA9, ZFP28, ZN334, ZN568, ZN37A, ZN181, ZN510, ZN862, ZN140, ZN208, ZN248, ZN571, ZN699, ZN726, ZIK1, ZNF2, Z705F, ZNF14, ZN471, ZN624, ZNF84, ZNF7, ZN891, ZN337, Z705G, ZN529, ZN729, ZN419, Z705A, ZN302, ZN486, ZN621, ZN688, ZN33A, ZN554, ZN878, ZN772, ZN224, ZN184, ZN544, ZNF57, ZN283, ZN549, ZN211, ZN615, ZN253, ZN226, ZN730, Z585A, ZN732, ZN681, ZN667, ZN649, ZN470, ZN484, ZN431, ZN382, ZN254, ZN124, ZN607, ZN317, ZN620, ZN141, ZN584, ZN540, ZN75D, ZN555, ZN658, ZN684, RBAK, ZN829, ZN582, ZN112, ZN716, HKR1, ZN350, ZN480, ZN416, ZNF92, ZN100, ZN736, ZNF74, ZN443, ZN195, ZN530, ZN782, ZN791, ZN331, Z354C, ZN157, ZN727, ZN550, ZN793, ZN235, ZN724, ZN573, ZN577, ZN789, ZN718, ZN300, ZN383, ZN429, ZN677, ZN850, ZN454, ZN257, ZN264, ZN485, ZN737, ZNF44, ZN596, ZN565, ZN543, ZFP69, SUMO1, ZNF12, ZN169, ZN433, ZN175, ZN347, ZNF25, ZN519, Z585B, ZN517, ZN846, ZN230, ZNF66, ZN713, ZN816, ZN426, ZN674, ZN627, ZNF20, Z587B, ZN316, ZN233, ZN611, ZN556, ZN234, ZN560, ZNF77, ZN682, ZN614, ZN785, ZN445, ZFP30, ZN225, ZN551, ZN610, ZN528, ZN284, ZN418, ZN490, ZN805, Z780B, ZN763, ZN285, ZNF85, ZN223, ZNF90, ZN557, ZN425, ZN229, ZN606, ZN155, ZN222, ZN442, ZNF91, ZN135, ZN778, ZN534, ZN586, ZN567, ZN440, ZN583, ZN441, ZNF43, ZN589, ZN563, ZN561, ZN136, ZN630, ZN527, ZN333, Z324B, ZN786, ZN709, ZN792, ZN599, ZN613, ZF69B, ZN799, ZN569, ZN564, ZN546, ZFP92, ZN723, ZN439, ZFP57, ZNF19, ZN404, ZN274, CBX3, ZN250, ZN570, ZN675, ZN695, ZN548, ZN132, ZN738, ZN420, ZN626, ZN559, ZN460, ZN268, ZN304, ZN605, ZN844, SUMO5, ZN101, ZN783, ZN417, ZN182, ZN823, ZN177, ZN197, ZN717, ZN669, ZN256, ZN251, CBX4, CDY2, CDYL2, ZN562, ZN461, Z324A, ZN766, ID2, ZN214, CBX7, ID1, CREM, SCX, ASCL1, ZN764, SCML2, TWST1, CREB1, TERF1, ID3, CBX8, GSX1, NKX22, ATF1, TWST2, ZNF17, TOX3, TOX4, ZMYM3, I2BP1, RHXF1, SSX2, I2BPL, ZN680, TRI68, HXA13, PHC3, TCF24, HXB13, HEY1, PHC2, ZNF81, FIGLA, SAM11, KMT2B, HEY2, JDP2, HXC13, ASCL4, HHEX, GSX2, ETV7, ASCL3, PHC1, OTP, I2BP2, VGLL2, HXA11, PDLI4, ASCL2, CDX4, ZN860, LMBL4, PDIP3, NKX25, CEBPB, ISL1, CDX2, PROP1, SIN3B, SMBT1, HXC11, HXC10, PRS6A, VSX1, NKX23, MTG16, HMX3, HMX1, KIF22, CSTF2, CEBPE, DLX2, PPARG, PRIC1, UNC4, BARX2, ALX3, TCF15, TERA, VSX2, HXD12, CDX1, TCF23, ALX1, HXA10, RX, CXXC5, SCML1, NFIL3, DLX6, MTG8, CEBPD, SEC13, FIP1, ALX4, LHX3, PRIC2, MAGI3, NELL1, PRRX1, MTG8R, RAX2, DLX3, DLX1, NKX26, NAB1, SAMD7, PITX3, WDR5, MEOX2, NAB2, DHX8, CBX6, EMX2, CPSF6, HXC12, KDM4B, LMBL3, PHX2A, EMX1, NC2B, DLX4, SRY, ZN777, ZN398, GATA3, BSH, SF3B4, TEAD1, TEAD3, RGAP1, PHF1, GATA2, FOXO3, ZN212, IRX4, ZBED6, LHX4, SIN3A, RBBP7, NKX61, R51A1, MB3L1, DLX5, NOTC1, TERF2, ZN282, RGS12, ZN840, SPI2B, PAX7, NKX62, ASXL2, FOXO1, GATA1, ZMYM5, LRP1, MIXL1, SGT1, LMCD1, CEBPA, SOX14, WTIP, PRP19, NKX11, RBBP4, DMRT2, SMCA2, and functionally active fragments thereof.

In some embodiments, the transcriptional repressor domain comprises an amino acid sequence as set forth in any one of SEQ ID NOs: 25-50.

In some embodiments, in the complex: 1) the fusion of one of the first fusion and the second fusion comprises a DNA methylation domain-dCas9 or dCas12 or TALE-n×GCN4, and wherein the other fusion comprises a transcriptional repressor domain-scFv; or 2) the fusion of one of the first fusion and the second fusion comprises a DNA methylation domain-dCas9 or dCas12 or TALE-scFv, and wherein the other fusion comprises a transcriptional repressor domain-GCN4; or 3) the fusion of one of the first fusion and the second fusion comprises a DNA methylation domain-dCas9 or dCas12 or TALE-n×GFP11, and wherein the other fusion comprises a transcriptional repressor domain-GFP1-10; or 4) the fusion of one of the first fusion and the second fusion comprises a DNA methylation domain-dCas9 or dCas12 or TALE-GFP1-10, and wherein the other fusion comprises a transcriptional repressor domain-GFP11; or 5) the fusion of one of the first fusion and the second fusion comprises a DNA methylation domain-dCas9 or dCas12 or TALEn×GCN4, and wherein the other fusion comprises a scFv-transcriptional repressor domain; or 6) the fusion of one of the first fusion and the second fusion comprises a DNA methylation domain-dCas9 or dCas12 or TALE-scFv, and wherein the other fusion comprises a GCN4-transcriptional repressor domain; or 7) the fusion of one of the first fusion and the second fusion comprises a DNA methylation domain-dCas9 or dCas12 or TALE-n×GFP11, and wherein the other fusion comprises a GFP1-10-transcriptional repressor domain; or 8) the fusion of one of the first fusion and the second fusion comprises a DNA methylation domain-dCas9 or dCas12 or TALE-GFP1-10, and wherein the other fusion comprises a GFP11-transcriptional repressor domain; wherein - indicates that the domains at both ends thereof are directly or indirectly linked in order from N-terminus to C-terminus; n×GCN4 or n×GFP11 denotes respectively n copies of GCN4 or n copies of GFP11 linked by a linker sequence, and n is any integer selected from 1 to 20.

In some embodiments, the first fusion and/or the second fusion comprises an amino acid sequence as set forth in any one of SEQ ID NOs: 51-76, 78-82, 85-93, 103-105, 110-115, 123, 124, 361 and 362.

In some embodiments, the complex comprises an amino acid sequence as set forth in any one of SEQ ID NOs: 133-142, 153, 154, 158-163, 168, 345 and 346.

In some embodiments, in the complex: 1) the fusion of one of the first fusion and the second fusion comprises an n×GCN4-dCas9 or dCas12 or TALE-transcriptional repressor domain, and wherein the other fusion comprises a DNA methylation domain-scFv; or 2) the fusion of one of the first fusion and the second fusion comprises a scFv-dCas9 or dCas12 or TALE-transcriptional repressor domain, and wherein the other fusion comprises a DNA methylation domain-GCN4; or 3) the fusion of one of the first fusion and the second fusion comprises an n×GFP11-dCas9 or dCas12 or TALE-transcriptional repressor domain, and wherein the other fusion comprises a DNA methylation domain-GFP1-10; or 4) the fusion of one of the first fusion and the second fusion comprises a GFP1-10-dCas9 or dCas12 or TALE-transcriptional repressor domain, and wherein the other fusion comprises a DNA methylation domain-GFP 11; or 5) the fusion of one of the first fusion and the second fusion comprises an n×GCN4-dCas9 or dCas12 or TALE-transcriptional repressor domain, and wherein the other fusion comprises a scFv-DNA methylation domain; or 6) the fusion of one of the first fusion and the second fusion comprises a scFv-dCas9 or dCas12 or TALE-transcriptional repressor domain, and wherein the other fusion comprises a GCN4-DNA methylation domain; or 7) the fusion of one of the first fusion and the second fusion comprises an n×GFP11-dCas9 or dCas12 or TALE-transcriptional repressor domain, and wherein the other fusion comprises a GFP1-10-DNA methylation domain; or 8) the fusion of one of the first fusion and the second fusion comprises a GFP1-10-dCas9 or dCas12 or TALE-transcriptional repressor domain, and wherein the other fusion comprises a GFP11-DNA methylation domain; wherein - indicates that the domains at both ends thereof are directly or indirectly linked in order from N-terminus to C-terminus; n×GCN4 or n×GFP11 denotes respectively n copies of GCN4 or n copies of GFP11 linked by a linker sequence, and n is any integer selected from 1 to 20.

In some embodiments, the first fusion and/or the second fusion comprises an amino acid sequence as set forth in any one of SEQ ID NOs: 83, 84, 94-102, 106-109, 116-122, 363 and 364.

In some embodiments, the complex comprises an amino acid sequence as set forth in any one of SEQ ID NOs: 143-152, 155-157, 164-167, 347 and 348.

In some embodiments, the complex further comprises a nuclear localization signal and/or a label domain.

In another aspect, the present application provides a nucleic acid encoding the complex described in the present application.

In some embodiments, the nucleic acid is a recombinant vector.

In some embodiments, the recombinant vector further comprises a non-coding region.

In some embodiments, the non-coding region is selected from an intron, a regulatory element, a promoter, an enhancer, a termination sequence, as well as a 5' untranslated region and a 3' untranslated region.

In some embodiments, the nucleic acid comprises a first nucleic acid fragment encoding the first fusion and a second nucleic acid fragment encoding the second fusion.

In some embodiments, the first nucleic acid fragment and the second nucleic acid fragment are linked by a nucleic acid fragment encoding a cleavage peptide.

In some embodiments, the cleavage peptide is a 2A peptide and/or an IRES.

In some embodiments, the 2A peptide is selected from P2A, T2A, E2A and F2A.

In some embodiments, the nucleic acid comprises a nucleic acid sequence as set forth in any one of SEQ ID NOs: 169-335 and 349-360.

In another aspect, the present application provides a delivery vector, comprising the complex described in the present application and/or the nucleic acid described in the present application, and optionally a liposome and/or a lipid nanoparticle.

**In** another aspect, the present application provides a composition comprising the complex described in the present application, the nucleic acid described in the present application, and/or the delivery vector described in the present application.

**In** another aspect, the present application provides a cell comprising the complex described in the present application, the nucleic acid described in the present application, the delivery vector described in the present application, and/or the composition described in the present application.

**In** another aspect, the present application provides a kit comprising the complex described in the present application, the nucleic acid described in the present application, the delivery vector described in the present application, the composition described in the present application, and/or the cell described in the present application.

**In** another aspect, the present application provides a method for regulating target gene expression, comprising administering the complex described in the present application, the nucleic acid described in the present application, the delivery vector described in the present application, the composition described in the present application, the cell described in the present application, and/or the kit described in the present application.

**In** some embodiments, the method comprises introducing the complex, the nucleic acid, the delivery vector, the composition, the cell, and/or the kit into a cell containing the target gene.

**In** some embodiments, the method comprises contacting the complex, the nucleic acid, the delivery vector, and/or the composition with a regulatory element near and/or of the target gene.

**In** some embodiments, the regulatory element comprises a core promoter, a proximal promoter, a distal enhancer, a silencer, an insulator element, a boundary element, and/or a locus control region.

**In** another aspect, the present application provides a method for treating or alleviating a disease or a disorder thereof associated with abnormal target gene expression and/or abnormal target gene activity, wherein the method comprises administering to a subject in need thereof an effective amount of the complex described in the present application, the nucleic acid described in the present application, the delivery vector described in the present application, the composition described in the present application, the cell described in the present application, and/or the kit described in the present application.

In another aspect, the present application provides the use of the complex described in the present application, the nucleic acid described in the present application, the delivery vector described in the present application, the composition described in the present application, the cell described in the present application, and/or the kit described in the present application in the manufacture of a medicament for treating or alleviating a disease or a disorder thereof associated with abnormal target gene expression and/or abnormal target gene activity.

In another aspect, the present application provides the complex described in the present application, the nucleic acid described in the present application, the delivery vector described in the present application, the composition described in the present application, the cell described in the present application or the kit described in the present application, which is used for treating or alleviating a disease or a disorder thereof associated with abnormal target gene expression and/or abnormal target gene activity.

Those skilled in the art can easily discern other aspects and advantages of the present application from the following detailed description. Only exemplary embodiments of the present application are shown and described in the following detailed description. As those skilled in the art will recognize, the teachings of the present application enable those skilled in the art to make changes in the specific embodiments disclosed without departing from the spirit and scope of the invention to which the present application relates. Accordingly, the drawings and descriptions in the specification of the present application are illustrative only and not restrictive.

### BRIEF DESCRIPTION OF DRAWINGS

The specific features of the invention to which the present application relates are set forth in the appended claims. The features and advantages of the invention to which the present application relates can be better understood by referring to the exemplary embodiments described in detail below and the accompanying drawings. A brief description of the accompanying drawings is as follows:
FIG. 1 shows a schematic structural view of a complex of the present application.
FIG. 2 shows the inhibitory effect of the complex of the present application on PTP1b gene expression.
FIGs. 3 and 4 show the inhibitory effect of the complex of the present application on PCSK9 gene expression.

### DETAILED DESCRIPTION

The embodiments of the invention of the present application will be described below with specific examples. Those skilled in the art may easily understand other advantages and effects of the invention of the present application from the disclosure of the specification.

### Term Definitions

In the present application, the term "recruitment" generally refers to the recruitment between protein molecules, which specifically refers to the recruitment of other molecules by proteins to perform specific biological functions. This recruitment mainly depends on the affinity of intermolecular interactions, and usually its affinity is considered to be related to the spatial structure of protein molecules and is relatively complicated. The interaction mechanism may illustratively include, but is not limited to, hydrogen bonding, ionic interaction, hydrophobic interaction, van der Waals force and other non-covalent bonding. For example, some proteins can recruit enzymes to catalyze chemical reactions, or recruit other proteins to form complexes. These recruitments are crucial for many cellular processes, such as signal transduction, DNA replication, and gene expression, among others.

In the present application, the term "nucleic acid binding domain" generally refers to a portion of a polypeptide or composition that is capable of binding to a specific nucleic acid, which may comprise a region that contacts a nucleic acid, a nucleic acid and/or a protein substance. Examples of nucleic acid binding domains may include, but are not limited to, a helix-turn-helix domain, a zinc finger domain, a leucine zipper (bZIP) domain, a winged helix domain, a winged helix-turn-helix domain, a helix-loop-helix domain, an HMG-box domain, a Wor3 domain, an immunoglobulin domain, a B3 domain, a TALE domain, and/or a CRISPR/CasX protein, and other domains.

In the present application, the term "DNA binding domain" generally refers to an independently folded protein domain containing at least one motif that recognizes double-stranded or single-stranded DNA. For example, the DNA binding domain may recognize a specific DNA sequence (recognition or regulatory sequence) or have a general affinity for DNA. In certain instances, other domains of the DNA binding domain often modulate the activity of the DNA binding domain; the DNA binding function may be structural or involve transcriptional regulation, and sometimes the two functions overlap. In certain embodiments of the methods and gene expression regulating molecules provided according to the present application, the DNA binding domain may comprise a (DNA) nuclease, such as a nuclease capable of targeting DNA in a sequence-specific manner or capable of being directed or instructed to target DNA in a sequence-specific manner, such as a CRISPR-Cas system, a zinc finger nuclease (ZFN), a transcription activator-like effector nuclease (TALEN), or a meganuclease. In some embodiments, the DNA binding domain is a DNA nuclease derived from the CRISPR-Cas system. For example, the DNA nuclease derived from the CRISPR-Cas system is a Cas protein.

In the present application, the term "TALE DNA binding domain" or "TALE" is a polypeptide comprising one or more TALE repeat domains/units. A naturally occurring TALE or a "wild-type TALE" is a nucleic acid binding protein secreted by numerous species of proteobacteria. A TALE polypeptide contains a nucleic acid binding domain composed of tandem repeats of highly conserved monomeric polypeptides that are predominantly 33, 34 or 35 amino acids in length and differ from each other predominantly at amino acid positions 12 and 13. In preferred embodiments, the nucleic acid is a DNA. As used herein, a polypeptide monomer of TALE is used to refer to a highly conserved repetitive polypeptide sequence within the TALE nucleic acid binding domain, and the term "repetitive variable diresidue" or "RVD" is used to refer to amino acids that are highly variable at positions 12 and 13 of the polypeptide monomer. A general representation of the TALE monomer contained within the DNA binding domain is X₁₋₁₁-(X₁₂X₁₃)-X_{14-33 or 34 or 35}, wherein the subscript indicates the amino acid position, and X denotes any amino acid. X₁₂X₁₃ indicates an RVD. In some TALE polypeptide monomers, the variable amino acid at position 13 is deleted or absent, and in such monomers, the RVD is composed of a single amino acid. In such cases, the RVD may alternatively be represented as X*, wherein X denotes X₁₂, and (*) indicates that X₁₃ is absent. The DNA binding domain comprises several repeats of the TALE monomer, and this can be represented as (X₁₋₁₁-(X₁₂X₁₃)-X_{14-33 or 34 or 35})_{z}, wherein in preferred embodiments, z is at least 5-40. In a further preferred embodiment, z is at least 10-26.

The TALE monomer has a nucleotide binding affinity determined by the type of amino acid within its RVD. For example, a polypeptide monomer with an RVD of NI preferentially binds to adenine (A), a polypeptide monomer with an RVD of NG preferentially binds to thymine (T), a polypeptide monomer with an RVD of HD preferentially binds to cytosine (C), and a monomer with an RVD of NN preferentially bind to adenine (A) and guanine (G). In some other embodiments, a monomer with an RVD of IG preferentially binds to T. Thus, the number and order of polypeptide monomer repeats in the nucleic acid binding domain of TALE determines its nucleic acid target specificity. In a further embodiment of the present application, a monomer with an RVD of NS recognizes all four base pairs, and can bind to A, T, G or C. The structure and function of TALEs are further described, e.g., in Moscou et al., Science 326: 1501 (2009); Boch et al., Science 326: 1509-1512 (2009); and Zhang et al., Nature Biotechnology 29: 149-153 (2011), each of which is incorporated by reference in its entirety. The repeat domain of TALE is involved in the binding of TALE to its cognate target DNA sequence. These repeat units (or "repeat sequences") exhibit at least some sequence homology to other TALE repeat sequences within naturally occurring TALE proteins. See, e.g., U.S. Patent Publication No. 20110301073. The TALE binding domain involved in the present application can be "engineered" to bind to a predetermined nucleotide sequence, for example, via engineering (altering one or more amino acids) of a recognition helix region of a naturally occurring TALE protein. Thus, an engineered DNA binding protein (TALE) is a non-naturally occurring protein. Non-limiting examples of methods for engineering DNA binding proteins are design and selection. The designed DNA binding proteins are non-naturally occurring proteins whose design and/or composition results primarily from rational criteria. Rational design criteria include the application of replacement rules and computational algorithms for processing information in an information database that stores existing TALE design and binding data. See, e.g., U.S. Patents 6,140,081; 6,453,242; and 6,534,261; see also WO 98/53058; WO 98/53059; WO 98/53060; WO02/016536 and WO 03/016496, and U.S. Publication No. 20110301073.

In the present application, "Cas enzyme" is used interchangeably with "Cas protein", "CRISPR protein", "CRISPR enzyme", "CRISPR-Cas protein", "CRISPR-Cas enzyme", "Cas", "CRISPR effector" or "Cas effector protein", and generally refers to a class of enzymes that are complementary to a CRISPR sequence and are capable of using the CRISPR sequence as a guide to recognize and cleave a specific DNA strand. Non-limiting examples of Cas proteins include: Cas1, Cas1B, Cas2, Cas3, Cas4, Cas5, Cas6, Cas7, Cas8, Cas9 (also known as Csn1 and Csx12), Cas10, Csy1, Csy2, Csy3, Cse1, Cse2, Csc1, Csc2, Csa5, Csn2, Csm2, Csm3, Csm4, Csm5, Csm6, Cmr1, Cmr3, Cmr4, Cmr5, Cmr6, Csb1, Csb2, Csb3, Csx17, Csx14, Csx10, Csx16, CsaX, Csx3, Csx1, Csx15, Csf1, Csf2, Csf3, Csf4, and/or homologs thereof, or modified forms thereof. These proteins are known, for example, the amino acid sequence of S. pyogenes Cas9 protein can be found in SwissProt database under accession number Q99ZW2.

In the present application, the term "class II Cas nuclease" generally refers to a class of Cas proteins that perform recognition and/or cleavage functions in the form of a single protein, as defined by the updated classification scheme for CRISPR/Cas loci (Makarova et al., (2015) Nat Rev Microbiol, 13(11):722-36; Shmakov et al., (2015) Mol Cell, 60:385-397).

In the present application, the term "class II type II Cas nuclease and class II type V Cas nuclease" generally refer to monoproteinous, RNA-guided endonucleases among class II Cas nucleases. Herein, type V-B Cas nuclease in types II and V requires tracrRNA (trans-activating CRISPR RNA) and crRNA (CRISPR RNA) to work together to function normally, and crRNA and tracrRNA can be artificially combined into a single guide RNA (sgRNA); type V-A Cas nuclease in type V requires the use of crRNA alone to perform the guide function. Non-limiting examples of class II type II Cas nucleases include Cas9 and its family-related nucleases, and non-limiting examples of class II type V Cas nucleases include Cas12a (also known as Cpf1), Cas12b (also known as C2c1), Cas12c (also known as C2c3), Cas12d (CasY), Cas12e (CasX), Cas12g, Cas12h, Cas12i, C2c1, C2c4, C2c5, C2c8, C2c9, C2c10, Cas14a, Cas14b, Cas14c nucleases and/or TnpB.

In the present application, the term "dCas" may refer to a dCas protein or a fragment thereof. For example, as used herein, "dCas9" may refer to a dCas9 protein or a fragment thereof. As used herein, the terms "iCas" and "dCas" are used interchangeably to refer to a catalytically inactive CRISPR-associated protein. In one embodiment, the dCas protein comprises one or more mutations in DNA cleavage domain. In one embodiment, the dCas protein comprises one or more mutations in RuvC or domain. In one embodiment, the dCas molecule comprises one or more mutations in both RuvC and HNH domains. In one embodiment, the dCas protein is a fragment of a wild-type Cas protein. In one embodiment, the dCas protein comprises a functional domain from a wild-type Cas protein, wherein the functional domain is selected from a Reel domain, a bridge helix domain, or a PAM interaction domain. In one embodiment, the nuclease activity of dCas is reduced by at least 40%, at least 45%, at least 50%, at least 55%, at least 60%, at least 65%, at least 70%, at least 75%, at least 80%, at least 85%, at least 90%, at least 95%, or at least 99% compared to the nuclease activity of the corresponding wild-type Cas protein.

In the present application, the term "capable of binding" is used interchangeably with "binding to", "specifically recognizing", "targeting", etc., and generally refers to that the binding molecule (e.g., a gene expression regulatory molecule of the present application) is capable of interacting with a nucleotide at a target gene or a target site, or that the binding molecule (e.g., a gene expression regulatory molecule of the present application) has sufficient affinity for the target gene or the target site, such interaction may be by means of conjugation, coupling, attachment, providing complementarity, providing covalent or non-covalent force, improving binding stability, and the like.

In the present application, the terms "guide RNA", "guide DNA" and "gRNA" are used interchangeably, and generally refer to a DNA molecule that can guide a nuclease (such as Argonaute, or Ago) to bind to and/or cleave a target gene. In some preferred embodiments, the guide DNA may include: a single-stranded DNA molecule (ssDNA), a single-stranded DNA molecule that is phosphorylated at the 5' end, a single-stranded DNA molecule that is hydroxylated at the 5' end, having a base fragment capable of being complementary to the target gene, and/or having a length of 8-35 nt. In some embodiments of the present application, the term "guide RNA" refers to an RNA that comprises: (1) an "activating" nucleotide sequence that binds to a guide RNA-guided endonuclease (e.g., a class II Cas nuclease, e.g., a type II, V, or VI Cas endonuclease) and activates the RNA-guided endonuclease; and (2) a "target" nucleotide sequence comprising a nucleotide sequence that hybridizes to a target nucleic acid. The "activating" nucleotide sequence and the "target" nucleotide sequence may be on separate RNA molecules (e.g., a "dual-guide RNA"); or they may be on the same RNA molecule ("single-guide RNA", also known as sgRNA).

In the present application, the term "DNA methyltransferase" generally refers to an enzyme that catalyzes the transfer of a methyl group to a DNA. Non-limiting examples of DNA methyltransferases include DNMT1, DNMT 3A, DNMT 3B, and DNMT 3L. For example, through DNA methylation, a DNA methyltransferase can modify the activity of a DNA fragment (e.g., regulate gene expression) without altering the DNA sequence. As described herein, a gene expression regulatory molecule may comprise one or more (e.g., two) DNA methyltransferases. When a DNA methyltransferase is included as part of a gene expression regulatory molecule, the DNA methyltransferase may be referred to as a "DNA methyltransferase domain." In various aspects, the DNA methyltransferase domain comprises a variant or homolog of an amino acid sequence having at least 50%, at least 55%, at least 60%, at least 65%, at least 70%, at least 75%, at least 80%, at least 85%, 90%, at least 91%, at least 92%, at least 93%, at least 94%, at least 95%, at least 96%, at least 97%, at least 98%, at least 99%, or 100% sequence identity to DNMT 3A. In various aspects, the DNA methyltransferase domain comprises a variant or homolog of an amino acid sequence having at least 50%, at least 55%, at least 60%, at least 65%, at least 70%, at least 75%, at least 80%, at least 85%, at least 90%, at least 91%, at least 92%, at least 93%, at least 94%, at least 95%, at least 96%, at least 97%, at least 98%, at least 99%, or 100% sequence identity to DNMT 3L.

In the present application, the term "functionally active fragment" generally refers to a fragment having a partial region of a full-length protein or nucleic acid but retaining or partially retaining the biological activity or function of the full-length protein or nucleic acid. For example, a functionally active fragment may retain or partially retain the ability of the full-length protein to bind to another molecule. For example, a functionally active fragment of a DNA methyltransferase may retain or partially retain the biologically active function of the full-length DNA methyltransferase in catalyzing the transfer of a methyl group to a DNA.

In the present application, the term "transcriptional repressor" generally refers to a substance and/or agent, such as a protein (eg, a transcription factor or a fragment thereof), that binds to a target nucleic acid sequence and causes a decrease in the expression level of a gene product associated with the target nucleic acid sequence. For example, the gene product may be an RNA (e.g., mRNA) transcribed from the gene or a polypeptide translated from an mRNA transcribed from the gene. Typically, an increase or decrease in the level of mRNA results in an increase or decrease in the level of polypeptide translated therefrom. Expression levels can be determined using standard techniques for measuring mRNA or protein. Non-limiting examples of transcriptional repressors include: an mSin3 interaction domain (SID) protein, a methyl-CpG-binding domain 2 (MBD2), a MBD3, a DNA methyltransferase (DNMT) 1 (DNMT1), a DNMT2A, a DNMT3A, a DNMT3B, a DNMT3L, a retinoblastoma protein (Rb), a methyl CpG binding protein 2 (Mecp2), a GATA-1 and its cofactor Fog1, a MAT2 modulator (ROM2), an arabidopsis HD2A protein (AtHD2A), a lysine-specific demethylase 1 (LSD1), and/or a Krüppel-associated box (KRAB).

In the present application, the term "KRAB" is also referred to as "Krüppel-associated box domain" generally refers to a transcriptional repression domain of about 45 to about 75 amino acid residues that is found in the transcription factor of a human zinc finger protein. In various aspects, the KRAB domain may comprise a variant or homolog of an amino acid sequence having at least 50%, at least 55%, at least 60%, at least 65%, at least 70%, at least 75%, at least 80%, at least 85%, at least 90%, at least 91%, at least 92%, at least 93%, at least 94%, at least 95%, at least 96%, at least 97%, at least 98%, at least 99%, or 100% sequence identity to ZIM3 KRAB domain or KOX1 KRAB domain.

In the present application, the term "split green fluorescent protein" generally refers to a polypeptide that is capable of splitting and upon recombination immediately forms an active green fluorescent protein.

In the present application, the term "GCN4" is a transcription factor in S. cerevisiae, the "master regulator" in the yeast genome, regulating nearly one tenth of the yeast genome, and it is a highly conserved protein whose homolog in mammals is Activating Transcription factor-4 (ATF4).

In the present application, the term "PDZ protein" generally refers to a naturally occurring protein containing a PDZ domain. Exemplary PDZ proteins include CASK, MPP1, DLG1, DLG2, PSD95, NeDLG, TIP-33, SYN1a, TIP-43, LDP, LIM, LIMK1, LIMK2, MPP2, N0S l, AF6, PTN_4, prIL16, 41.8kD, KIAA0559, RGS 12, KIAA0316, DVL1, TIP-40, TIAM1, MINT1, MAGI-1, MAGI-2, MAGI-3, KIAA0303, CBP, MINT3, TIP-2, KIAA0561 and/or TIP-1.

In the present application, the term "single chain antibody" or "scFv" generally refers to a single chain polypeptide containing one or more antigen binding sites. In addition, although the H and L chains of the Fv fragment are encoded by different genes, they can be linked together directly or via a peptide, for example, by recombinant methods, wherein a synthetic linker can be used to link the H and L chains into a single protein chain (known as a single chain antibody, sAb; Bird et al. 1988 Science 242: 423-426; and Huston et al. 1988 PNAS 85: 5879-5883). Such a single chain antibody is also encompassed within the term "antibody", and can be used as a binding determinant in the design and preparation of multispecific binding molecules, and can be prepared by recombinant techniques or by enzymatic or chemical cleavage of intact antibodies.

In the present application, the term "direct or indirect binding" generally refers to a relatively "direct linking" or "indirect linking". "Direct linking" generally refers to a direct linkage. For example, the direct linking may be a case where the linked substances (e.g., amino acid sequence segments) are directly linked without any spacer component (e.g., an amino acid residue or a derivative thereof); for example, an amino acid sequence segment X is directly linked to another amino acid sequence segment Y via an amide bond formed by the C-terminal amino acid of the amino acid sequence segment X and the N-terminal amino acid of the amino acid sequence segment Y. "Indirectly linking" generally refers to a case where the linked substances (e.g., amino acid sequence segments) are indirectly linked, and there is a spacer component (e.g., an amino acid residue or a derivative thereof) between them. For example, the spacer component used in the present application may be a stretch of amino acid residues, the sequence of which is selected from an amino acid sequence as set forth in any one of SEQ ID NOs: 125-132 (SEQ ID NO: 126 is GSG).

In the present application, a "nuclear localization sequence" or "NLS" generally refers to a peptide that directs a protein to the cell nucleus. In certain embodiments, the NLS comprises five basic positively charged amino acids. The NLS may be located anywhere on the peptide chain. In certain embodiments, the NLS is an NLS derived from SV40. In certain embodiments, the NLS comprises a sequence as set forth in any one of SEQ ID NOs: 338-340. In some embodiments, the NLS has an amino acid sequence that is at least 50%, 55%, 60%, 65%, 70%, 75%, 80%, 85%, 90%, 91%, 92%, 93%, 94%, 95%, 96%, 97%, 98%, 99%, or 100% identical to any one of SEQ ID NOs: 338-340.

In the present application, the term "label" refers to a peptide, which can be introduced into an expression vector, and which can be used to allow the deletion and/or purification of the expression product of one or more vector insertion fragments. Such labels are well known in the art, and include a radiolabeled amino acid or a polypeptide linked to a biotin moiety detectable by labeled avidin (e.g., streptavidin containing fluorescent label or enzymatic activity detectable by optical or colorimetric methods). Affinity labels such as FLAG, glutathione-S- transferase, maltose binding protein, cellulose binding domain, thioredoxin, NusA, mistin, chitin binding domain, cutinase, AGT, GFP and other widely used labels, such as those used in protein expression and purification systems. Further non-limiting examples for polypeptides include, but are not limited to, the following: a histidine label, a radioisotope or a radionuclide (e.g., 3H, 14C, 35S, 90Y, 99Tc, 111In, 125I, 177Lu, 166Ho or 153Sm); a fluorescent label (e.g., FITC, rhodamine, lanthanide phosphorus), an enzyme label (e.g., horseradish peroxidase, luciferase, alkaline phosphatase); a chemiluminescent label; a biotin group; a pendant polypeptide antigenic determinant recognized by a second reporter (e.g., leucine zipper pair sequence, a binding site for secondary antibody, metal binding domain, antigenic determinant marker); and a magnetic reagent, such as a gadolinium chelate.

In the present application, the term "nucleic acid" is used interchangeably with "polynucleotide", "nucleotide", "nucleotide sequence" and "oligonucleotide", and generally refers to a nucleotide (e.g., deoxyribonucleotide or ribonucleotide) and a polymer thereof in single-, double-, or multi-stranded form or a complement thereof. For example, the nucleotide may be a ribonucleotide, a deoxyribonucleotide, or a modified version thereof. For example, the nucleotide may be a single-stranded and double-stranded DNA, a single-stranded and double-stranded RNA, and a hybrid molecule having a mixture of single-stranded and double-stranded DNA and RNA. For example, the nucleotides may include, but are not limited to, any type of RNA, such as mRNA, siRNA, miRNA, sgRNA, and guide RNA, and any type of DNA, genomic DNA, plasmid DNA, and minicircle DNA, and any fragments thereof. The terms also encompass nucleic acids containing known nucleotide analogs or modified backbone residues or linkages, which nucleic acids are synthetic, naturally occurring, and non-naturally occurring.

In the present application, the term "sequence encoding" or "nucleic acid encoding" generally refers to a nucleic acid (RNA or DNA molecule) comprising a nucleotide sequence encoding a protein. The coding sequence may further comprise initiation and termination signals operably linked to regulatory elements comprising a promoter and a polyadenylation signal capable of directing expression in cells of an individual or mammal to which the nucleic acid has been administered. The coding sequence can be codon optimized. In the present application, the term "intron" the term "intron" generally refers to a DNA fragment that has been transcribed, but has been removed from an RNA transcript by splicing together either end of the sequence (exon). An intron is considered to be an interfering sequence within the protein coding region of a gene, and usually does not contain the information represented by the protein produced by the gene.

In the present application, the term "recombinant vector" generally refers to a nucleic acid molecule capable of transporting it as well as another nucleic acid to which it is linked. One type of vector is a "plasmid", which refers to a circular double-stranded DNA loop into which additional DNA segments can be ligated. Alternatively, the vector may be linear. Another type of vector is a viral vector, wherein the additional DNA segments can be ligated into the viral genome. Particular vectors are capable of autonomous replication within a host cell into which they are introduced (e.g., a bacterial vector having a bacterial origin of replication and an episomal mammalian vector). Other vectors (e.g., non-episomal mammalian vectors) can be integrated into the genome of the host cell upon introduction into the host cell, and thereby are replicated along with the host genome.

In the present application, the term "regulatory element" refers to a genetic element capable of controlling the expression of a nucleic acid sequence. For example, splicing signals, promoter sequences, polyadenylation signals, transcription termination sequences, upstream regulatory domains, replication origins, internal ribosome entry sites ("IRES"), enhancers and the like, together provide for the replication, transcription and translation of the coding sequence in a recipient cell. Not all of these control sequences need to be present.

In the present application, the term "promoter" generally refers to a nucleotide sequence that controls or regulates the transcription of a nucleotide sequence (e.g., a coding sequence) operably associated with the promoter. The coding sequence controlled or regulated by the promoter can encode a polypeptide and/or a functional RNA. Generally, a "promoter" refers to a nucleotide sequence comprising an RNA polymerase II binding site and directing the initiation of transcription. Generally, the promoter is located 5' or upstream of the origin of the coding region relative to the corresponding coding sequence. The promoter may comprise other elements that act as regulators of gene expression; for example, a promoter region. In some embodiments, the promoter region may comprise at least one intron. Promoters may include, for example, constitutive, inducible, temporally regulated, developmentally regulated, chemically regulated, tissue-preferred and/or tissue-specific promoters for use in preparing recombinant nucleic acid molecules, such as "synthetic nucleic acid constructs" or "protein-RNA complexes". These different types of promoters are known in the art.

**In** the present application, the term "enhancer" generally refers to a regulatory DNA sequence, for example 50-1500 bp, that can be bound by a protein (activator) to stimulate or enhance the transcription of a gene or several genes. These activators (also known as transcription factors) interact with mediator complexes and recruit polymerase II and general transcription factors, and then begin to transcribe genes. Enhancers are usually cis-acting, but can be located upstream or downstream of the initiation site of the gene or the gene they regulate. **In** addition, enhancers may be forward or backward oriented, and do not need to be located near the transcription initiation site to affect transcription, as some enhancers have been found hundreds of thousands of base pairs upstream or downstream of the initiation site. Enhancers can also be found within introns.

**In** the present application, the term "cleavage peptide" refers to a class of polypeptides capable of performing the function of cleavage proteins. For example, the cleavage peptide can achieve protein cleavage via ribosome skipping rather than protease hydrolysis. For example, the cleavage peptide may be a cleavage 2A peptide, which may include T2A, F2A, P2A and/or E2A.

**In** the present application, the term "delivery vector" generally refers to a transfer vehicle capable of delivering an agent (e.g., a nucleic acid molecule) to a target cell. The delivery vector can deliver the agent to a specific subclass of cells. For example, a delivery vector can be targeted to certain cell types by virtue of an inherent feature of the delivery vector or by a moiety coupled to the vector, contained within it (or bound to the vector, such that the moiety and the delivery vector are maintained together, such that the moiety is sufficient to target the delivery vector). The delivery vector can also increase the in vivo half-life of the agent to be delivered and/or the bioavailability of the agent to be delivered. Delivery vectors may include viral vectors, virus-like particles, polycationic vectors, peptide vectors, liposomes, and/or hybrid vectors. For example, if the target cell is a hepatocyte, the properties of the delivery vector (e.g., size, charge, and/or pH) may be effective to deliver the delivery vector and/or molecules encapsulated therein to the target cell, reduce immune clearance, and/or facilitate retention in the target cell.

**In** the present application, the term "liposome" generally refers to a vesicle having an internal space separated from the external medium by one or more bilayer membranes. In some embodiments, the bilayer membrane may be formed by amphoteric molecules, such as synthetic or naturally derived lipids containing spatially isolated hydrophilic and hydrophobic domains; In some other embodiments, the bilayer membrane may be formed by amphiphilic polymers and surfactants. In some embodiments, the liposome is a spherical vesicle structure composed of a monolayer or multilayer lipid bilayer surrounding an inner aqueous compartment, and a relatively impermeable outer lipophilic phospholipid bilayer. In some embodiments, liposomes are biocompatible, non-toxic, can deliver hydrophilic and lipophilic drug molecules, protect their cargo from degradation by plasma enzymes, and transport their cargo across biological membranes and blood-brain barrier (BBB). Liposomes can be made from several different types of lipids, such as phospholipids. The liposomes may comprise natural phospholipids and lipids (such as 1,2-distearoyl-sn-glycero-3-phosphatidylcholine (DSPC), sphingomyelin, phosphatidylcholine, monosialoganglioside, or any combination thereof. In order to modify the structure and properties of liposomes, several other additives can be added to the liposomes. For example, the liposomes may further comprise cholesterol, sphingomyelin and/or 1,2-dioleoyl-sn-glycero-3-phosphoethanolamine (DOPE), for example, to increase stability and/or prevent leakage of cargo inside the liposomes.

The term a "lipid nanoparticle (LNP)" generally refers to a particle comprising multiple (i.e., more than one) lipid molecules physically associated (e.g., covalently or non-covalently) to each other by intermolecular forces. LNPs may be, for example, microspheres (including unilamellar and multilamellar vesicles, such as liposomes), dispersed phases in emulsions, micelles, or internal phases in suspensions. LNPs can encapsulate nucleic acids within cationic lipid particles (eg, liposomes), and can be delivered to cells relatively easily. In some examples, the lipid nanoparticles do not contain any viral components, which helps to minimize safety and immunogenicity issues. The lipid particles are useful for in vitro, ex vivo and in vivo delivery. The lipid particles are also useful for cell populations of various sizes. The LNP of the present application can be readily prepared by various methods known in the art, such as by mixing an organic phase with an aqueous phase. The mixing of the two phases can be achieved by a microfluidic device and an impinging flow reactor. The more thoroughly the organic phase and the aqueous phase are mixed, the better the encapsulation efficiency and the particle size distribution of the obtained LNPs. Preferably, the particle size of LNP can be adjusted by changing the mixing speed of the organic phase and the aqueous phase. The faster the mixing speed, the smaller the particle size of the prepared LNPs. The encapsulation efficiency can be optimized by adjusting the N/P (ionizable lipid/nucleic acid) ratio of the LNP system. In some examples, LNPs can be used to deliver DNA molecules and/or RNA molecules (e.g., mRNA of Cas, sgRNA). In certain cases, LNPs can be used to deliver RNP complexes of Cas/gRNA. In some embodiments, LNPs are used to deliver mRNA and gRNA.

In the present application, the term "subject" generally refers to an animal, typically a mammal, such as a human, a non-human primate (ape, gibbon, gorilla, chimpanzee, orangutan, macaque), a domestic animal (dog and cat), a farm animal (poultry such as chicken and duck, horse, cattle, goat, sheep, pig) and an experimental animal (mouse, rat, rabbit, guinea pig). Human subjects include fetal, neonatal, infant, adolescent, and adult subjects. Subjects include animal disease models, such as mice and other animal models of blood coagulation diseases (such as HemA), and other animal models known to those skilled in the art.

In the present application, the term "comprising" generally means including the explicitly specified features, but not excluding other elements.

In the present application, the term "selected from" generally means including the selected objects and all combinations thereof. For example, "selected from (:) A, B and C" is intended to include all combinations of A, B and C, for example, A, B, C, A+B, A+C, B+C or A+B+C.

In the present application, the term "about" generally refers to a variation within a range of 0.5%-10% above or below a specified value, for example, 0.5%, 1%, 1.5%, 2%, 2.5%, 3%, 3.5%, 4%, 4.5%, 5%, 5.5%, 6%, 6.5%, 7%, 7.5%, 8%, 8.5%, 9%, 9.5%, or 10% above or below the specified value.

### DETAILED DESCRIPTION

In one aspect, the present application provides a complex comprising a first fusion and a second fusion, wherein the fusion of one of the first fusion and the second fusion comprises a DNA methylation domain and at least one recruitment domain A, and wherein the other fusion comprises a transcriptional repressor domain and at least one recruitment domain A'; wherein the recruitment domain A and the recruitment domain A' are capable of interacting, such that the fusion of one of the first fusion and the second fusion or a portion thereof is capable of being recruited to the vicinity of the other fusion.

In another aspect, the present application provides a nucleic acid encoding the complex described in the present application. For example, the nucleic acid comprises DNA and/or mRNA. For example, the nucleic acid can be used to treat or alleviate a disease or a disorder thereof associated with abnormal target gene expression and/or abnormal target gene activity. In some embodiments, the nucleic acid is mRNA; one or more modification techniques can be used to produce a more stable mRNA. Known mRNA modification techniques can be roughly divided into three categories: using artificially synthesized non-natural ribonucleic acid instead of natural ribonucleic acid to synthesize mRNA; adding 5' caps, 3' poly (A) "tail" and UTR (untranslated region) sequences; and using special new formulation techniques to effectively protect mRNA. Among them, the preferred mRNA modification techniques may involve synthesizing mRNA by replacing natural ribonucleic acids with artificially synthesized non-natural ribonucleic acids. Chemical modifications on eukaryotic mRNA can be roughly divided into three categories: methylation, pseudouridine (Ψ) and hypoxanthine. For example, the chemical modification may be selected from: pseudouridine, N1-methylpseudouridine, N1-ethylpseudouridine, 2-thiouridine, 4'-thiouridine, 5-methylcytosine, 2-thio-1-methyl-1-deaza-pseudouridine, 2-thio-1-methyl-pseudouridine, 2-thio-5-aza-uridine, 2-thio-dihydropseudouridine, 2-thio-dihydrouridine, 2-thio-pseudouridine, 4-methoxy-2-thio-pseudouridine, 4-methoxy-pseudouridine, 4-thio-1-methyl-pseudouridine, 4-thio-pseudouridine, 5-aza-uridine, dihydropseudouridine, 5-methyluridine, 5-methoxyuridine and 2'-O-methyluridine. For example, the nucleic acid is a recombinant vector comprising a nucleic acid encoding the complex described in the present application. For example, a recombinant vector may refer to a nucleic acid molecule capable of transporting another nucleic acid to which it is linked. Recombinant vectors may include single-stranded, double-stranded, or partially double-stranded nucleic acid molecules; nucleic acid molecules comprising one or more free ends, no free ends (e.g., circular); nucleic acid molecules comprising DNA, RNA, or both; and other types of polynucleotides known in the art. For example, viral vectors can be used. Viral vectors may comprise virus-derived DNA or RNA sequences for packaging into a virus (e.g., a retrovirus, a replication defective retrovirus, an adenovirus, a replication defective adenovirus and an adeno-associated virus AAV). Viruses and viral vectors can be used for in vitro, ex vivo and/or in vivo delivery.

**In** another aspect, the present application provides a delivery vector, comprising the complex described in the present application and/or the nucleic acid described in the present application, and optionally a liposome and/or a lipid nanoparticle. For example, the delivery vector can be introduced into cells by a physical delivery method. Examples of physical methods include microinjection, electroporation, and hydrodynamic delivery. For example, LNPs can encapsulate nucleic acids in cationic lipid particles (e.g., liposomes), and can be delivered to cells relatively easily. In some examples, the lipid nanoparticles do not contain any viral components, which helps to minimize safety and immunogenicity issues. Lipid particles are useful for in vitro, ex vivo and in vivo delivery. Components of LNPs may include a cationic lipid, an ionizable lipid, a PEGylated lipid and/or a supporting lipid, and optionally a cholesterol component.

In another aspect, the present application provides a composition comprising the complex described in the present application, the nucleic acid described in the present application, and/or the delivery vector described in the present application. For example, the complex in the composition, the nucleic acid (or recombinant vector) encoding the complex and the delivery vector may be contained simultaneously in one composition, or separately in different compositions. For example, when the complex in the composition, the nucleic acid (or recombinant vector) encoding the complex, and/or the delivery vector are used, they can be used simultaneously, or they can be used separately.

In another aspect, the present application provides a cell comprising the complex described in the present application, the nucleic acid described in the present application, the delivery vector described in the present application, and/or the composition described in the present application.

In another aspect, the present application provides a kit comprising the complex described in the present application, the nucleic acid described in the present application, the delivery vector described in the present application, the composition described in the present application, and/or the cell described in the present application. For example, the kit further comprises at least one container for placing the above components. For example, the kit comprises more than one of the above components, and further comprises a second, third and/or other containers in addition to the container, into which the more than one of the above components can be separately placed. For example, the kit may place various combinations of the above components in the containers. For example, the kit further comprises a buffer reagent, a means for mixing, a means for measuring, a means for sorting and/or a means for labeling. For example, the kit further comprises a package for containing the various containers. For example, the kit further comprises instructions for using the kit components. For example, the instructions comprise a physical paper form and/or a machine-readable electronic form.

**In** another aspect, the present application provides a method for regulating target gene expression, comprising administering the complex described in the present application, the nucleic acid described in the present application, the delivery vector described in the present application, the composition described in the present application, the cell described in the present application, and/or the kit described in the present application. For example, the method for inhibiting target gene expression is to introduce the complex, the nucleic acid, the delivery vector, the composition, the cell and/or the kit into cells containing the target gene. For example, the introduction into cells can be carried out by non-viral or viral-based transfection methods. For example, the non-viral transfection methods include any suitable method for introducing into cells without using viral DNA or viral particles as a delivery system, and non-limiting examples of non-viral transfection methods include nanoparticle encapsulation of nucleic acids encoding the complex (e.g., lipid nanoparticles, gold nanoparticles, etc.), calcium phosphate transfection, liposome transfection, nucleofection, sonoporation, transfection by heat shock, magnetofection, and electroporation. For example, virus-based transfection methods include any viral vector suitable for use in the methods described in the present application, non-limiting examples of which include, but are not limited to, retroviral, adenoviral, lentiviral, and/or adeno-associated viral vectors. For example, the method for inhibiting target gene expression further comprises introducing the complex, the nucleic acid, the delivery vector, the composition, the cell and/or the kit into cells from the external environment. For another example, the method for inhibiting target gene expression comprises contacting the complex, the nucleic acid, the delivery vector, and/or the composition with a transcriptional regulatory element near and/or of the target gene. For example, the contacting means that the first fusion, the second fusion, and the guide RNA described in the present application are contacted with a transcriptional regulatory element near and/or of the target gene, and the guide RNA forms a complex with a fusion comprising the DNA binding domain, the complex specifically recognizes and hybridizes to a specific region in the target gene, while the first fusion and the second fusion are recruited to the vicinity of the DNA binding domain through direct or indirect interaction between their recruitment domain A and recruitment domain A', thereby regulating the expression of the target nucleic acid. For example, the method comprises allowing the first fusion, the second fusion and the guide RNA described in the present application to exist in the form of a complex (e.g., an assembled ribonucleoprotein complex), and contacting the complex with a transcriptional regulatory element near and/or of the target gene.

In another aspect, the present application provides a method for treating or alleviating a disease or a disorder thereof associated with abnormal target gene expression and/or abnormal target gene activity, wherein the method comprises administering to a subject in need thereof an effective amount of the complex described in the present application, the nucleic acid described in the present application, the delivery vector described in the present application, the composition described in the present application, the cell described in the present application, and/or the kit described in the present application. For example, the treatment method comprises mixing the complex, the nucleic acid, the delivery vector, the composition, the cell and/or the kit with a therapeutic agent, and delivering it systemically to a subject in need thereof, so that it is extensively exposed to a substantial portion of the body, which can be carried out by any means known in the art, including, but not limited to, intravenous, intraarterial, subcutaneous, intracavitary, and intraperitoneal delivery. For example, the treatment method comprises mixing the complex, the nucleic acid, the delivery vector, the composition, the cell and/or the kit with a therapeutic agent, and delivering it locally to a subject in need thereof, so that it directly reaches the target site in the organism, which can be carried out by, for example, direct injection into the disease site (e.g., a tumor or inflammation site) or the target organ (e.g., liver, heart, pancreas, kidney, etc.). For example, the local delivery includes local administration or local injection techniques, including, but not limited to, intramuscular, subcutaneous, or intradermal injection. For example, the local delivery does not exclude systemic pharmacological effects. For example, the diseases include cardiovascular disease, non-alcoholic steatohepatitis, AMD, age-related macular degeneration, type 2 diabetes, obesity, liver failure, dislipidemia, diabetic atherosclerosis and/or hypertension.

In another aspect, the present application provides the use of the complex described in the present application, the nucleic acid described in the present application, the delivery vector described in the present application, the composition described in the present application, the cell described in the present application, and/or the kit described in the present application in the manufacture of a medicament for treating or alleviating a disease or a disorder thereof associated with abnormal target gene expression and/or abnormal target gene activity.

In another aspect, the present application provides the complex described in the present application, the nucleic acid described in the present application, the delivery vector described in the present application, the composition described in the present application, the cell described in the present application or the kit described in the present application, which is used for treating or alleviating a disease or a disorder thereof associated with abnormal target gene expression and/or abnormal target gene activity.

### First fusion or second fusion

In some embodiments, the first fusion and the second fusion of the complex of the present application can be generally divided into two situations: (1) one of the two fusions comprises a nucleic acid binding domain, a DNA methylation domain and a recruitment domain A, and the other fusion comprises a transcriptional repressor domain and a recruitment domain A', or (2) one of the two fusions comprises a nucleic acid binding domain, a transcriptional repressor domain and a recruitment domain A, and the other fusion comprises a DNA methylation domain and a recruitment domain A'.

Specifically, in some embodiments of the above-mentioned situation (1), one of the two fusions may comprise, in order from N-terminus to C-terminus, a DNA methylation domain, a nucleic acid binding domain and a recruitment domain A. For example, in some embodiments of the above-mentioned situation (2), one of the two fusions may comprise, from N-terminus to C-terminus, a recruitment domain A, a nucleic acid binding domain and a transcriptional repressor domain. For example, in some embodiments of the above-mentioned situation (1), the other of the two fusions may comprise, from N-terminus to C-terminus, a transcriptional repressor domain and a recruitment domain A', or a recruitment domain A' and a transcriptional repressor domain, that is, the transcriptional repressor domain and the recruitment domain A' are linked in an interchangeable order. For example, in some embodiments of the above-mentioned situation (2), the other of the two fusions may comprise, from N-terminus to C-terminus, a DNA methylation domain and a recruitment domain A', or a recruitment domain A' and a DNA methylation domain, that is, the DNA methylation domain and the recruitment domain A' are linked in an interchangeable order.

In some more specific embodiments, the nucleic acid binding domain is a DNA binding domain. For example, the DNA binding domain may be selected from: a TALE domain, a zinc finger domain, a tetR domain, a meganuclease, a Cas protein, an Argonaute (Ago) protein, and a homolog, a modified form or a variant thereof. For example, the DNA binding domain may be a Cas protein, and the Cas protein is a class II Cas nuclease. Further, the Cas protein may be selected from a class II type II Cas nuclease and a class II type V Cas nuclease; for example, the Cas protein may be a Cas9 or Cas12 protein. In certain embodiments, the Cas protein may be a dead Cas9 (dCas9) protein or a dead Cas12 (dCas12) protein. For example, the DNA binding domain described in the present application may comprise, but is not limited to, an amino acid sequence as set forth in any one of SEQ ID NOs: 1-9, 343 and 344.

In some more specific embodiments, the transcriptional repressor is selected from one or more of the following domains: KRAB, ZIM3, ZNF680, ZNF554, ZNF264, ZNF582, ZNF324, ZNF669, ZNF354A, ZNF82, ZNF595, ZNF419, ZNF566, ZIM2, EHMT2, SUV39H1, ZFPM1, TRIM28, EZH2, MXD1, SID, LSD1, HP1a, HDAC3, HDAC1, PRMT1, SETDB1, hSIRT1, ZNF436, ZNF257, ZNF675, ZNF490, ZNF320, ZNF331, ZNF816, ZNF41, ZNF189, ZNF528, ZNF543, ZNF140, ZNF610, ZNF350, ZNF8, ZNF30, ZNF98, ZNF677, ZNF596, ZNF214, ZNF37A, ZNF34, ZNF250, ZNF547, ZNF273, ZFP82, ZNF224, ZNF33A, ZNF45, ZNF175, ZNF184, ZFP28-1, ZFP28-2, ZNF18, ZNF213, ZNF394, ZFP1, ZFP14, ZNF416, ZNF557, ZNF729, ZNF254, ZNF764, ZNF785, ZNF10, CBX5, RYBP, YAF2, MGA, CBX1, SCMH1, MPP8, SUMO3, HERC2, BIN1, PCGF2, TOX, FOXA1, FOXA2, IRF2BP1, IRF2BP2, IRF2BPL IRF-2BP1_2 N-terminal domain, HOXA13, HOXB13, HOXC13, HOXA11, HOXC11, HOXC10, HOXA10, HOXB9, HOXA9, ZFP28, ZN334, ZN568, ZN37A, ZN181, ZN510, ZN862, ZN140, ZN208, ZN248, ZN571, ZN699, ZN726, ZIK1, ZNF2, Z705F, ZNF14, ZN471, ZN624, ZNF84, ZNF7, ZN891, ZN337, Z705G, ZN529, ZN729, ZN419, Z705A, ZN302, ZN486, ZN621, ZN688, ZN33A, ZN554, ZN878, ZN772, ZN224, ZN184, ZN544, ZNF57, ZN283, ZN549, ZN211, ZN615, ZN253, ZN226, ZN730, Z585A, ZN732, ZN681, ZN667, ZN649, ZN470, ZN484, ZN431, ZN382, ZN254, ZN124, ZN607, ZN317, ZN620, ZN141, ZN584, ZN540, ZN75D, ZN555, ZN658, ZN684, RBAK, ZN829, ZN582, ZN112, ZN716, HKR1, ZN350, ZN480, ZN416, ZNF92, ZN100, ZN736, ZNF74, ZN443, ZN195, ZN530, ZN782, ZN791, ZN331, Z354C, ZN157, ZN727, ZN550, ZN793, ZN235, ZN724, ZN573, ZN577, ZN789, ZN718, ZN300, ZN383, ZN429, ZN677, ZN850, ZN454, ZN257, ZN264, ZN485, ZN737, ZNF44, ZN596, ZN565, ZN543, ZFP69, SUMO1, ZNF12, ZN169, ZN433, ZN175, ZN347, ZNF25, ZN519, Z585B, ZN517, ZN846, ZN230, ZNF66, ZN713, ZN816, ZN426, ZN674, ZN627, ZNF20, Z587B, ZN316, ZN233, ZN611, ZN556, ZN234, ZN560, ZNF77, ZN682, ZN614, ZN785, ZN445, ZFP30, ZN225, ZN551, ZN610, ZN528, ZN284, ZN418, ZN490, ZN805, Z780B, ZN763, ZN285, ZNF85, ZN223, ZNF90, ZN557, ZN425, ZN229, ZN606, ZN155, ZN222, ZN442, ZNF91, ZN135, ZN778, ZN534, ZN586, ZN567, ZN440, ZN583, ZN441, ZNF43, ZN589, ZN563, ZN561, ZN136, ZN630, ZN527, ZN333, Z324B, ZN786, ZN709, ZN792, ZN599, ZN613, ZF69B, ZN799, ZN569, ZN564, ZN546, ZFP92, ZN723, ZN439, ZFP57, ZNF19, ZN404, ZN274, CBX3, ZN250, ZN570, ZN675, ZN695, ZN548, ZN132, ZN738, ZN420, ZN626, ZN559, ZN460, ZN268, ZN304, ZN605, ZN844, SUMO5, ZN101, ZN783, ZN417, ZN182, ZN823, ZN177, ZN197, ZN717, ZN669, ZN256, ZN251, CBX4, CDY2, CDYL2, ZN562, ZN461, Z324A, ZN766, ID2, ZN214, CBX7, ID1, CREM, SCX, ASCL1, ZN764, SCML2, TWST1, CREB1, TERF1, ID3, CBX8, GSX1, NKX22, ATF1, TWST2, ZNF17, TOX3, TOX4, ZMYM3, I2BP1, RHXF1, SSX2, I2BPL, ZN680, TRI68, HXA13, PHC3, TCF24, HXB13, HEY1, PHC2, ZNF81, FIGLA, SAM11, KMT2B, HEY2, JDP2, HXC13, ASCL4, HHEX, GSX2, ETV7, ASCL3, PHC1, OTP, I2BP2, VGLL2, HXA11, PDLI4, ASCL2, CDX4, ZN860, LMBL4, PDIP3, NKX25, CEBPB, ISL1, CDX2, PROP1, SIN3B, SMBT1, HXC11, HXC10, PRS6A, VSX1, NKX23, MTG16, HMX3, HMX1, KIF22, CSTF2, CEBPE, DLX2, PPARG, PRIC1, UNC4, BARX2, ALX3, TCF15, TERA, VSX2, HXD12, CDX1, TCF23, ALX1, HXA10, RX, CXXC5, SCML1, NFIL3, DLX6, MTG8, CEBPD, SEC13, FIP1, ALX4, LHX3, PRIC2, MAGI3, NELL1, PRRX1, MTG8R, RAX2, DLX3, DLX1, NKX26, NAB1, SAMD7, PITX3, WDR5, MEOX2, NAB2, DHX8, CBX6, EMX2, CPSF6, HXC12, KDM4B, LMBL3, PHX2A, EMX1, NC2B, DLX4, SRY, ZN777, ZN398, GATA3, BSH, SF3B4, TEAD1, TEAD3, RGAP1, PHF1, GATA2, FOXO3, ZN212, IRX4, ZBED6, LHX4, SIN3A, RBBP7, NKX61, R51A1, MB3L1, DLX5, NOTC1, TERF2, ZN282, RGS12, ZN840, SPI2B, PAX7, NKX62, ASXL2, FOXO1, GATA1, ZMYM5, LRP1, MIXL1, SGT1, LMCD1, CEBPA, SOX14, WTIP, PRP19, NKX11, RBBP4, DMRT2, SMCA2, and functionally active fragments thereof.

In some more specific embodiments, the DNA methylation domain comprises at least one DNA methyltransferase or a functionally active fragment thereof. For example, the DNA methyltransferase is selected from DNMT3A, DNMT3B, DNMT3c, DNMT1, DNMT2 and DNMT3L. For example, the DNA methylation domain comprises at least one DNMT3A and at least one DNMT3L. For example, the at least one DNMT3A and the at least one DNMT3L are linked in an interchangeable order. For example, the DNA methylation domain comprises one DNMT3A and one DNMT3L, and they are linked in an interchangeable order. For example, the DNA methyltransferase comprises an amino acid sequence as set forth in any one of SEQ ID NOs: 19-24.

The first fusion and the second fusion of the complex of the present application form an aggregated complex through the interaction between the recruitment domains they each comprise. Therefore, the present application provides non-limiting examples of combinations of recruitment domain A and recruitment domain A': (1) the domain of one of the recruitment domain A and the recruitment domain A' is a GCN4, and wherein the other domain is a scFv; or (2) the domain of one of the recruitment domain A and the recruitment domain A' is a GFP11 fragment, and wherein the other domain is a GFP1-10; or (3) the domain of one of the recruitment domain A and the recruitment domain A' is a GVKESLV, and wherein the other domain is a PDZ protein domain. Similarly, just as GFP11 and GFP1-10, which are respectively derived from split GFP (SEQ ID NO: 15), form the recruitment domain A and the recruitment domain A', the same can be applied to other types of fluorescent proteins, such as mCherry (SEQ ID NO: 16), eYFP (SEQ ID NO: 18), eCFP (SEQ ID NO: 17), etc., that is, different groups of recruitment domain A and recruitment domain A' can be obtained by split mCherry, split eYFP, or split eCFP for use in the complex provided in the present application. In some embodiments, one of the first fusion and the second fusion of the complex of the present application may comprise two or more recruitment domains, and they are linked by a linker sequence. The amino acid sequences of exemplary recruitment domains may comprise those as set forth in any one of SEQ ID NOs: 10-14.

In consideration of the aforementioned circumstances, the present application can provide the following amino acid sequences of the first fusion or the second fusion:

| **SEQ ID NO:** | **Domai n Inform ation** | **Amino acid sequence** (dCas or TALE: **bold,** DNMT: ***bold italics,*** transcriptional repressor: < **underlined bold** >; recruitment domain: *underlined italics)* |
|---|---|---|
| 51 | DNMT | MNHDQEFDPPKVYPPVPAEKRKPIRVLSLFDGIATGLLVLKDLGIQV |
| | 3A(CD )-DNMT 3L(CD) -dCas9-10xGC N4 | DRYIASEVCEDSITVGMVRHQGKIMYVGDVRSVTQKHIQEWGPFDL VIGGSPCNDLSIVNPARKGLYEGTGRLFFEFYRLLHDARPKEGDDRP FFWLFENVVAMGVSDKRDISRFLESNPVMIDAKEVSAAHRARYFWG NLPGMNRPLASTVNDKLELQECLEHGRIAKFSKVRTITTRSNSIKQG KDQHFPVFMNEKEDILWCTEMERVFGFPVHYTDVSNMSRLARQRL LGRSWSVPVIRHLFAPLKEYFACVSSGNSNANSRGPSFSSGLVPLSLR GSHMGPMEIYKTVSAWKRQPVRVLSLFRNIDKVLKSLGFLESGSGSG GGTLKYVEDVTNVVRRDVEKWGPFDLVYGSTQPLGSSCDRCPGWY MFQFHRILQYALPRQESQRPFFWIFMDNLLLTEDDQETTTRFLQTE AVTLQDVRGRDYQNAMRVWSNIPGLKSKHAPLTPKEEEYLQAQVRS RSKLDAPKVDLLVKNCLLPLREYFKYFSQNSLPLGGPSSGAPPPSGGS PAGSPTSTEEGTSESATPESGPGTSTEPSEGSAPGSPAGSPTSTEEGTST EPSEGSAPGTSTEPSEPKKKRKVMDKKYSIGLAIGTNSVGWAVITD EYKVPSKKFKVLGNTDRHSIKKNLIGALLFDSGETAEATRLKRTA RRRYTRRKNRICYLQEIFSNEMAKVDDSFFHRLEESFLVEEDKKH ERHPIFGNIVDEVAYHEKYPTIYHLRKKLVDSTDKADLRLIYLAL AHMIKFRGHFLIEGDLNPDNSDVDKLFIQLVQTYNQLFEENPINA SGVDAKAILSARLSKSRRLENLIAQLPGEKKNGLFGNLIALSLGL TPNFKSNFDLAEDAKLQLSKDTYDDDLDNLLAQIGDQYADLFLA AKNLSDAILLSDILRVNTEITKAPLSASMIKRYDEHHQDLTLLKAL VRQQLPEKYKEIFFDQSKNGYAGYIDGGASQEEFYKFIKPILEKM DGTEELLVKLNREDLLRKQRTFDNGSIPHQIHLGELHAILRRQED FYPFLKDNREKIEKILTFRIPYYVGPLARGNSRFAWMTRKSEETI TPWNFEEVVDKGASAQSFIERMTNFDKNLPNEKVLPKHSLLYEY FTVYNELTKVKYVTEGMRKPAFLSGEQKKAIVDLLFKTNRKVTV KQLKEDYFKKIECFDSVEISGVEDRFNASLGTYHDLLKIIKDKDF LDNEENEDILEDIVLTLTLFEDREMIEERLKTYAHLFDDKVMKQL KRRRYTGWGRLSRKLINGIRDKQSGKTILDFLKSDGFANRNFMQ LIHDDSLTFKEDIQKAQVSGQGDSLHEHIANLAGSPAIKKGILQT VKVVDELVKVMGRHKPENIVIEMARENQTTQKGQKNSRERMK RIEEGIKELGSQILKEHPVENTQLQNEKLYLYYLQNGRDMYVDQ ELDINRLSDYDVDAIVPQSFLKDDSIDNKVLTRSDKNRGKSDNVPS EEVVKKMKNYWRQLLNAKLITQRKFDNLTKAERGGLSELDKAG FIKRQLVETRQITKHVAQILDSRMNTKYDENDKLIREVKVITLKS KLVSDFRKDFQFYKVREINNYHHAHDAYLNAVVGTALIKKYPKL ESEFVYGDYKVYDVRKMIAKSEQEIGKATAKYFFYSNIMNFFKT EITLANGEIRKRPLIETNGETGEIVWDKGRDFATVRKVLSMPQVN IVKKTEVQTGGFSKESILPKRNSDKLIARKKDWDPKKYGGFDSPT VAYSVLVVAKVEKGKSKKLKSVKELLGITIMERSSFEKNPIDFLE AKGYKEVKKDLIIKLPKYSLFELENGRKRMLASAGELQKGNELA LPSKYVNFLYLASHYEKLKGSPEDNEQKQLFVEQHKHYLDEIIEQ ISEFSKRVILADANLDKVLSAYNKHRDKPIREQAENIIHLFTLTNL GAPAAFKYFDTTIDRKRYTSTKEVLDATLIHQSITGLYETRIDLSQ LGGDYPYDVPDYASLGSGSPKKKRKVEDPKKKRKVDGIGSGSNGSS GEELLSKNYHLENEVARLKKGSGSGGSGSGGSGSGSGGSGSGGSGSGE ELLSKNYHLENEVARLKKGSGSGGSGSGGSGSGSGGSGSGGSGSGEEL LSKNYHLENEVARLKKGSGSGGSGSGGSGSGSGGSGSGGSGSGEELLS KNYHLENEVARLKKGSGSGGSGSGGSGSGSGGSGSGGSGSGEELLSKN YHLENEVARLKKGSGSGGSGSGGSGSGSGGSGSGGSGSGEELLSKNYH LENEVARLKKGSGSGGSGSGGSGSGSGGSGSGGSGSGEELLSKNYHLE NEVARLKKGSGSGGSGSGGSGSGSGGSGSGGSGSGEELLSKNYHLENE VARLKKGSGSGGSGSGGSGSGSGGSGSGGSGSGEELLSKNYHLENEVA RLKKGSGSGGSGSGGSGSGSGGSGSGGSGSGEELLSKNYHLENEVARL KKGSGSGQRGSKRTADGSEFESPKKKRKV |
| 52 | scFv-KRAB | MGPDIVMTQSPSSLSASVGDRVTITCRSSTGAVTTSNYASWVQEKPGKLFK GLIGGTNNRAPGVPSRFSGSLIGDKATLTISSLQPEDFATYFCALWYSNHW VFGQGTKVELKRGGGGSGGGGSGGGGSSGGGSEVKLLESGGGLVQPG GSLKLSCAVSGFSLTDYGVNWVRQAPGRGLEWIGVIWGDGITDYNSALK DRFIISKDNGKNTVYLQMSKVRSDDTALYYCVTGLFDYWGQGTLVTVSSY PYDVPDYAGGGGGSGGGGSGGGGSGGGGSSLDPGGGGSG<RTLVT FKDVFVDFTREEWKLLDTAQQIVYRNVMLENYKNLVSLGYQLT KPDVILRLEKGEEP>GSGKRTADGSEFESPKKKRKV |
| 53 | scFv-Zim3 | MGPDIVMTQSPSSLSASVGDRVTITCRSSTGAVTTSNYASWVQEKPGKLFK GLIGGTNNRAPGVPSRFSGSLIGDKATLTISSLQPEDFATYFCALWYSNHW VFGQGTKVELKRGGGGSGGGGSGGGGSSGGGSEVKLLESGGGLVQPG GSLKLSCAVSGFSLTDYGVNWVRQAPGRGLEWIGVIWGDGITDYNSALK DRFIISKDNGKNTVYLQMSKVRSDDTALYYCVTGLFDYWGQGTLVTVSSY PYDVPDYAGGGGGSGGGGSGGGGSGGGGSSLDPGGGGSG<VTFED VTVNFTQGEWQRLNPEQRNLYRDVMLENYSNLVSVGQGETTKP DVILRLEQGKEPWL>GSGKRTADGSEFESPKKKRKV |
| 54 | scFv-Znf680 | MGPDIVMTQSPSSLSASVGDRVTITCRSSTGAVTTSNYASWVQEKPGKLFK GLIGGTNNRAPGVPSRFSGSLIGDKATLTISSLQPEDFATYFCALWYSNHW VFGQGTKVELKRGGGGSGGGGSGGGGSSGGGSEVKLLESGGGLVQPG GSLKLSCAVSGFSLTDYGVNWVRQAPGRGLEWIGVIWGDGITDYNSALK DRFIISKDNGKNTVYLQMSKVRSDDTALYYCVTGLFDYWGQGTLVTVSSY PYDVPDYAGGGGGSGGGGSGGGGSGGGGSSLDPGGGGSG<LTFRD VAIEFSLEEWQCLDTAQRNLYRKVMFENYRNLVFLGIAVSKPHLI TCLEQGKEPWN>GSGKRTADGSEFESPKKKRKV |
| 55 | scFv-Znf554 | MGPDIVMTQSPSSLSASVGDRVTITCRSSTGAVTTSNYASWVQEKPGKLFK GLIGGTNNRAPGVPSRFSGSLIGDKATLTISSLQPEDFATYFCALWYSNHW VFGQGTKVELKRGGGGSGGGGSGGGGSSGGGSEVKLLESGGGLVQPG GSLKLSCAVSGFSLTDYGVNWVRQAPGRGLEWIGVIWGDGITDYNSALK DRFIISKDNGKNTVYLQMSKVRSDDTALYYCVTGLFDYWGQGTLVTVSSY PYDVPDYAGGGGGSGGGGSGGGGSGGGGSSLDPGGGGSG<VTFED VSMDFSQEEWELLEPAQKNLYREVMLENYRNVVSLEALKNQCT DVGIKEGPLSPAQT>GSGKRTADGSEFESPKKKRKV |
| 56 | scFv-Znf264 | MGPDIVMTQSPSSLSASVGDRVTITCRSSTGAVTTSNYASWVQEKPGKLFK GLIGGTNNRAPGVPSRFSGSLIGDKATLTISSLQPEDFATYFCALWYSNHW VFGQGTKVELKRGGGGSGGGGSGGGGSSGGGSEVKLLESGGGLVQPG GSLKLSCAVSGFSLTDYGVNWVRQAPGRGLEWIGVIWGDGITDYNSALK DRFIISKDNGKNTVYLQMSKVRSDDTALYYCVTGLFDYWGQGTLVTVSSY PYDVPDYAGGGGGSGGGGSGGGGSGGGGSSLDPGGGGSG<VTFDD VAVTFTKEEWGQLDLAQRTLYQEVMLENCGLLVSLGCPVPKAE LICHLEHGQEPWT>GSGKRTADGSEFESPKKKRKV |
| 57 | scFv-Znf582 | MGPDIVMTQSPSSLSASVGDRVTITCRSSTGAVTTSNYASWVQEKPGKLFK GLIGGTNNRAPGVPSRFSGSLIGDKATLTISSLQPEDFATYFCALWYSNHW VFGQGTKVELKRGGGGSGGGGSGGGGSSGGGSEVKLLESGGGLVQPG GSLKLSCAVSGFSLTDYGVNWVRQAPGRGLEWIGVIWGDGITDYNSALK DRFIISKDNGKNTVYLQMSKVRSDDTALYYCVTGLFDYWGQGTLVTVSSY PYDVPDYAGGGGGSGGGGSGGGGSGGGGSSLDPGGGGSG<ELFRD VAIVFSQEEWQWLAPAQRDLYRDVMLETYSNLVSLGLAVSKPDV ISFLEQGKEPWM>GSGKRTADGSEFESPKKKRKV |
| 58 | scFv-Znf324 | MGPDIVMTQSPSSLSASVGDRVTITCRSSTGAVTTSNYASWVQEKPGKLFK GLIGGTNNRAPGVPSRFSGSLIGDKATLTISSLQPEDFATYFCALWYSNHW VFGQGTKVELKRGGGGSGGGGSGGGGSSGGGSEVKLLESGGGLVQPG GSLKLSCAVSGFSLTDYGVNWVRQAPGRGLEWIGVIWGDGITDYNSALK DRFIISKDNGKNTVYLQMSKVRSDDTALYYCVTGLFDYWGQGTLVTVSSY PYDVPDYAGGGGGSGGGGSGGGGSGGGGSSLDPGGGGSG<MAFED VAVYFSQEEWGLLDTAQRALYRRVMLDNFALVASLGLSTSRPRV VIQLERGEEPWV>GSGKRTADGSEFESPKKKRKV |
| 59 | scFv-Znf669 | MGPDIVMTQSPSSLSASVGDRVTITCRSSTGAVTTSNYASWVQEKPGKLFK GLIGGTNNRAPGVPSRFSGSLIGDKATLTISSLQPEDFATYFCALWYSNHW VFGQGTKVELKRGGGGSGGGGSGGGGSSGGGSEVKLLESGGGLVQPG GSLKLSCAVSGFSLTDYGVNWVRQAPGRGLEWIGVIWGDGITDYNSALK DRFIISKDNGKNTVYLQMSKVRSDDTALYYCVTGLFDYWGQGTLVTVSSY PYDVPDYAGGGGGSGGGGSGGGGSGGGGSSLDPGGGGSG<VAFED VAVNFTQEEWALLDSSQKNLYREVMQETCRNLASVGSQWKDQN IEDHFEKPGKDIRN>GSGKRTADGSEFESPKKKRKV |
| 60 | scFv-Znf3 54 a | MGPDIVMTQSPSSLSASVGDRVTITCRSSTGAVTTSNYASWVQEKPGKLFK GLIGGTNNRAPGVPSRFSGSLIGDKATLTISSLQPEDFATYFCALWYSNHW VFGQGTKVELKRGGGGSGGGGSGGGGSSGGGSEVKLLESGGGLVQPG GSLKLSCAVSGFSLTDYGVNWVRQAPGRGLEWIGVIWGDGITDYNSALK DRFIISKDNGKNTVYLQMSKVRSDDTALYYCVTGLFDYWGQGTLVTVSSY PYDVPDYAGGGGGSGGGGSGGGGSGGGGSSLDPGGGGSG<LTFED VAVLFTRDEWRKLAPSQRNLYRDVMLENYRNLVSLGLPFTKPK VISLLQQGEDPWE >GSGKRTADGSEFESPKKKRKV |
| 61 | scFv-Znf82 | MGPDIVMTQSPSSLSASVGDRVTITCRSSTGAVTTSNYASWVQEKPGKLFK GLIGGTNNRAPGVPSRFSGSLIGDKATLTISSLQPEDFATYFCALWYSNHW VFGQGTKVELKRGGGGSGGGGSGGGGSSGGGSEVKLLESGGGLVQPG GSLKLSCAVSGFSLTDYGVNWVRQAPGRGLEWIGVIWGDGITDYNSALK DRFIISKDNGKNTVYLQMSKVRSDDTALYYCVTGLFDYWGQGTLVTVSSY PYDVPDYAGGGGGSGGGGSGGGGSGGGGSSLDPGGGGSG<VMFSD VSIDFSPEEWEYLDLEQKDLYRDVMLENYSNLVSLGCFISKPDVIS SLEQGKEPWK>GSGKRTADGSEFESPKKKRKV |
| 62 | scFv-Znf595 | MGPDIVMTQSPSSLSASVGDRVTITCRSSTGAVTTSNYASWVQEKPGKLFK GLIGGTNNRAPGVPSRFSGSLIGDKATLTISSLQPEDFATYFCALWYSNHW VFGQGTKVELKRGGGGSGGGGSGGGGSSGGGSEVKLLESGGGLVQPG GSLKLSCAVSGFSLTDYGVNWVRQAPGRGLEWIGVIWGDGITDYNSALK DRFIISKDNGKNTVYLQMSKVRSDDTALYYCVTGLFDYWGQGTLVTVSSY PYDVPDYAGGGGGSGGGGSGGGGSGGGGSSLDPGGGGSG<VTFRD VAIEFSPEEWKCLDPAQQNLYRDVMLENYRNLVSLGFVISNPDLV TCLEQIKEPCN>GSGKRTADGSEFESPKKKRKV |
| 63 | scFv-Znf419 | MGPDIVMTQSPSSLSASVGDRVTITCRSSTGAVTTSNYASWVQEKPGKLFK GLIGGTNNRAPGVPSRFSGSLIGDKATLTISSLQPEDFATYFCALWYSNHW VFGQGTKVELKRGGGGSGGGGSGGGGSSGGGSEVKLLESGGGLVQPG GSLKLSCAVSGFSLTDYGVNWVRQAPGRGLEWIGVIWGDGITDYNSALK DRFIISKDNGKNTVYLQMSKVRSDDTALYYCVTGLFDYWGQGTLVTVSSY PYDVPDYAGGGGGSGGGGSGGGGSGGGGSSLDPGGGGSG<VTFED VAVYFSQEEWRLLDDAQRLLYRNVMLENFTLLASLGLASSKTHE ITQLESWEEPFM>GSGKRTADGSEFESPKKKRKV |
| 64 | scFv-Znf566 | MGPDIVMTQSPSSLSASVGDRVTITCRSSTGAVTTSNYASWVQEKPGKLFK GLIGGTNNRAPGVPSRFSGSLIGDKATLTISSLQPEDFATYFCALWYSNHW VFGQGTKVELKRGGGGSGGGGSGGGGSSGGGSEVKLLESGGGLVQPG GSLKLSCAVSGFSLTDYGVNWVRQAPGRGLEWIGVIWGDGITDYNSALK DRFIISKDNGKNTVYLQMSKVRSDDTALYYCVTGLFDYWGQGTLVTVSSY PYDVPDYAGGGGGSGGGGSGGGGSGGGGSSLDPGGGGSG<VMFSD VSVDFSQEEWECLNDDQRDLYRDVMLENYSNLVSMGHSISKPNV ISYLEQGKEPWL>GSGKRTADGSEFESPKKKRKV |
| 65 | scFv-Zim2 | MGPDIVMTQSPSSLSASVGDRVTITCRSSTGAVTTSNYASWVQEKPGKLFK GLIGGTNNRAPGVPSRFSGSLIGDKATLTISSLQPEDFATYFCALWYSNHW VFGQGTKVELKRGGGGSGGGGSGGGGSSGGGSEVKLLESGGGLVQPG GSLKLSCAVSGFSLTDYGVNWVRQAPGRGLEWIGVIWGDGITDYNSALK DRFIISKDNGKNTVYLQMSKVRSDDTALYYCVTGLFDYWGQGTLVTVSSY PYDVPDYAGGGGGSGGGGSGGGGSGGGGSSLDPGGGGSG<VTFED VLVDFSPEELSSLSAAQRNLYREVMLENYRNLVSLGHQFSKPDIIS RLEEEEESYAM>GSGKRTADGSEFESPKKKRKV |
| 66 | scFv-EHMT 2(G9A) | MGPDIVMTQSPSSLSASVGDRVTITCRSSTGAVTTSNYASWVQEKPGKLFK GLIGGTNNRAPGVPSRFSGSLIGDKATLTISSLQPEDFATYFCALWYSNHW VFGQGTKVELKRGGGGSGGGGSGGGGSSGGGSEVKLLESGGGLVQPG GSLKLSCAVSGFSLTDYGVNWVRQAPGRGLEWIGVIWGDGITDYNSALK DRFIISKDNGKNTVYLQMSKVRSDDTALYYCVTGLFDYWGQGTLVTVSSY PYDVPDYAGGGGGSGGGGSGGGGSGGGGSSLDPGGGGSG<TGSAAI AEVLLNARCDLHAVNYHGDTPLHIAARESYHDCVLLFLSRGANP ELRNKEGDTAWDLTPERSDVWFALQLNRKLRLGVGNRAIRTEKI ICRDVARGYENVPIPCVNGVDGEPCPEDYKYISENCETSTMNIDR NITHLQHCTCVDDCSSSNCLCGQLSIRCWYDKDGRLLQEFNKIEP PLIFECNQACSCWRNCKNRVVQSGIKVRLQLYRTAKMGWGVRA LQTIPQGTFICEYVGELISDAEADVREDDSYLFDLDNKDGEVYCID ARYYGNISRFINHLCDPNIIPVRVFMLHQDLRFPRIAFFSSRDIRTG EELGFDYGDRFWDIKSKYFTCQCGSEKCKHSAEAIALEQSRLAR LDPHPELLPELGSLPPVNT>GSGKRTADGSEFESPKKKRKV |
| 67 | scFv-SUV39 H1 | MGPDIVMTQSPSSLSASVGDRVTITCRSSTGAVTTSNYASWVQEKPGKLFK GLIGGTNNRAPGVPSRFSGSLIGDKATLTISSLQPEDFATYFCALWYSNHW VFGQGTKVELKRGGGGSGGGGSGGGGSSGGGSEVKLLESGGGLVQPG GSLKLSCAVSGFSLTDYGVNWVRQAPGRGLEWIGVIWGDGITDYNSALK DRFIISKDNGKNTVYLQMSKVRSDDTALYYCVTGLFDYWGQGTLVTVSSY PYDVPDYAGGGGGSGGGGSGGGGSGGGGSSLDPGGGGSG<AENLK GCSVCCKSSWNQLQDLCRLAKLSCPALGISKRNLYDFEVEYLCD YKKIREQEYYLVKWRGYPDSESTWEPRQNLKCVRILKQFHKDL ERELLRRHHRSKTPRHLDPSLANYLVQKAKQRRALRRWEQELN AKRSHLGRITVENEVDLDGPPRAFVYINEYRVGEGITLNQVAVGC ECQDCLWAPTGGCCPGASLHKFAYNDQGQVRLRAGLPIYECNS RCRCGYDCPNRVVQKGIRYDLCIFRTDDGRGWGVRTLEKIRKNS FVMEYVGEIITSEEAERRGQIYDRQGATYLFDLDYVEDVYTVDA AYYGNISHFVNHSCDPNLQVYNVFIDNLDERLPRIAFFATRTIRAG EELTFDYNMQVDPVDMESTRMDSNFGLAGLPGSPKKRVRIECKC GTESCRKYLF>GSGKRTADGSEFESPKKKRKV |
| 68 | scFv-ZFPM1 (FOG) | MGPDIVMTQSPSSLSASVGDRVTITCRSSTGAVTTSNYASWVQEKPGKLFK GLIGGTNNRAPGVPSRFSGSLIGDKATLTISSLQPEDFATYFCALWYSNHW VFGQGTKVELKRGGGGSGGGGSGGGGSSGGGSEVKLLESGGGLVQPG GSLKLSCAVSGFSLTDYGVNWVRQAPGRGLEWIGVIWGDGITDYNSALK DRFIISKDNGKNTVYLQMSKVRSDDTALYYCVTGLFDYWGQGTLVTVSSY PYDVPDYAGGGGGGSGGGGSGGGGSGGGGSSLDPGGGGSG<SRRKQ SNPRQIKRSLGDMEAREEVQLVGASHMEQKATAPEAPSPPSA >GSGKRTADGSEFESPKKKRKV |
| 69 | scFv-TRIM2 8(KAP 1) | MGPDIVMTQSPSSLSASVGDRVTITCRSSTGAVTTSNYASWVQEKPGKLFK GLIGGTNNRAPGVPSRFSGSLIGDKATLTISSLQPEDFATYFCALWYSNHW VFGQGTKVELKRGGGGSGGGGSGGGGSSGGGSEVKLLESGGGLVQPG GSLKLSCAVSGFSLTDYGVNWVRQAPGRGLEWIGVIWGDGITDYNSALK DRFIISKDNGKNTVYLQMSKVRSDDTALYYCVTGLFDYWGQGTLVTVSSY PYDVPDYAGGGGGSGGGGSGGGGSGGGGSSLDPGGGGSG<AASAA AASAAAASAASGSPGPGEGSAGGEKRSTAPSAAASASASAAASSP AGGGAEALELLEHCGVCRERLRPEREPRLLPCLHSACSACLGPA APAAANSSGDGGAAGDGTVVDCPVCKQQCFSKDIVENYFMRDS GSKAATDAQDANQCCTSCEDNAPATSYCVECSEPLCETCVEAHQ RVKYTKDHTVRSTGPAKSRDGERTVYCNVHKHEPLVLFCESCDT LTCRDCQLNAHKDHQYQFLEDAVRNQRKLLASLVKRLGDKHAT LQKSTKEVRSSIRQVSDVQKRVQVDVKMAILQIMKELNKRGRVL VNDAQKVTEGQQERLERQHWTMTKIQKHQEHILRFASWALESD NNTALLLSKKLIYFQLHRALKMIVDPVEPHGEMKFQWDLNAWT KSAEAFGKIVAERPGTNSTGPAPMAPPRAPGPLSKQGSGSSQPME VQEGYGFGSGDDPYSSAEPHVSGVKRSRSGEGEVSGLMRKVPRV SLERLDLDLTADSQPPVFKVFPGSTTEDYNLIVIERGAAAAATGQ PGTAPAGTPGAPPLAGMAIVKEEETEAAIGAPPTATEGPETKPVL MALAEGPGAEGPRLASPSGSTSSGLEVVAPEGTSAPGGGPGTLD DSATICRVCQKPGDLVMCNQCEFCFHLDCHLPALQDVPGEEWS CSLCHVLPDLKEEDGSLSLDGADSTGVVAKLSPANQRKCERVLL ALFCHEPCRPLHQLATDSTFSLDQPGGTLDLTLIRARLQEKLSPP YSSPQEFAQDVGRMFKQFNKLTEDKADVQSIIGLQRFFETRMNE AFGDTKFSAVLVEPPPMSLPGAGLSSQELSGGPGDGP>GSGKRTA DGSEFESPKKKRKV |
| 70 | scFv-EZH2 | MGPDIVMTQSPSSLSASVGDRVTITCRSSTGAVTTSNYASWVQEKPGKLFK GLIGGTNNRAPGVPSRFSGSLIGDKATLTISSLQPEDFATYFCALWYSNHW VFGQGTKVELKRGGGGSGGGGSGGGGSSGGGSEVKLLESGGGLVQPG GSLKLSCAVSGFSLTDYGVNWVRQAPGRGLEWIGVIWGDGITDYNSALK DRFIISKDNGKNTVYLQMSKVRSDDTALYYCVTGLFDYWGQGTLVTVSSY PYDVPDYAGGGGGSGGGGSGGGGSGGGGSSLDPGGGGSG<GQTGK KSEKGPVCWRKRVKSEYMRLRQLKRFRRADEVKSMFSSNRQKI LERTEILNQEWKQRRIQPVHILTSVSSLRGTRECSVTSDLDFPTQV IPLKTLNAVASVPIMYSWSPLQQNFMVEDETVLHNIPYMGDEVL DQDGTFIEELIKNYDGKVHGDRECGFINDEIFVELVNALGQYNDD DDDDDGDDPEEREEKQKDLEDHRDDKESRPPRKFPSDKIFEAISS MFPDKGTAEELKEKYKELTEQQLPGALPPECTPNIDGPNAKSVQ REQSLHSFHTLFCRRCFKYDCFLHPFHATPNTYKRKNTETALDN KPCGPQCYQHLEGAKEFAAALTAERIKTPPKRPGGRRRGRLPNN SSRPSTPTINVLESKDTDSDREAGTETGGENNDKEEEEKKDETSSS SEANSRCQTPIKMKPNIEPPENVEWSGAEASMFRVLIGTYYDNFC AIARLIGTKTCRQVYEFRVKESSIIAPAPAEDVDTPPRKKKRKHR LWAAHCRKIQLKKDGSSNHVYNYQPCDHPRQPCDSSCPCVIAQN FCEKFCQCSSECQNRFPGCRCKAQCNTKQCPCYLAVRECDPDLC LTCGAADHWDSKNVSCKNCSIQRGSKKHLLLAPSDVAGWGIFIK DPVQKNEFISEYCGEIISQDEADRRGKVYDKYMCSFLFNLNNDFV VDATRKGNKIRFANHSVNPNCYAKVMMVNGDHRIGIFAKRAIQT GEELFFDYRYSQADALKYVGIEREMEIP>GSGKRTADGSEFESPKK KRKV |
| 71 | scFv-MXD1( SID FL) | MGPDIVMTQSPSSLSASVGDRVTITCRSSTGAVTTSNYASWVQEKPGKLFK GLIGGTNNRAPGVPSRFSGSLIGDKATLTISSLQPEDFATYFCALWYSNHW VFGQGTKVELKRGGGGSGGGGSGGGGSSGGGSEVKLLESGGGLVQPG GSLKLSCAVSGFSLTDYGVNWVRQAPGRGLEWIGVIWGDGITDYNSALK DRFIISKDNGKNTVYLQMSKVRSDDTALYYCVTGLFDYWGQGTLVTVSSY PYDVPDYAGGGGGSGGGGSGGGGSGGGGSSLDPGGGGSG<AAAVR MNIQMLLEAADYLERREREAEHGYASMLPYNNKDRDALKRRNK SKKNNSSSRSTHNEMEKNRRAHLRLCLEKLKGLVPLGPESSRHT TLSLLTKAKLHIKKLEDCDRKAVHQIDQLQREQRHLKRQLEKL GIERIRMDSIGSTVSSERSDSDREIDVDVESTDYLTGDLDWSSSSVS DSDERGSMQSLGSDEGYSSTSIKRIKLQDSHKACLGL>GSGKRTA DGSEFESPKKKRKV |
| 72 | scFv-SID | MGPDIVMTQSPSSLSASVGDRVTITCRSSTGAVTTSNYASWVQEKPGKLFK GLIGGTNNRAPGVPSRFSGSLIGDKATLTISSLQPEDFATYFCALWYSNHW VFGQGTKVELKRGGGGSGGGGSGGGGSSGGGSEVKLLESGGGLVQPG GSLKLSCAVSGFSLTDYGVNWVRQAPGRGLEWIGVIWGDGITDYNSALK DRFIISKDNGKNTVYLQMSKVRSDDTALYYCVTGLFDYWGQGTLVTVSSY PYDVPDYAGGGGGSGGGGSGGGGSGGGGSSLDPGGGGSG<MNIQM LLEAADYLERREREAEHGYASMLP >GSGKRTADGSEFESPKKKRKV |
| 73 | scFv-LSD1( KDMA 1) | MGPDIVMTQSPSSLSASVGDRVTITCRSSTGAVTTSNYASWVQEKPGKLFK GLIGGTNNRAPGVPSRFSGSLIGDKATLTISSLQPEDFATYFCALWYSNHW VFGQGTKVELKRGGGGSGGGGSGGGGSSGGGSEVKLLESGGGLVQPG GSLKLSCAVSGFSLTDYGVNWVRQAPGRGLEWIGVIWGDGITDYNSALK DRFIISKDNGKNTVYLQMSKVRSDDTALYYCVTGLFDYWGQGTLVTVSSY PYDVPDYAGGGGGSGGGGSGGGGSGGGGSSLDPGGGGSG<LSGKK AAAAAAAAAAATGTEAGPGTAGGSENGSEVAAQPAGLSGPAE VGPGAVGERTPRKKEPPRASPPGGLAEPPGSAGPQAGPTVVPGS ATPMETGIAETPEGRRTSRRKRAKVEYREMDESLANLSEDEYYS EEERNAKAEKEKKLPPPPPQAPPEEENESEPEEPSGQAGGLQDDS SGGYGDGQASGVEGAAFQSRLPHDRMTSQEAACFPDIISGPQQT QKVFLFIRNRTLQLWLDNPKIQLTFEATLQQLEAPYNSDTVLVH RVHSYLERHGLINFGIYKRIKPLPTKKTGKVIIIGSGVSGLAAARQ LQSFGMDVTLLEARDRVGGRVATFRKGNYVADLGAMVVTGLG GNPMAVVSKQVNMELAKIKQKCPLYEANGQADTVKVPKEKDE MVEQEFNRLLEATSYLSHQLDFNVLNNKPVSLGQALEVVIQLQE KHVKDEQIEHWKKIVKTQEELKELLNKMVNLKEKIKELHQQYK EASEVKPPRDITAEFLVKSKHRDLTALCKEYDELAETQGKLEEK LQELEANPPSDVYLSSRDRQILDWHFANLEFANATPLSTLSLKHW DQDDDFEFTGSHLTVRNGYSCVPVALAEGLDIKLNTAVRQVRYT ASGCEVIAVNTRSTSQTFIYKCDAVLCTLPLGVLKQQPPAVQFVP PLPEWKTSAVQRMGFGNLNKVVLCFDRVFWDPSVNLFGHVGST TASRGELFLFWNLYKAPILLALVAGEAAGIMENISDDVIVGRCLA ILKGIFGSSAVPQPKETVVSRWRADPWARGSYSYVAAGSSGNDY DLMAQPITPGPSIPGAPQPIPRLFFAGEHTIRNYPATVHGALLSGL REAGRIADQFLGAMYTLPRQATPGVPAQQSPSM>GSGKRTADGS EFESPKKKRKV |
| 74 | scFv-HP1a long | MGPDIVMTQSPSSLSASVGDRVTITCRSSTGAVTTSNYASWVQEKPGKLFK GLIGGTNNRAPGVPSRFSGSLIGDKATLTISSLQPEDFATYFCALWYSNHW VFGQGTKVELKRGGGGSGGGGSGGGGSSGGGSEVKLLESGGGLVQPG GSLKLSCAVSGFSLTDYGVNWVRQAPGRGLEWIGVIWGDGITDYNSALK DRFIISKDNGKNTVYLQMSKVRSDDTALYYCVTGLFDYWGQGTLVTVSSY PYDVPDYAGGGGGSGGGGSGGGGSGGGGSSLDPGGGGSG<GKKTK RTADSSSSEDEEEYVVEKVLDRRVVKGQVEYLLKWKGFSEEHNT WEPEKNLDCPELISEFMKKYKKMKEGENNKPREKSESNKRKSN FSNSADDIKSKKKREQSNDIARGFERGLEPEKIIGATDSCGDLMFL MKWKDTDEADLVLAKEANVKCPQIVIAFYEERLTWHAYPEDAE NKEKETAKS >GSGKRTADGSEFESPKKKRKV |
| 75 | scFv-HP1a short | MGPDIVMTOSPSSLSASVGDRVTITCRSSTGAVTTSNYASWVOEKPGKLFK GLIGGTNNRAPGVPSRFSGSLIGDKATLTISSLQPEDFATYFCALWYSNHW VFGQGTKVELKRGGGGSGGGGSGGGGSSGGGSEVKLLESGGGLVQPG GSLKLSCAVSGFSLTDYGVNWVRQAPGRGLEWIGVIWGDGITDYNSALK DRFIISKDNGKNTVYLQMSKVRSDDTALYYCVTGLFDYWGQGTLVTVSSY PYDVPDYAGGGGGSGGGGSGGGGSGGGGSSLDPGGGGSG<MKEGE NNKPREKSESNKRKSNFSNSADDIKSKKKREQSNDIARGFERGLE PEKIIGATDSCGDLMFLMKWKDTDEADLVLAKEANVKCPQIVIA FYEERLTWHAYPEDAENKEKETAKS >GSGKRTADGSEFESPKKKRKV |
| 76 | scFv-HDAC 3 | MGPDIVMTQSPSSLSASVGDRVTITCRSSTGAVTTSNYASWVQEKPGKLFK GLIGGTNNRAPGVPSRFSGSLIGDKATLTISSLQPEDFATYFCALWYSNHW VFGQGTKVELKRGGGGSGGGGSGGGGSSGGGSEVKLLESGGGLVQPG GSLKLSCAVSGFSLTDYGVNWVRQAPGRGLEWIGVIWGDGITDYNSALK DRFIISKDNGKNTVYLQMSKVRSDDTALYYCVTGLFDYWGQGTLVTVSSY PYDVPDYAGGGGGSGGGGSGGGGSGGGGSSLDPGGGGSG<AKTVA YFYDPDVGNFHYGAGHPMKPHRLALTHSLVLHYGLYKKMIVFK PYQASQHDMCRFHSEDYIDFLQRVSPTNMQGFTKSLNAFNVGDD CPVFPGLFEFCSRYTGASLQGATQLNNKICDIAINWAGGLHHAK KFEASGFCYVNDIVIGILELLKYHPRVLYIDIDIHHGDGVQEAFYL TDRVMTVSFHKYGNYFFPGTGDMYEVGAESGRYYCLNVPLRDG IDDQSYKHLFQPVINQVVDFYQPTCIVLQCGADSLGCDRLGCFNL SIRGHGECVEYVKSFNIPLLVLGGGGYTVRNVARCWTYETSLLV EEAISEELPYSEYFEYFAPDFTLHPDVSTRIENQNSRQYLDQIRQTI FENLKMLNHAPSVQIHDVPADLLTYDRTDEADAEERGPEENYSR PEAPNEFYDGDHDNDKESDVEI >GSGKRTADGSEFESPKKKRKV |
| 77 | 3xGCN 4-dCas9-3xGCN 4 | MPAAKRVKLDGSEELLSKNYHLENEVARLKKGSGSGGSGSGGSGSGS GGSGSGGSGSGEELLSKNYHLENEVARLKKGSGSGGSGSGGSGSGSGG SGSGGSGSGEELLSKNYHLENEVARLKKGSGSGGSGSGGSGSGSGGSG SGGSGSGPKKKRKVMDKKYSIGLAIGTNSVGWAVITDEYKVPSKK FKVLGNTDRHSIKKNLIGALLFDSGETAEATRLKRTARRRYTRR KNRICYLQEIFSNEMAKVDDSFFHRLEESFLVEEDKKHERHPIFG NIVDEVAYHEKYPTIYHLRKKLVDSTDKADLRLIYLALAHMIKFR GHFLIEGDLNPDNSDVDKLFIQLVQTYNQLFEENPINASGVDAKA ILSARLSKSRRLENLIAQLPGEKKNGLFGNLIALSLGLTPNFKSNF DLAEDAKLQLSKDTYDDDLDNLLAQIGDQYADLFLAAKNLSDAI LLSDILRVNTEITKAPLSASMIKRYDEHHQDLTLLKALVRQQLPE KYKEIFFDQSKNGYAGYIDGGASQEEFYKFIKPILEKMDGTEELL VKLNREDLLRKQRTFDNGSIPHQIHLGELHAILRRQEDFYPFLKD NREKIEKILTFRIPYYVGPLARGNSRFAWMTRKSEETITPWNFEE VVDKGASAQSFIERMTNFDKNLPNEKVLPKHSLLYEYFTVYNEL TKVKYVTEGMRKPAFLSGEQKKAIVDLLFKTNRKVTVKQLKED YFKKIECFDSVEISGVEDRFNASLGTYHDLLKIIKDKDFLDNEENE DILEDIVLTLTLFEDREMIEERLKTYAHLFDDKVMKQLKRRRYT GWGRLSRKLINGIRDKQSGKTILDFLKSDGFANRNFMQLIHDDSL TFKEDIQKAQVSGQGDSLHEHIANLAGSPAIKKGILQTVKVVDEL VKVMGRHKPENIVIEMARENQTTQKGQKNSRERMKRIEEGIKE LGSQILKEHPVENTQLQNEKLYLYYLQNGRDMYVDQELDINRLS DYDVDAIVPQSFLKDDSIDNKVLTRSDKNRGKSDNVPSEEVVKK MKNYWRQLLNAKLITQRKFDNLTKAERGGLSELDKAGFIKRQL VETRQITKHVAQILDSRMNTKYDENDKLIREVKVITLKSKLVSDF RKDFQFYKVREINNYHHAHDAYLNAVVGTALIKKYPKLESEFVY GDYKVYDVRKMIAKSEQEIGKATAKYFFYSNIMNFFKTEITLAN GEIRKRPLIETNGETGEIVWDKGRDFATVRKVLSMPQVNIVKKT EVQTGGFSKESILPKRNSDKLIARKKDWDPKKYGGFDSPTVAYS VLVVAKVEKGKSKKLKSVKELLGITIMERSSFEKNPIDFLEAKGY KEVKKDLIIKLPKYSLFELENGRKRMLASAGELQKGNELALPSK YVNFLYLASHYEKLKGSPEDNEQKQLFVEQHKHYLDEIIEQISEF SKRVILADANLDKVLSAYNKHRDKPIREQAENIIHLFTLTNLGAP AAFKYFDTTIDRKRYTSTKEVLDATLIHQSITGLYETRIDLSQLGG DAYPYDVPDYASLGSGSPKKKRKVEDPKKKRKVDGSGSETPGTSGG SGSGSGGSGSGGSGSGEELLSKNYHLENEVARLKKGSGSGGSGSGGSG SGSGGSGSGGSGSGEELLSKNYHLENEVARLKKGSGSGGSGSGGSGSG SGGSGSGGSGSGEELLSKNYHLENEVARLKKGSGSGQRGSKRTADGSE FESPKKKRKV* |
| 78 | scFv-KRAB | MPAAKRVKLDGS*GPDIVMTQSPSSLSASVGDRVTITCRSSTGAVTTSNYA* *SWVQEKPGKLFKGLIGGTNNRAPGVPSRFSGSLIGDKATLTISSLQPEDF* *ATYFCALWYSNHWVFGQGTKVELKRGGGGSGGGGSGGGGSSGGGSEV* *KLLESGGGLVQPGGSLKLSCAVSGFSLTDYGVNWVRQAPGRGLEWIGVI* *WGDGITDYNSALKDRFIISKDNGKNTVYLQMSKVRSDDTALYYCVTGLFD* *YWGQGTLVTVSS*YPYDVPDYAGGGGGSGGGGSGGGGSGGGGSLDPG GGGSG<**RTLVTFKDVFVDFTREEWKLLDTAQQIVYRNVMLENYK** **NLVSLGYQLTKPDVILRLEKGEEP**>* |
| 79 | DNMM T3A-DNMT 3L-dCas9-1xGCN 4 | MPAAKRVKLDGSNHDQEFDPPKVYPPVPAEKRKPIRVLSLFDGIATG LLVLKDLGIQVDRYIASEVCEDSITVGMVRHQGKIMYVGDVRSVTQK HIQEWGPFDLVIGGSPCNDLSIVNPARKGLYEGTGRLFFEFYRLLHD ARPKEGDDRPFFWLFENVVAMGVSDKRDISRFLESNPVMIDAKEVS AAHRARYFWGNLPGMNRPLASTVNDKLELQECLEHGRIAKFSKVRT ITTRSNSIKQGKDQHFPVFMNEKEDILWCTEMERVFGFPVHYTDVS NMSRLARQRLLGRSWSVPVIRHLFAPLKEYFACVSSGNSNANSRGPS FSSGLVPLSLRGSHMGPMEIYKTVSAWKRQPVRVLSLFRNIDKVLKSL GFLESGSGSGGGTLKYVEDVTNVVRRDVEKWGPFDLVYGSTQPLGS SCDRCPGWYMFQFHRILQYALPRQESQRPFFWIFMDNLLLTEDDQE TTTRFLQTEAVTLQDVRGRDYQNAMRVWSNIPGLKSKHAPLTPKEE EYLQAQVRSRSKLDAPKVDLLVKNCLLPLREYFKYFSQNSLPLGGPSS GAPPPSGGSPAGSPTSTEEGTSESATPESGPGTSTEPSEGSAPGSPAGSP TSTEEGTSTEPSEGSAPGTSTEPSEPKKKRKVMDKKYSIGLAIGTNSV GWAVITDEYKVPSKKFKVLGNTDRHSIKKNLIGALLFDSGETAE ATRLKRTARRRYTRRKNRICYLQEIFSNEMAKVDDSFFHRLEESF LVEEDKKHERHPIFGNIVDEVAYHEKYPTIYHLRKKLVDSTDKA DLRLIYLALAHMIKFRGHFLIEGDLNPDNSDVDKLFIQLVQTYNQ LFEENPINASGVDAKAILSARLSKSRRLENLIAQLPGEKKNGLFG NLIALSLGLTPNFKSNFDLAEDAKLQLSKDTYDDDLDNLLAQIGD QYADLFLAAKNLSDAILLSDILRVNTEITKAPLSASMIKRYDEHH QDLTLLKALVRQQLPEKYKEIFFDQSKNGYAGYIDGGASQEEFY KFIKPILEKMDGTEELLVKLNREDLLRKQRTFDNGSIPHQIHLGE LHAILRRQEDFYPFLKDNREKIEKILTFRIPYYVGPLARGNSRFA WMTRKSEETITPWNFEEVVDKGASAQSFIERMTNFDKNLPNEKV LPKHSLLYEYFTVYNELTKVKYVTEGMRKPAFLSGEQKKAIVDL LFKTNRKVTVKQLKEDYFKKIECFDSVEISGVEDRFNASLGTYHD LLKIIKDKDFLDNEENEDILEDIVLTLTLFEDREMIEERLKTYAHL FDDKVMKQLKRRRYTGWGRLSRKLINGIRDKQSGKTILDFLKSD GFANRNFMQLIHDDSLTFKEDIQKAQVSGQGDSLHEHIANLAGSP AIKKGILQTVKVVDELVKVMGRHKPENIVIEMARENQTTQKGQ KNSRERMKRIEEGIKELGSQILKEHPVENTQLQNEKLYLYYLQN GRDMYVDQELDINRLSDYDVDAIVPQSFLKDDSIDNKVLTRSDKN RGKSDNVPSEEVVKKMKNYWRQLLNAKLITQRKFDNLTKAERG GLSELDKAGFIKRQLVETRQITKHVAQILDSRMNTKYDENDKLIR EVKVITLKSKLVSDFRKDFQFYKVREINNYHHAHDAYLNAVVGT ALIKKYPKLESEFVYGDYKVYDVRKMIAKSEQEIGKATAKYFFY SNIMNFFKTEITLANGEIRKRPLIETNGETGEIVWDKGRDFATVR KVLSMPQVNIVKKTEVQTGGFSKESILPKRNSDKLIARKKDWDP KKYGGFDSPTVAYSVLVVAKVEKGKSKKLKSVKELLGITIMERS SFEKNPIDFLEAKGYKEVKKDLIIKLPKYSLFELENGRKRMLASA GELQKGNELALPSKYVNFLYLASHYEKLKGSPEDNEQKQLFVEQ HKHYLDEIIEQISEFSKRVILADANLDKVLSAYNKHRDKPIREQAE NIIHLFTLTNLGAPAAFKYFDTTIDRKRYTSTKEVLDATLIHQSIT GLYETRIDLSQLGGDAYPYDVPDYASLGSGSPKKKRKVEDPKKKR KVDGIGSGSNGSSGEELLSKNYHLENEVARLKKGSGSGGSGSGGSGSG SGGSGSGGSGSGEELLSKNYHGSGSGQRGSKRTADGSEFESPKKKRK V* |
| 80 | DNMT 3A-DNMT 3L-dCas9-2xGCN 4 | MPAAKRVKLDGSNHDQEFDPPKVYPPVPAEKRKPIRVLSLFDGIATG LLVLKDLGIQVDRYIASEVCEDSITVGMVRHQGKIMYVGDVRSVTQK HIQEWGPFDLVIGGSPCNDLSIVNPARKGLYEGTGRLFFEFYRLLHD ARPKEGDDRPFFWLFENVVAMGVSDKRDISRFLESNPVMIDAKEVS AAHRARYFWGNLPGMNRPLASTVNDKLELQECLEHGRIAKFSKVRT ITTRSNSIKQGKDQHFPVFMNEKEDILWCTEMERVFGFPVHYTDVS NMSRLARQRLLGRSWSVPVIRHLFAPLKEYFACVSSGNSNANSRGPS FSSGLVPLSLRGSHMGPMEIYKTVSAWKRQPVRVLSLFRNIDKVLKSL GFLESGSGSGGGTLKYVEDVTNVVRRDVEKWGPFDLVYGSTQPLGS SCDRCPGWYMFQFHRILQYALPRQESQRPFFWIFMDNLLLTEDDQE TTTRFLQTEAVTLQDVRGRDYQNAMRVWSNIPGLKSKHAPLTPKEE EYLQAQVRSRSKLDAPKVDLLVKNCLLPLREYFKYFSQNSLPLGGPSS GAPPPSGGSPAGSPTSTEEGTSESATPESGPGTSTEPSEGSAPGSPAGSP TSTEEGTSTEPSEGSAPGTSTEPSEPKKKRKVMDKKYSIGLAIGTNSV GWAVITDEYKVPSKKFKVLGNTDRHSIKKNLIGALLFDSGETAE ATRLKRTARRRYTRRKNRICYLQEIFSNEMAKVDDSFFHRLEESF LVEEDKKHERHPIFGNIVDEVAYHEKYPTIYHLRKKLVDSTDKA DLRLIYLALAHMIKFRGHFLIEGDLNPDNSDVDKLFIQLVQTYNQ LFEENPINASGVDAKAILSARLSKSRRLENLIAQLPGEKKNGLFG NLIALSLGLTPNFKSNFDLAEDAKLQLSKDTYDDDLDNLLAQIGD QYADLFLAAKNLSDAILLSDILRVNTEITKAPLSASMIKRYDEHH QDLTLLKALVRQQLPEKYKEIFFDQSKNGYAGYIDGGASQEEFY KFIKPILEKMDGTEELLVKLNREDLLRKQRTFDNGSIPHQIHLGE LHAILRRQEDFYPFLKDNREKIEKILTFRIPYYVGPLARGNSRFA WMTRKSEETITPWNFEEVVDKGASAQSFIERMTNFDKNLPNEKV LPKHSLLYEYFTVYNELTKVKYVTEGMRKPAFLSGEQKKAIVDL LFKTNRKVTVKQLKEDYFKKIECFDSVEISGVEDRFNASLGTYHD LLKIIKDKDFLDNEENEDILEDIVLTLTLFEDREMIEERLKTYAHL FDDKVMKQLKRRRYTGWGRLSRKLINGIRDKQSGKTILDFLKSD GFANRNFMQLIHDDSLTFKEDIQKAQVSGQGDSLHEHIANLAGSP AIKKGILQTVKVVDELVKVMGRHKPENIVIEMARENQTTQKGQ KNSRERMKRIEEGIKELGSQILKEHPVENTQLQNEKLYLYYLQN GRDMYVDQELDINRLSDYDVDAIVPQSFLKDDSIDNKVLTRSDKN RGKSDNVPSEEVVKKMKNYWRQLLNAKLITQRKFDNLTKAERG GLSELDKAGFIKRQLVETRQITKHVAQILDSRMNTKYDENDKLIR EVKVITLKSKLVSDFRKDFQFYKVREINNYHHAHDAYLNAVVGT ALIKKYPKLESEFVYGDYKVYDVRKMIAKSEQEIGKATAKYFFY SNIMNFFKTEITLANGEIRKRPLIETNGETGEIVWDKGRDFATVR KVLSMPQVNIVKKTEVQTGGFSKESILPKRNSDKLIARKKDWDP KKYGGFDSPTVAYSVLVVAKVEKGKSKKLKSVKELLGITIMERS SFEKNPIDFLEAKGYKEVKKDLIIKLPKYSLFELENGRKRMLASA GELQKGNELALPSKYVNFLYLASHYEKLKGSPEDNEQKQLFVEQ HKHYLDEIIEQISEFSKRVILADANLDKVLSAYNKHRDKPIREQAE NIIHLFTLTNLGAPAAFKYFDTTIDRKRYTSTKEVLDATLIHQSIT GLYETRIDLSQLGGDAYPYDVPDYASLGSGSPKKKRKVEDPKKKR KVDGIGSGSNGSSGEELLSKNYHLENEVARLKKGSGSGGSGSGGSGSG SGGSGSGGSGSGEELLSKNYHLENEVARLKKGSGSGQRGSKRTADGSE FESPKKKRKV* |
| 81 | DNMT 3A-DNMT 3L-dCas9-4xGCN 4 | MPAAKRVKLDGSNHDQEFDPPKVYPPVPAEKRKPIRVLSLFDGIATG LLVLKDLGIQVDRYIASEVCEDSITVGMVRHQGKIMYVGDVRSVTQK HIQEWGPFDLVIGGSPCNDLSIVNPARKGLYEGTGRLFFEFYRLLHD ARPKEGDDRPFFWLFENVVAMGVSDKRDISRFLESNPVMIDAKEVS AAHRARYFWGNLPGMNRPLASTVNDKLELQECLEHGRIAKFSKVRT ITTRSNSIKQGKDQHFPVFMNEKEDILWCTEMERVFGFPVHYTDVS NMSRLARQRLLGRSWSVPVIRHLFAPLKEYFACVSSGNSNANSRGPS FSSGLVPLSLRGSHMGPMEIYKTVSAWKRQPVRVLSLFRNIDKVLKSL GFLESGSGSGGGTLKYVEDVTNVVRRDVEKWGPFDLVYGSTQPLGS SCDRCPGWYMFQFHRILQYALPRQESQRPFFWIFMDNLLLTEDDQE TTTRFLQTEAVTLQDVRGRDYQNAMRVWSNIPGLKSKHAPLTPKEE EYLQAQVRSRSKLDAPKVDLLVKNCLLPLREYFKYFSQNSLPLGGPSS GAPPPSGGSPAGSPTSTEEGTSESATPESGPGTSTEPSEGSAPGSPAGSP TSTEEGTSTEPSEGSAPGTSTEPSEPKKKRKVMDKKYSIGLAIGTNSV GWAVITDEYKVPSKKFKVLGNTDRHSIKKNLIGALLFDSGETAE ATRLKRTARRRYTRRKNRICYLQEIFSNEMAKVDDSFFHRLEESF LVEEDKKHERHPIFGNIVDEVAYHEKYPTIYHLRKKLVDSTDKA DLRLIYLALAHMIKFRGHFLIEGDLNPDNSDVDKLFIQLVQTYNQ LFEENPINASGVDAKAILSARLSKSRRLENLIAQLPGEKKNGLFG NLIALSLGLTPNFKSNFDLAEDAKLQLSKDTYDDDLDNLLAQIGD QYADLFLAAKNLSDAILLSDILRVNTEITKAPLSASMIKRYDEHH QDLTLLKALVRQQLPEKYKEIFFDQSKNGYAGYIDGGASQEEFY KFIKPILEKMDGTEELLVKLNREDLLRKQRTFDNGSIPHQIHLGE LHAILRRQEDFYPFLKDNREKIEKILTFRIPYYVGPLARGNSRFA WMTRKSEETITPWNFEEVVDKGASAQSFIERMTNFDKNLPNEKV LPKHSLLYEYFTVYNELTKVKYVTEGMRKPAFLSGEQKKAIVDL LFKTNRKVTVKQLKEDYFKKIECFDSVEISGVEDRFNASLGTYHD LLKIIKDKDFLDNEENEDILEDIVLTLTLFEDREMIEERLKTYAHL FDDKVMKQLKRRRYTGWGRLSRKLINGIRDKQSGKTILDFLKSD GFANRNFMQLIHDDSLTFKEDIQKAQVSGQGDSLHEHIANLAGSP AIKKGILQTVKVVDELVKVMGRHKPENIVIEMARENQTTQKGQ KNSRERMKRIEEGIKELGSQILKEHPVENTQLQNEKLYLYYLQN GRDMYVDQELDINRLSDYDVDAIVPQSFLKDDSIDNKVLTRSDKN RGKSDNVPSEEVVKKMKNYWRQLLNAKLITQRKFDNLTKAERG GLSELDKAGFIKRQLVETRQITKHVAQILDSRMNTKYDENDKLIR EVKVITLKSKLVSDFRKDFQFYKVREINNYHHAHDAYLNAVVGT ALIKKYPKLESEFVYGDYKVYDVRKMIAKSEQEIGKATAKYFFY SNIMNFFKTEITLANGEIRKRPLIETNGETGEIVWDKGRDFATVR KVLSMPQVNIVKKTEVQTGGFSKESILPKRNSDKLIARKKDWDP KKYGGFDSPTVAYSVLVVAKVEKGKSKKLKSVKELLGITIMERS SFEKNPIDFLEAKGYKEVKKDLIIKLPKYSLFELENGRKRMLASA GELQKGNELALPSKYVNFLYLASHYEKLKGSPEDNEQKQLFVEQ HKHYLDEIIEQISEFSKRVILADANLDKVLSAYNKHRDKPIREQAE NIIHLFTLTNLGAPAAFKYFDTTIDRKRYTSTKEVLDATLIHQSIT GLYETRIDLSQLGGDAYPYDVPDYASLGSGSPKKKRKVEDPKKKR KVDGIGSGSNGSSGEELLSKNYHLENEVARLKKGSGSGGSGSGGSGSG SGGSGSGGSGSGEELLSKNYHLENEVARLKKGSGSGGSGSGGSGSGSG GSGSGGSGSGEELLSKNYHLENEVARLKKGSGSGGSGSGGSGSGSGGS GSGGSGSGEELLSKNYHLENEVARLKKGSGSGQRGSKRTADGSEFESP KKKRKV* |
| 82 | DNMT 3A-DNMT 3L-dCas9-10xGC N4 | MPAAKRVKLDGSNHDQEFDPPKVYPPVPAEKRKPIRVLSLFDGIATG LLVLKDLGIQVDRYIASEVCEDSITVGMVRHQGKIMYVGDVRSVTQK HIQEWGPFDLVIGGSPCNDLSIVNPARKGLYEGTGRLFFEFYRLLHD ARPKEGDDRPFFWLFENVVAMGVSDKRDISRFLESNPVMIDAKEVS AAHRARYFWGNLPGMNRPLASTVNDKLELQECLEHGRIAKFSKVRT ITTRSNSIKQGKDQHFPVFMNEKEDILWCTEMERVFGFPVHYTDVS NMSRLARQRLLGRSWSVPVIRHLFAPLKEYFACVSSGNSNANSRGPS FSSGLVPLSLRGSHMGPMEIYKTVSAWKRQPVRVLSLFRNIDKVLKSL GFLESGSGSGGGTLKYVEDVTNVVRRDVEKWGPFDLVYGSTQPLGS SCDRCPGWYMFQFHRILQYALPRQESQRPFFWIFMDNLLLTEDDQE TTTRFLQTEAVTLQDVRGRDYQNAMRVWSNIPGLKSKHAPLTPKEE EYLQAQVRSRSKLDAPKVDLLVKNCLLPLREYFKYFSQNSLPLGGPSS GAPPPSGGSPAGSPTSTEEGTSESATPESGPGTSTEPSEGSAPGSPAGSP TSTEEGTSTEPSEGSAPGTSTEPSEPKKKRKVMDKKYSIGLAIGTNSV GWAVITDEYKVPSKKFKVLGNTDRHSIKKNLIGALLFDSGETAE ATRLKRTARRRYTRRKNRICYLQEIFSNEMAKVDDSFFHRLEESF LVEEDKKHERHPIFGNIVDEVAYHEKYPTIYHLRKKLVDSTDKA DLRLIYLALAHMIKFRGHFLIEGDLNPDNSDVDKLFIQLVQTYNQ LFEENPINASGVDAKAILSARLSKSRRLENLIAQLPGEKKNGLFG NLIALSLGLTPNFKSNFDLAEDAKLQLSKDTYDDDLDNLLAQIGD QYADLFLAAKNLSDAILLSDILRVNTEITKAPLSASMIKRYDEHH QDLTLLKALVRQQLPEKYKEIFFDQSKNGYAGYIDGGASQEEFY KFIKPILEKMDGTEELLVKLNREDLLRKQRTFDNGSIPHQIHLGE LHAILRRQEDFYPFLKDNREKIEKILTFRIPYYVGPLARGNSRFA WMTRKSEETITPWNFEEVVDKGASAQSFIERMTNFDKNLPNEKV LPKHSLLYEYFTVYNELTKVKYVTEGMRKPAFLSGEQKKAIVDL LFKTNRKVTVKQLKEDYFKKIECFDSVEISGVEDRFNASLGTYHD LLKIIKDKDFLDNEENEDILEDIVLTLTLFEDREMIEERLKTYAHL FDDKVMKQLKRRRYTGWGRLSRKLINGIRDKQSGKTILDFLKSD GFANRNFMQLIHDDSLTFKEDIQKAQVSGQGDSLHEHIANLAGSP AIKKGILQTVKVVDELVKVMGRHKPENIVIEMARENQTTQKGQ KNSRERMKRIEEGIKELGSQILKEHPVENTQLQNEKLYLYYLQN GRDMYVDQELDINRLSDYDVDAIVPQSFLKDDSIDNKVLTRSDKN RGKSDNVPSEEVVKKMKNYWRQLLNAKLITQRKFDNLTKAERG GLSELDKAGFIKRQLVETRQITKHVAQILDSRMNTKYDENDKLIR EVKVITLKSKLVSDFRKDFQFYKVREINNYHHAHDAYLNAVVGT ALIKKYPKLESEFVYGDYKVYDVRKMIAKSEQEIGKATAKYFFY SNIMNFFKTEITLANGEIRKRPLIETNGETGEIVWDKGRDFATVR KVLSMPQVNIVKKTEVQTGGFSKESILPKRNSDKLIARKKDWDP KKYGGFDSPTVAYSVLVVAKVEKGKSKKLKSVKELLGITIMERS SFEKNPIDFLEAKGYKEVKKDLIIKLPKYSLFELENGRKRMLASA GELQKGNELALPSKYVNFLYLASHYEKLKGSPEDNEQKQLFVEQ HKHYLDEIIEQISEFSKRVILADANLDKVLSAYNKHRDKPIREQAE NIIHLFTLTNLGAPAAFKYFDTTIDRKRYTSTKEVLDATLIHQSIT GLYETRIDLSQLGGDAYPYDVPDYASLGSGSPKKKRKVEDPKKKR KVDGIGSGSNGSSGEELLSKNYHLENEVARLKKGSGSGGSGSGGSGSG SGGSGSGGSGSGEELLSKNYHLENEVARLKKGSGSGGSGSGGSGSGSG GSGSGGSGSGEELLSKNYHLENEVARLKKGSGSGGSGSGGSGSGSGGS GSGGSGSGEELLSKNYHLENEVARLKKGSGSGGSGSGGSGSGSGGSGS GGSGSGEELLSKNYHLENEVARLKKGSGSGGSGSGGSGSGSGGSGSGG SGSGEELLSKNYHLENEVARLKKGSGSGGSGSGGSGSGSGGSGSGGSG SGEELLSKNYHLENEVARLKKGSGSGGSGSGGSGSGSGGSGSGGSGSG EELLSKNYHLENEVARLKKGSGSGGSGSGGSGSGSGGSGSGGSGSGEE LLSKNYHLENEVARLKKGSGSGGSGSGGSGSGSGGSGSGGSGSGEELLS KNYHLENEVARLKKGSGSGQRGSKRTADGSEFESPKKKRKV* |
| 83 | DNMT 3A-DNMT 3L-scFV | MPAAKRVKLDGSNHDQEFDPPKVYPPVPAEKRKPIRVLSLFDGIATG LLVLKDLGIQVDRYIASEVCEDSITVGMVRHQGKIMYVGDVRSVTQK HIQEWGPFDLVIGGSPCNDLSIVNPARKGLYEGTGRLFFEFYRLLHD ARPKEGDDRPFFWLFENVVAMGVSDKRDISRFLESNPVMIDAKEVS AAHRARYFWGNLPGMNRPLASTVNDKLELQECLEHGRIAKFSKVRT ITTRSNSIKQGKDQHFPVFMNEKEDILWCTEMERVFGFPVHYTDVS NMSRLARQRLLGRSWSVPVIRHLFAPLKEYFACVSSGNSNANSRGPS FSSGLVPLSLRGSHMGPMEIYKTVSAWKRQPVRVLSLFRNIDKVLKSL GFLESGSGSGGGTLKYVEDVTNVVRRDVEKWGPFDLVYGSTQPLGS SCDRCPGWYMFQFHRILQYALPRQESQRPFFWIFMDNLLLTEDDQE TTTRFLQTEAVTLQDVRGRDYQNAMRVWSNIPGLKSKHAPLTPKEE EYLQAQVRSRSKLDAPKVDLLVKNCLLPLREYFKYFSQNSLPLGGPSS GAPPPSGGSPAGSPTSTEEGTSESATPESGPGTSTEPSEGSAPGSPAGSP TSTEEGTSTEPSEGSAPGTSTEPSEGPDIVMTQSPSSLSASVGDRVTITCR SSTGAVTTSNYASWVQEKPGKLFKGLIGGTNNRAPGVPSRFSGSLIGDKA TLTISSLQPEDFATYFCALWYSNHWVFGQGTKVELKRGGGGSGGGGSG GGGSSGGGSEVKLLESGGGLVQPGGSLKLSCAVSGFSLTDYGVNWVRQA PGRGLEWIGVIWGDGITDYNSALKDRFIISKDNGKNTVYLQMSKVRSDDT ALYYCVTGLFDYWGQGTLVTVSSYPYDVPDYAGGGGGSGGGGSGGGG SGGGGS* |
| 84 | 3xGCN 4-dCas9-KRAB | MPAAKRVKLDGSEELLSKNYHLENEVARLKKGSGSGGSGSGGSGSGS GGSGSGGSGSGEELLSKNYHLENEVARLKKGSGSGGSGSGGSGSGSGG SGSGGSGSGEELLSKNYHLENEVARLKKGSGSGGSGSGGSGSGSGGSG SGGSGSGPKKKRKVMDKKYSIGLAIGTNSVGWAVITDEYKVPSKK FKVLGNTDRHSIKKNLIGALLFDSGETAEATRLKRTARRRYTRR KNRICYLQEIFSNEMAKVDDSFFHRLEESFLVEEDKKHERHPIFG NIVDEVAYHEKYPTIYHLRKKLVDSTDKADLRLIYLALAHMIKFR GHFLIEGDLNPDNSDVDKLFIQLVQTYNQLFEENPINASGVDAKA ILSARLSKSRRLENLIAQLPGEKKNGLFGNLIALSLGLTPNFKSNF DLAEDAKLQLSKDTYDDDLDNLLAQIGDQYADLFLAAKNLSDAI LLSDILRVNTEITKAPLSASMIKRYDEHHQDLTLLKALVRQQLPE KYKEIFFDQSKNGYAGYIDGGASQEEFYKFIKPILEKMDGTEELL VKLNREDLLRKQRTFDNGSIPHQIHLGELHAILRRQEDFYPFLKD NREKIEKILTFRIPYYVGPLARGNSRFAWMTRKSEETITPWNFEE VVDKGASAQSFIERMTNFDKNLPNEKVLPKHSLLYEYFTVYNEL TKVKYVTEGMRKPAFLSGEQKKAIVDLLFKTNRKVTVKQLKED YFKKIECFDSVEISGVEDRFNASLGTYHDLLKIIKDKDFLDNEENE DILEDIVLTLTLFEDREMIEERLKTYAHLFDDKVMKQLKRRRYT GWGRLSRKLINGIRDKQSGKTILDFLKSDGFANRNFMQLIHDDSL TFKEDIQKAQVSGQGDSLHEHIANLAGSPAIKKGILQTVKVVDEL VKVMGRHKPENIVIEMARENQTTQKGQKNSRERMKRIEEGIKE LGSQILKEHPVENTQLQNEKLYLYYLQNGRDMYVDQELDINRLS DYDVDAIVPQSFLKDDSIDNKVLTRSDKNRGKSDNVPSEEVVKK MKNYWRQLLNAKLITQRKFDNLTKAERGGLSELDKAGFIKRQL VETRQITKHVAQILDSRMNTKYDENDKLIREVKVITLKSKLVSDF RKDFQFYKVREINNYHHAHDAYLNAVVGTALIKKYPKLESEFVY GDYKVYDVRKMIAKSEQEIGKATAKYFFYSNIMNFFKTEITLAN GEIRKRPLIETNGETGEIVWDKGRDFATVRKVLSMPQVNIVKKT EVQTGGFSKESILPKRNSDKLIARKKDWDPKKYGGFDSPTVAYS VLVVAKVEKGKSKKLKSVKELLGITIMERSSFEKNPIDFLEAKGY KEVKKDLIIKLPKYSLFELENGRKRMLASAGELQKGNELALPSK YVNFLYLASHYEKLKGSPEDNEQKQLFVEQHKHYLDEIIEQISEF SKRVILADANLDKVLSAYNKHRDKPIREQAENIIHLFTLTNLGAP AAFKYFDTTIDRKRYTSTKEVLDATLIHQSITGLYETRIDLSQLGG DAYPYDVPDYASLGSGSPKKKRKVEDPKKKRKVDGSGSETPGTSES ATPES<RTLVTFKDVFVDFTREEWKLLDTAQQIVYRNVMLENYK NLVSLGYQLTKPDVILRLEKGEEP>* |
| 85 | GFP1-10-KRAB | MPAAKRVKLDGS*SKGEELFTGVVPILVELDGDVNGHKFSVRGEGEGD* *ATIGKLTLKFICTTGKLPVPWPTLVTTLTYGVQCFSRYPDHMKRHDFFKS* *AMPEGYVQERTISFKDDGKYKTRAVVKFEGDTLVNRIELKGTDFKEDGN* *ILGHKLEYNFNSHNVYITADKQKNGIKANFTVRHNVEDGSVQLADHYQQ* *NTPIGDGPVLLPDNHYLSTQTVLSKDPNEK*LDPGGGGSG<**RTLVTFKD** **VFVDFTREEWKLLDTAQQIVYRNVMLENYKNLVSLGYQLTKPD** **VILRLEKGEEP**>* |
| 86 | DNMT 3A-DNMT 3L-dCas9-1xGFP 11 | MPAAKRVKLDGSNHDQEFDPPKVYPPVPAEKRKPIRVLSLFDGIATG LLVLKDLGIQVDRYIASEVCEDSITVGMVRHQGKIMYVGDVRSVTQK HIQEWGPFDLVIGGSPCNDLSIVNPARKGLYEGTGRLFFEFYRLLHD ARPKEGDDRPFFWLFENVVAMGVSDKRDISRFLESNPVMIDAKEVS AAHRARYFWGNLPGMNRPLASTVNDKLELQECLEHGRIAKFSKVRT ITTRSNSIKQGKDQHFPVFMNEKEDILWCTEMERVFGFPVHYTDVS NMSRLARQRLLGRSWSVPVIRHLFAPLKEYFACVSSGNSNANSRGPS FSSGLVPLSLRGSHMGPMEIYKTVSAWKRQPVRVLSLFRNIDKVLKSL GFLESGSGSGGGTLKYVEDVTNVVRRDVEKWGPFDLVYGSTQPLGS SCDRCPGWYMFQFHRILQYALPRQESQRPFFWIFMDNLLLTEDDQE TTTRFLQTEAVTLQDVRGRDYQNAMRVWSNIPGLKSKHAPLTPKEE EYLQAQVRSRSKLDAPKVDLLVKNCLLPLREYFKYFSQNSLPLGGPSS GAPPPSGGSPAGSPTSTEEGTSESATPESGPGTSTEPSEGSAPGSPAGSP TSTEEGTSTEPSEGSAPGTSTEPSEPKKKRKVMDKKYSIGLAIGTNSV GWAVITDEYKVPSKKFKVLGNTDRHSIKKNLIGALLFDSGETAE ATRLKRTARRRYTRRKNRICYLQEIFSNEMAKVDDSFFHRLEESF LVEEDKKHERHPIFGNIVDEVAYHEKYPTIYHLRKKLVDSTDKA DLRLIYLALAHMIKFRGHFLIEGDLNPDNSDVDKLFIQLVQTYNQ LFEENPINASGVDAKAILSARLSKSRRLENLIAQLPGEKKNGLFG NLIALSLGLTPNFKSNFDLAEDAKLQLSKDTYDDDLDNLLAQIGD QYADLFLAAKNLSDAILLSDILRVNTEITKAPLSASMIKRYDEHH QDLTLLKALVRQQLPEKYKEIFFDQSKNGYAGYIDGGASQEEFY KFIKPILEKMDGTEELLVKLNREDLLRKQRTFDNGSIPHQIHLGE LHAILRRQEDFYPFLKDNREKIEKILTFRIPYYVGPLARGNSRFA WMTRKSEETITPWNFEEVVDKGASAQSFIERMTNFDKNLPNEKV LPKHSLLYEYFTVYNELTKVKYVTEGMRKPAFLSGEQKKAIVDL LFKTNRKVTVKQLKEDYFKKIECFDSVEISGVEDRFNASLGTYHD LLKIIKDKDFLDNEENEDILEDIVLTLTLFEDREMIEERLKTYAHL FDDKVMKQLKRRRYTGWGRLSRKLINGIRDKQSGKTILDFLKSD GFANRNFMQLIHDDSLTFKEDIQKAQVSGQGDSLHEHIANLAGSP AIKKGILQTVKVVDELVKVMGRHKPENIVIEMARENQTTQKGQ KNSRERMKRIEEGIKELGSQILKEHPVENTQLQNEKLYLYYLQN GRDMYVDQELDINRLSDYDVDAIVPQSFLKDDSIDNKVLTRSDKN RGKSDNVPSEEVVKKMKNYWRQLLNAKLITQRKFDNLTKAERG GLSELDKAGFIKRQLVETRQITKHVAQILDSRMNTKYDENDKLIR EVKVITLKSKLVSDFRKDFQFYKVREINNYHHAHDAYLNAVVGT ALIKKYPKLESEFVYGDYKVYDVRKMIAKSEQEIGKATAKYFFY SNIMNFFKTEITLANGEIRKRPLIETNGETGEIVWDKGRDFATVR KVLSMPQVNIVKKTEVQTGGFSKESILPKRNSDKLIARKKDWDP KKYGGFDSPTVAYSVLVVAKVEKGKSKKLKSVKELLGITIMERS SFEKNPIDFLEAKGYKEVKKDLIIKLPKYSLFELENGRKRMLASA GELQKGNELALPSKYVNFLYLASHYEKLKGSPEDNEQKQLFVEQ HKHYLDEIIEQISEFSKRVILADANLDKVLSAYNKHRDKPIREQAE NIIHLFTLTNLGAPAAFKYFDTTIDRKRYTSTKEVLDATLIHQSIT GLYETRIDLSQLGGDAYPYDVPDYASLGSGSPKKKRKVEDPKKKR KVDGSGSETPGTSESATPESGSGSNGSSGSMRDHMVLHEYVNAAGITG GSGGAAARSGSGQRGSKRTADGSEFESPKKKRKV* |
| 87 | DNMT 3A-DNMT 3L-dCas9-7xGFP 11 | MPAAKRVKLDGSNHDQEFDPPKVYPPVPAEKRKPIRVLSLFDGIATG LLVLKDLGIQVDRYIASEVCEDSITVGMVRHQGKIMYVGDVRSVTQK HIQEWGPFDLVIGGSPCNDLSIVNPARKGLYEGTGRLFFEFYRLLHD ARPKEGDDRPFFWLFENVVAMGVSDKRDISRFLESNPVMIDAKEVS AAHRARYFWGNLPGMNRPLASTVNDKLELQECLEHGRIAKFSKVRT ITTRSNSIKQGKDQHFPVFMNEKEDILWCTEMERVFGFPVHYTDVS NMSRLARQRLLGRSWSVPVIRHLFAPLKEYFACVSSGNSNANSRGPS FSSGLVPLSLRGSHMGPMEIYKTVSAWKRQPVRVLSLFRNIDKVLKSL GFLESGSGSGGGTLKYVEDVTNVVRRDVEKWGPFDLVYGSTQPLGS SCDRCPGWYMFQFHRILQYALPRQESQRPFFWIFMDNLLLTEDDQE TTTRFLQTEAVTLQDVRGRDYQNAMRVWSNIPGLKSKHAPLTPKEE EYLQAQVRSRSKLDAPKVDLLVKNCLLPLREYFKYFSQNSLPLGGPSS GAPPPSGGSPAGSPTSTEEGTSESATPESGPGTSTEPSEGSAPGSPAGSP TSTEEGTSTEPSEGSAPGTSTEPSEPKKKRKVMDKKYSIGLAIGTNSV GWAVITDEYKVPSKKFKVLGNTDRHSIKKNLIGALLFDSGETAE ATRLKRTARRRYTRRKNRICYLQEIFSNEMAKVDDSFFHRLEESF LVEEDKKHERHPIFGNIVDEVAYHEKYPTIYHLRKKLVDSTDKA DLRLIYLALAHMIKFRGHFLIEGDLNPDNSDVDKLFIQLVQTYNQ LFEENPINASGVDAKAILSARLSKSRRLENLIAQLPGEKKNGLFG NLIALSLGLTPNFKSNFDLAEDAKLQLSKDTYDDDLDNLLAQIGD QYADLFLAAKNLSDAILLSDILRVNTEITKAPLSASMIKRYDEHH QDLTLLKALVRQQLPEKYKEIFFDQSKNGYAGYIDGGASQEEFY KFIKPILEKMDGTEELLVKLNREDLLRKQRTFDNGSIPHQIHLGE LHAILRRQEDFYPFLKDNREKIEKILTFRIPYYVGPLARGNSRFA WMTRKSEETITPWNFEEVVDKGASAQSFIERMTNFDKNLPNEKV LPKHSLLYEYFTVYNELTKVKYVTEGMRKPAFLSGEQKKAIVDL LFKTNRKVTVKQLKEDYFKKIECFDSVEISGVEDRFNASLGTYHD LLKIIKDKDFLDNEENEDILEDIVLTLTLFEDREMIEERLKTYAHL FDDKVMKQLKRRRYTGWGRLSRKLINGIRDKQSGKTILDFLKSD GFANRNFMQLIHDDSLTFKEDIQKAQVSGQGDSLHEHIANLAGSP AIKKGILQTVKVVDELVKVMGRHKPENIVIEMARENQTTQKGQ KNSRERMKRIEEGIKELGSQILKEHPVENTQLQNEKLYLYYLQN GRDMYVDQELDINRLSDYDVDAIVPQSFLKDDSIDNKVLTRSDKN RGKSDNVPSEEVVKKMKNYWRQLLNAKLITQRKFDNLTKAERG GLSELDKAGFIKRQLVETRQITKHVAQILDSRMNTKYDENDKLIR EVKVITLKSKLVSDFRKDFQFYKVREINNYHHAHDAYLNAVVGT ALIKKYPKLESEFVYGDYKVYDVRKMIAKSEQEIGKATAKYFFY SNIMNFFKTEITLANGEIRKRPLIETNGETGEIVWDKGRDFATVR KVLSMPQVNIVKKTEVQTGGFSKESILPKRNSDKLIARKKDWDP KKYGGFDSPTVAYSVLVVAKVEKGKSKKLKSVKELLGITIMERS SFEKNPIDFLEAKGYKEVKKDLIIKLPKYSLFELENGRKRMLASA GELQKGNELALPSKYVNFLYLASHYEKLKGSPEDNEQKQLFVEQ HKHYLDEIIEQISEFSKRVILADANLDKVLSAYNKHRDKPIREQAE NIIHLFTLTNLGAPAAFKYFDTTIDRKRYTSTKEVLDATLIHQSIT GLYETRIDLSQLGGDAYPYDVPDYASLGSGSPKKKRKVEDPKKKR KVDGSGSETPGTSESATPESGSGSNGSSGSMRDHMVLHEYVNAAGITG GSGGRDHMVLHEYVNAAGITGGSGGRDHMVLHEYVNAAGITGGSGGR DHMVLHEYVNAAGITGGSGGRDHMVLHEYVNAAGITGGSGGRDHMVL HEYVNAAGITGGSGGRDHMVLHEYVNAAGITAAARSGSGQRGSKRTA DGSEFESPKKKRKV* |
| 88 | DNMT 3A-hDNM T3L-dCas9-1xGCN 4 | MPAAKRVKLDGSNHDQEFDPPKVYPPVPAEKRKPIRVLSLFDGIATG LLVLKDLGIQVDRYIASEVCEDSITVGMVRHQGKIMYVGDVRSVTQK HIQEWGPFDLVIGGSPCNDLSIVNPARKGLYEGTGRLFFEFYRLLHD ARPKEGDDRPFFWLFENVVAMGVSDKRDISRFLESNPVMIDAKEVS AAHRARYFWGNLPGMNRPLASTVNDKLELQECLEHGRIAKFSKVRT ITTRSNSIKQGKDQHFPVFMNEKEDILWCTEMERVFGFPVHYTDVS NMSRLARQRLLGRSWSVPVIRHLFAPLKEYFACVSSGNSNANSRGPS FSSGLVPLSLRGSHMFETVPVWRRQPVRVLSLFEDIKKELTSLGFLES GSDPGQLKHVVDVTDTVRKDVEEWGPFDLVYGATPPLGHTCDRPPS WYLFQFHRLLQYARPKPGSPRPFFWMFVDNLVLNKEDLDVASRFLE MEPVTIPDVHGGSLQNAVRVWSNIPAIRSSRHWALVSEEELSLLAQN KQSSKLAAKWPTKLVKNCFLPLREYFKYFSGGPSSGAPPPSGGSPAGS PTSTEEGTSESATPESGPGTSTEPSEGSAPGSPAGSPTSTEEGTSTEPSE GSAPGTSTEPSEPKKKRKVMDKKYSIGLAIGTNSVGWAVITDEYKV PSKKFKVLGNTDRHSIKKNLIGALLFDSGETAEATRLKRTARRRY TRRKNRICYLQEIFSNEMAKVDDSFFHRLEESFLVEEDKKHERHP IFGNIVDEVAYHEKYPTIYHLRKKLVDSTDKADLRLIYLALAHMI KFRGHFLIEGDLNPDNSDVDKLFIQLVQTYNQLFEENPINASGVD AKAILSARLSKSRRLENLIAQLPGEKKNGLFGNLIALSLGLTPNFK SNFDLAEDAKLQLSKDTYDDDLDNLLAQIGDQYADLFLAAKNLS DAILLSDILRVNTEITKAPLSASMIKRYDEHHQDLTLLKALVRQQ LPEKYKEIFFDQSKNGYAGYIDGGASQEEFYKFIKPILEKMDGTE ELLVKLNREDLLRKQRTFDNGSIPHQIHLGELHAILRRQEDFYPF LKDNREKIEKILTFRIPYYVGPLARGNSRFAWMTRKSEETITPWN FEEVVDKGASAQSFIERMTNFDKNLPNEKVLPKHSLLYEYFTVY NELTKVKYVTEGMRKPAFLSGEQKKAIVDLLFKTNRKVTVKQL KEDYFKKIECFDSVEISGVEDRFNASLGTYHDLLKIIKDKDFLDNE ENEDILEDIVLTLTLFEDREMIEERLKTYAHLFDDKVMKQLKRR RYTGWGRLSRKLINGIRDKQSGKTILDFLKSDGFANRNFMQLIH DDSLTFKEDIQKAQVSGQGDSLHEHIANLAGSPAIKKGILQTVKV VDELVKVMGRHKPENIVIEMARENQTTQKGQKNSRERMKRIEE GIKELGSQILKEHPVENTQLQNEKLYLYYLQNGRDMYVDQELDI NRLSDYDVDAIVPQSFLKDDSIDNKVLTRSDKNRGKSDNVPSEEV VKKMKNYWRQLLNAKLITQRKFDNLTKAERGGLSELDKAGFIK RQLVETRQITKHVAQILDSRMNTKYDENDKLIREVKVITLKSKLV SDFRKDFQFYKVREINNYHHAHDAYLNAVVGTALIKKYPKLESE FVYGDYKVYDVRKMIAKSEQEIGKATAKYFFYSNIMNFFKTEITL ANGEIRKRPLIETNGETGEIVWDKGRDFATVRKVLSMPQVNIVK KTEVQTGGFSKESILPKRNSDKLIARKKDWDPKKYGGFDSPTVA YSVLVVAKVEKGKSKKLKSVKELLGITIMERSSFEKNPIDFLEAK GYKEVKKDLIIKLPKYSLFELENGRKRMLASAGELQKGNELALP SKYVNFLYLASHYEKLKGSPEDNEQKQLFVEQHKHYLDEIIEQIS EFSKRVILADANLDKVLSAYNKHRDKPIREQAENIIHLFTLTNLG APAAFKYFDTTIDRKRYTSTKEVLDATLIHQSITGLYETRIDLSQL GGDAYPYDVPDYASLGSGSPKKKRKVEDPKKKRKVDGIGSGSNGSS GEELLSKNYHLENEVARLKKGSGSGGSGSGGSGSGSGGSGSGGSGSGE ELLSKNYHGSGSGQRGSKRTADGSEFESPKKKRKV* |
| 89 | DNMT 3A-hDNM T3L-dCas9-2xGCN 4 | MPAAKRVKLDGSNHDQEFDPPKVYPPVPAEKRKPIRVLSLFDGIATG LLVLKDLGIQVDRYIASEVCEDSITVGMVRHQGKIMYVGDVRSVTQK HIQEWGPFDLVIGGSPCNDLSIVNPARKGLYEGTGRLFFEFYRLLHD ARPKEGDDRPFFWLFENVVAMGVSDKRDISRFLESNPVMIDAKEVS AAHRARYFWGNLPGMNRPLASTVNDKLELQECLEHGRIAKFSKVRT ITTRSNSIKQGKDQHFPVFMNEKEDILWCTEMERVFGFPVHYTDVS NMSRLARQRLLGRSWSVPVIRHLFAPLKEYFACVSSGNSNANSRGPS FSSGLVPLSLRGSHMFETVPVWRRQPVRVLSLFEDIKKELTSLGFLES GSDPGQLKHVVDVTDTVRKDVEEWGPFDLVYGATPPLGHTCDRPPS WYLFQFHRLLQYARPKPGSPRPFFWMFVDNLVLNKEDLDVASRFLE MEPVTIPDVHGGSLQNAVRVWSNIPAIRSSRHWALVSEEELSLLAQN KQSSKLAAKWPTKLVKNCFLPLREYFKYFSGGPSSGAPPPSGGSPAGS PTSTEEGTSESATPESGPGTSTEPSEGSAPGSPAGSPTSTEEGTSTEPSE GSAPGTSTEPSEPKKKRKVMDKKYSIGLAIGTNSVGWAVITDEYKV PSKKFKVLGNTDRHSIKKNLIGALLFDSGETAEATRLKRTARRRY TRRKNRICYLQEIFSNEMAKVDDSFFHRLEESFLVEEDKKHERHP IFGNIVDEVAYHEKYPTIYHLRKKLVDSTDKADLRLIYLALAHMI KFRGHFLIEGDLNPDNSDVDKLFIQLVQTYNQLFEENPINASGVD AKAILSARLSKSRRLENLIAQLPGEKKNGLFGNLIALSLGLTPNFK SNFDLAEDAKLQLSKDTYDDDLDNLLAQIGDQYADLFLAAKNLS DAILLSDILRVNTEITKAPLSASMIKRYDEHHQDLTLLKALVRQQ LPEKYKEIFFDQSKNGYAGYIDGGASQEEFYKFIKPILEKMDGTE ELLVKLNREDLLRKQRTFDNGSIPHQIHLGELHAILRRQEDFYPF LKDNREKIEKILTFRIPYYVGPLARGNSRFAWMTRKSEETITPWN FEEVVDKGASAQSFIERMTNFDKNLPNEKVLPKHSLLYEYFTVY NELTKVKYVTEGMRKPAFLSGEQKKAIVDLLFKTNRKVTVKQL KEDYFKKIECFDSVEISGVEDRFNASLGTYHDLLKIIKDKDFLDNE ENEDILEDIVLTLTLFEDREMIEERLKTYAHLFDDKVMKQLKRR RYTGWGRLSRKLINGIRDKQSGKTILDFLKSDGFANRNFMQLIH DDSLTFKEDIQKAQVSGQGDSLHEHIANLAGSPAIKKGILQTVKV VDELVKVMGRHKPENIVIEMARENQTTQKGQKNSRERMKRIEE GIKELGSQILKEHPVENTQLQNEKLYLYYLQNGRDMYVDQELDI NRLSDYDVDAIVPQSFLKDDSIDNKVLTRSDKNRGKSDNVPSEEV VKKMKNYWRQLLNAKLITQRKFDNLTKAERGGLSELDKAGFIK RQLVETRQITKHVAQILDSRMNTKYDENDKLIREVKVITLKSKLV SDFRKDFQFYKVREINNYHHAHDAYLNAVVGTALIKKYPKLESE FVYGDYKVYDVRKMIAKSEQEIGKATAKYFFYSNIMNFFKTEITL ANGEIRKRPLIETNGETGEIVWDKGRDFATVRKVLSMPQVNIVK KTEVQTGGFSKESILPKRNSDKLIARKKDWDPKKYGGFDSPTVA YSVLVVAKVEKGKSKKLKSVKELLGITIMERSSFEKNPIDFLEAK GYKEVKKDLIIKLPKYSLFELENGRKRMLASAGELQKGNELALP SKYVNFLYLASHYEKLKGSPEDNEQKQLFVEQHKHYLDEIIEQIS EFSKRVILADANLDKVLSAYNKHRDKPIREQAENIIHLFTLTNLG APAAFKYFDTTIDRKRYTSTKEVLDATLIHQSITGLYETRIDLSQL GGDAYPYDVPDYASLGSGSPKKKRKVEDPKKKRKVDGIGSGSNGSS GEELLSKNYHLENEVARLKKGSGSGGSGSGGSGSGSGGSGSGGSGSGE ELLSKNYHLENEVARLKKGSGSGQRGSKRTADGSEFESPKKKRKV* |
| 90 | DNMT 3A-hDNM T3L-dCas9-10xGC N4 | MPAAKRVKLDGSNHDQEFDPPKVYPPVPAEKRKPIRVLSLFDGIATG LLVLKDLGIQVDRYIASEVCEDSITVGMVRHQGKIMYVGDVRSVTQK HIQEWGPFDLVIGGSPCNDLSIVNPARKGLYEGTGRLFFEFYRLLHD ARPKEGDDRPFFWLFENVVAMGVSDKRDISRFLESNPVMIDAKEVS AAHRARYFWGNLPGMNRPLASTVNDKLELQECLEHGRIAKFSKVRT ITTRSNSIKQGKDQHFPVFMNEKEDILWCTEMERVFGFPVHYTDVS NMSRLARQRLLGRSWSVPVIRHLFAPLKEYFACVSSGNSNANSRGPS FSSGLVPLSLRGSHMFETVPVWRRQPVRVLSLFEDIKKELTSLGFLES GSDPGQLKHVVDVTDTVRKDVEEWGPFDLVYGATPPLGHTCDRPPS WYLFQFHRLLQYARPKPGSPRPFFWMFVDNLVLNKEDLDVASRFLE MEPVTIPDVHGGSLQNAVRVWSNIPAIRSSRHWALVSEEELSLLAQN KQSSKLAAKWPTKLVKNCFLPLREYFKYFSGGPSSGAPPPSGGSPAGS PTSTEEGTSESATPESGPGTSTEPSEGSAPGSPAGSPTSTEEGTSTEPSE GSAPGTSTEPSEPKKKRKVMDKKYSIGLAIGTNSVGWAVITDEYKV PSKKFKVLGNTDRHSIKKNLIGALLFDSGETAEATRLKRTARRRY TRRKNRICYLQEIFSNEMAKVDDSFFHRLEESFLVEEDKKHERHP IFGNIVDEVAYHEKYPTIYHLRKKLVDSTDKADLRLIYLALAHMI KFRGHFLIEGDLNPDNSDVDKLFIQLVQTYNQLFEENPINASGVD AKAILSARLSKSRRLENLIAQLPGEKKNGLFGNLIALSLGLTPNFK SNFDLAEDAKLQLSKDTYDDDLDNLLAQIGDQYADLFLAAKNLS DAILLSDILRVNTEITKAPLSASMIKRYDEHHQDLTLLKALVRQQ LPEKYKEIFFDQSKNGYAGYIDGGASQEEFYKFIKPILEKMDGTE ELLVKLNREDLLRKQRTFDNGSIPHQIHLGELHAILRRQEDFYPF LKDNREKIEKILTFRIPYYVGPLARGNSRFAWMTRKSEETITPWN FEEVVDKGASAQSFIERMTNFDKNLPNEKVLPKHSLLYEYFTVY NELTKVKYVTEGMRKPAFLSGEQKKAIVDLLFKTNRKVTVKQL KEDYFKKIECFDSVEISGVEDRFNASLGTYHDLLKIIKDKDFLDNE ENEDILEDIVLTLTLFEDREMIEERLKTYAHLFDDKVMKQLKRR RYTGWGRLSRKLINGIRDKQSGKTILDFLKSDGFANRNFMQLIH DDSLTFKEDIQKAQVSGQGDSLHEHIANLAGSPAIKKGILQTVKV VDELVKVMGRHKPENIVIEMARENQTTQKGQKNSRERMKRIEE GIKELGSQILKEHPVENTQLQNEKLYLYYLQNGRDMYVDQELDI NRLSDYDVDAIVPQSFLKDDSIDNKVLTRSDKNRGKSDNVPSEEV VKKMKNYWRQLLNAKLITQRKFDNLTKAERGGLSELDKAGFIK RQLVETRQITKHVAQILDSRMNTKYDENDKLIREVKVITLKSKLV SDFRKDFQFYKVREINNYHHAHDAYLNAVVGTALIKKYPKLESE FVYGDYKVYDVRKMIAKSEQEIGKATAKYFFYSNIMNFFKTEITL ANGEIRKRPLIETNGETGEIVWDKGRDFATVRKVLSMPQVNIVK KTEVQTGGFSKESILPKRNSDKLIARKKDWDPKKYGGFDSPTVA YSVLVVAKVEKGKSKKLKSVKELLGITIMERSSFEKNPIDFLEAK GYKEVKKDLIIKLPKYSLFELENGRKRMLASAGELQKGNELALP SKYVNFLYLASHYEKLKGSPEDNEQKQLFVEQHKHYLDEIIEQIS EFSKRVILADANLDKVLSAYNKHRDKPIREQAENIIHLFTLTNLG APAAFKYFDTTIDRKRYTSTKEVLDATLIHQSITGLYETRIDLSQL GGDAYPYDVPDYASLGSGSPKKKRKVEDPKKKRKVDGIGSGSNGSS GEELLSKNYHLENEVARLKKGSGSGGSGSGGSGSGSGGSGSGGSGSGE ELLSKNYHLENEVARLKKGSGSGGSGSGGSGSGSGGSGSGGSGSGEEL LSKNYHLENEVARLKKGSGSGGSGSGGSGSGSGGSGSGGSGSGEELLS KNYHLENEVARLKKGSGSGGSGSGGSGSGSGGSGSGGSGSGEELLSKN YHLENEVARLKKGSGSGGSGSGGSGSGSGGSGSGGSGSGEELLSKNYH LENEVARLKKGSGSGGSGSGGSGSGSGGSGSGGSGSGEELLSKNYHLE NEVARLKKGSGSGGSGSGGSGSGSGGSGSGGSGSGEELLSKNYHLENE VARLKKGSGSGGSGSGGSGSGSGGSGSGGSGSGEELLSKNYHLENEVA RLKKGSGSGGSGSGGSGSGSGGSGSGGSGSGEELLSKNYHLENEVARL KKGSGSGQRGSKRTADGSEFESPKKKRKV* |
| 91 | DNMT 3A-hDNM T3L-dCas12 a-1xGCN 4 | MPAAKRVKLDGSNHDQEFDPPKVYPPVPAEKRKPIRVLSLFDGIATG LLVLKDLGIQVDRYIASEVCEDSITVGMVRHQGKIMYVGDVRSVTQK HIQEWGPFDLVIGGSPCNDLSIVNPARKGLYEGTGRLFFEFYRLLHD ARPKEGDDRPFFWLFENVVAMGVSDKRDISRFLESNPVMIDAKEVS AAHRARYFWGNLPGMNRPLASTVNDKLELQECLEHGRIAKFSKVRT ITTRSNSIKQGKDQHFPVFMNEKEDILWCTEMERVFGFPVHYTDVS NMSRLARQRLLGRSWSVPVIRHLFAPLKEYFACVSSGNSNANSRGPS FSSGLVPLSLRGSHMFETVPVWRRQPVRVLSLFEDIKKELTSLGFLES GSDPGQLKHVVDVTDTVRKDVEEWGPFDLVYGATPPLGHTCDRPPS WYLFQFHRLLQYARPKPGSPRPFFWMFVDNLVLNKEDLDVASRFLE MEPVTIPDVHGGSLQNAVRVWSNIPAIRSSRHWALVSEEELSLLAQN KQSSKLAAKWPTKLVKNCFLPLREYFKYFSGGPSSGAPPPSGGSPAGS PTSTEEGTSESATPESGPGTSTEPSEGSAPGSPAGSPTSTEEGTSTEPSE GSAPGTSTEPSEPKKKRKVMSKLEKFTNCYSLSKTLRFKAIPVGKT QENIDNKRLLVEDEKRAEDYKGVKKLLDRYYLSFINDVLHSIKL KNLNNYISLFRKKTRTEKENKELENLEINLRKEIAKAFKGNEGYK SLFKKDIIETILPEFLDDKDEIALVNSFNGFTTAFTGFFDNRENMFS EEAKSTSIAFRCINENLTRYISNMDIFEKVDAIFDKHEVQEIKEKIL NSDYDVEDFFEGEFFNFVLTQEGIDVYNAIIGGFVTESGEKIKGLN EYINLYNQKTKQKLPKFKPLYKQVLSDRESLSFYGEGYTSDEEVL EVFRNTLNKNSEIFSSIKKLEKLFKNFDEYSSAGIFVKNGPAISTIS KDIFGEWNVIRDKWNAEYDDIHLKKKAVVTEKYEDDRRKSFKKI GSFSLEQLQEYADADLSVVEKLKEIIIQKVDEIYKVYGSSEKLFDA DFVLEKSLKKNDAVVAIMKDLLDSVKSFENYIKAFFGEGKETNR DESFYGDFVLAYDILLKVDHIYDAIRNYVTQKPYSKDKFKLYFQN PQFMGGWDKDKETDYRATILRYGSKYYLAIMDKKYAKCLQKID KDDVNGNYEKINYKLLPGPNKMLPKVFFSKKWMAYYNPSEDIQ KIYKNGTFKKGDMFNLNDCHKLIDFFKDSISRYPKWSNAYDFNFS ETEKYKDIAGFYREVEEQGYKVSFESASKKEVDKLVEEGKLYMF QIYNKDFSDKSHGTPNLHTMYFKLLFDENNHGQIRLSGGAELFM RRASLKKEELVVHPANSPIANKNPDNPKKTTTLSYDVYKDKRFSE DQYELHIPIAINKCPKNIFKINTEVRVLLKHDDNPYVIGIARGERN LLYIVVVDGKGNIVEQYSLNEIINNFNGIRIKTDYHSLLDKKEKER FEARQNWTSIENIKELKAGYISQVVHKICELVEKYDAVIALADLN SGFKNSRVKVEKQVYQKFEKMLIDKLNYMVDKKSNPCATGGAL KGYQITNKFESFKSMSTQNGFIFYIPAWLTSKIDPSTGFVNLLKTK YTSIADSKKFISSFDRIMYVPEEDLFEFALDYKNFSRTDADYIKKW KLYSYGNRIRIFRNPKKNNVFDWEEVCLTSAYKELFNKYGINYQ QGDIRALLCEQSDKAFYSSFMALMSLMLQMRNSITGRTDVAFLIS PVKNSDGIFYDSRNYEAQENAILPKNADANGAYNIARKVLWAIG QFKKAEDEKLDKVKIAISNKEWLEYAQTSVKHAYPYDVPDYASL GSGSPKKKRKVEDPKKKRKVDGIGSGSNGSSGEELLSKNYHLENEVAR LKKGSGSGGSGSGGSGSGSGGSGSGGSGSGEELLSKNYHGSGSGQRG SKRTADGSEFESPKKKRKV* |
| 92 | DNMT 3A-hDNM T3L-dCas12 i-1xGCN 4 | MPAAKRVKLDGSNHDQEFDPPKVYPPVPAEKRKPIRVLSLFDGIATG LLVLKDLGIQVDRYIASEVCEDSITVGMVRHQGKIMYVGDVRSVTQK HIQEWGPFDLVIGGSPCNDLSIVNPARKGLYEGTGRLFFEFYRLLHD ARPKEGDDRPFFWLFENVVAMGVSDKRDISRFLESNPVMIDAKEVS AAHRARYFWGNLPGMNRPLASTVNDKLELQECLEHGRIAKFSKVRT ITTRSNSIKQGKDQHFPVFMNEKEDILWCTEMERVFGFPVHYTDVS NMSRLARQRLLGRSWSVPVIRHLFAPLKEYFACVSSGNSNANSRGPS FSSGLVPLSLRGSHMFETVPVWRRQPVRVLSLFEDIKKELTSLGFLES GSDPGQLKHVVDVTDTVRKDVEEWGPFDLVYGATPPLGHTCDRPPS WYLFQFHRLLQYARPKPGSPRPFFWMFVDNLVLNKEDLDVASRFLE MEPVTIPDVHGGSLQNAVRVWSNIPAIRSSRHWALVSEEELSLLAQN KQSSKLAAKWPTKLVKNCFLPLREYFKYFSGGPSSGAPPPSGGSPAGS PTSTEEGTSESATPESGPGTSTEPSEGSAPGSPAGSPTSTEEGTSTEPSE GSAPGTSTEPSEPKKKRKVMGITMSDTTFRPYKSKLNPNERKMPM VVDTMNWCDNACSVFFDLFVGLYGGVKIGDVTNFLTEKQDTTT DADLLCAINWFRAMPVSNPDAVVGPEQLVVRFRDYSGHNPSPMA SMYLSAPVNPRYTWVDCRFLYQQMCQTLGANLDVDISTLVQTG LLPIQKDTQLNAYSFCSNVFGEGVKEDPSAKVTSYTAAANALSGA TNPIITYEDYRQILSSAITNRPSRKGAKDILAADLDSTKTGVLPGN YVSQRIAMYQASAASKARGVVLPNRLRVKAYLQNLVGLNYICTT WAAPMLRAVGDIKSKNSNNYRFVGERLERQQEIDNLLAVSNIRS AQAILQQFRANESNQFYIKKRHIGKGLPSLYDMWDLMDEKEGID NYIQSLKGDKTERLPIRELLQIIYPHRKSVSAELFLSAAELTDVED RQNRCMVHPTVRGSLVINFGKSSTIFGAITPPDTLIKFRGEMVPA GQTGMIWFTMHLVDGGKWVKHHIPCHSSRYFEEIYAWKKDLPT LPFPRRSLYGYAIGNKISDTRKIDLRRKKASKQYLRTLENMTHNV EFDPTTQFMVDQELNVTITARVKKIPPVRDVPVGSRIMGLNKNQ TIRDCYSIWERVEANTSGSFQHPVVQDVWLRLVADGHVTSTVNG PNGQIDQMSYSGPDVANCGNWMQERSNFITSVDDADQSIMKHFL WNNRVKLYAWNCVYAGTLMKAISKQMTTANRNLFRNEILSFVG GRFGCRLGSLAQVSLDLLRRMKSVISCYFSRNNAFTIEERSAFDK ELMELFESLDNKMNNKRKEKVNRTLSSIFQVAKQHNVVRLVVT KNLQTPDANVRSVMNQRSVDWCARKIEEKIVDLGNVIGIYVDLV EALDLSHLDPFIYIAKGDVGKECQFDEVESSSLLPIHTSMFRDWH GLVNKGRTTTPIYHSALRSFASHYGLRFDDLARIKAAELAKMLP DQKVFIPHRGGRFFMSTYPVTSTSRPVSYDGRQRFLNSGDMVAP VNTMLRGFRRARSAYPYDVPDYASLGSGSPKKKRKVEDPKKKRKV DGIGSGSNGSSGEELLSKNYHLENEVARLKKGSGSGGSGSGGSGSGSG GSGSGGSGSGEELLSKNYHGSGSGQRGSKRTADGSEFESPKKKRKV* |
| 93 | KRAB-scFv | MPAAKRVKLDGS<RTLVTFKDVFVDFTREEWKLLDTAQQIVYRN VMLENYKNLVSLGYQLTKPDVILRLEKGEEP>GSGMGPDIVMTQS PSSLSASVGDRVTITCRSSTGAVTTSNYASWVQEKPGKLFKGLIGGTNNRA PGVPSRFSGSLIGDKATLTISSLQPEDFATYFCALWYSNHWVFGQGTKVE LKRGGGGSGGGGSGGGGSSGGGSEVKLLESGGGLVQPGGSLKLSCAVS GFSLTDYGVNWVRQAPGRGLEWIGVIWGDGITDYNSALKDRFIISKDNG KNTVYLQMSKVRSDDTALYYCVTGLFDYWGQGTLVTVSSYPYDVPDYA GGGGGSGGGGSGGGGSGGGGSGSG* |
| 94 | 1xGCN 4-dCas9-KRAB | MPAAKRVKLDGSGSGGSGSGEELLSKNYHLENEVARLKKGSGSGGSG SGGSGSGSGGSGSGGSGSGPKKKRKVMDKKYSIGLAIGTNSVGWA VITDEYKVPSKKFKVLGNTDRHSIKKNLIGALLFDSGETAEATRL KRTARRRYTRRKNRICYLQEIFSNEMAKVDDSFFHRLEESFLVEE DKKHERHPIFGNIVDEVAYHEKYPTIYHLRKKLVDSTDKADLRLI YLALAHMIKFRGHFLIEGDLNPDNSDVDKLFIQLVQTYNQLFEEN PINASGVDAKAILSARLSKSRRLENLIAQLPGEKKNGLFGNLIALS LGLTPNFKSNFDLAEDAKLQLSKDTYDDDLDNLLAQIGDQYADL FLAAKNLSDAILLSDILRVNTEITKAPLSASMIKRYDEHHQDLTLL KALVRQQLPEKYKEIFFDQSKNGYAGYIDGGASQEEFYKFIKPIL EKMDGTEELLVKLNREDLLRKQRTFDNGSIPHQIHLGELHAILR RQEDFYPFLKDNREKIEKILTFRIPYYVGPLARGNSRFAWMTRKS EETITPWNFEEVVDKGASAQSFIERMTNFDKNLPNEKVLPKHSLL YEYFTVYNELTKVKYVTEGMRKPAFLSGEQKKAIVDLLFKTNRK VTVKQLKEDYFKKIECFDSVEISGVEDRFNASLGTYHDLLKIIKD KDFLDNEENEDILEDIVLTLTLFEDREMIEERLKTYAHLFDDKVM KQLKRRRYTGWGRLSRKLINGIRDKQSGKTILDFLKSDGFANRN FMQLIHDDSLTFKEDIQKAQVSGQGDSLHEHIANLAGSPAIKKGI LQTVKVVDELVKVMGRHKPENIVIEMARENQTTQKGQKNSRER MKRIEEGIKELGSQILKEHPVENTQLQNEKLYLYYLQNGRDMYV DQELDINRLSDYDVDAIVPQSFLKDDSIDNKVLTRSDKNRGKSDN VPSEEVVKKMKNYWRQLLNAKLITQRKFDNLTKAERGGLSELD KAGFIKRQLVETRQITKHVAQILDSRMNTKYDENDKLIREVKVIT LKSKLVSDFRKDFQFYKVREINNYHHAHDAYLNAVVGTALIKKY PKLESEFVYGDYKVYDVRKMIAKSEQEIGKATAKYFFYSNIMNFF KTEITLANGEIRKRPLIETNGETGEIVWDKGRDFATVRKVLSMPQ VNIVKKTEVQTGGFSKESILPKRNSDKLIARKKDWDPKKYGGFD SPTVAYSVLVVAKVEKGKSKKLKSVKELLGITIMERSSFEKNPID FLEAKGYKEVKKDLIIKLPKYSLFELENGRKRMLASAGELQKGN ELALPSKYVNFLYLASHYEKLKGSPEDNEQKQLFVEQHKHYLDE IIEQISEFSKRVILADANLDKVLSAYNKHRDKPIREQAENIIHLFTL TNLGAPAAFKYFDTTIDRKRYTSTKEVLDATLIHQSITGLYETRID LSQLGGDAYPYDVPDYASLGSGSPKKKRKVEDPKKKRKVDGSGSE TPGTSESATPES<RTLVTFKDVFVDFTREEWKLLDTAQQIVYRNV MLENYKNLVSLGYQLTKPDVILRLEKGEEP>* |
| 95 | 2xGCN 4-dCas9-KRAB | MPAAKRVKLDGSEELLSKNYHLENEVARLKKGSGSGGSGSGGSGSGS GGSGSGGSGSGEELLSKNYHLENEVARLKKGSGSGGSGSGGSGSGSGG SGSGGSGSGPKKKRKVMDKKYSIGLAIGTNSVGWAVITDEYKVPS KKFKVLGNTDRHSIKKNLIGALLFDSGETAEATRLKRTARRRYT RRKNRICYLQEIFSNEMAKVDDSFFHRLEESFLVEEDKKHERHPI FGNIVDEVAYHEKYPTIYHLRKKLVDSTDKADLRLIYLALAHMIK FRGHFLIEGDLNPDNSDVDKLFIQLVQTYNQLFEENPINASGVDA KAILSARLSKSRRLENLIAQLPGEKKNGLFGNLIALSLGLTPNFKS NFDLAEDAKLQLSKDTYDDDLDNLLAQIGDQYADLFLAAKNLSD AILLSDILRVNTEITKAPLSASMIKRYDEHHQDLTLLKALVRQQL PEKYKEIFFDQSKNGYAGYIDGGASQEEFYKFIKPILEKMDGTEE LLVKLNREDLLRKQRTFDNGSIPHQIHLGELHAILRRQEDFYPFL KDNREKIEKILTFRIPYYVGPLARGNSRFAWMTRKSEETITPWNF EEVVDKGASAQSFIERMTNFDKNLPNEKVLPKHSLLYEYFTVYN ELTKVKYVTEGMRKPAFLSGEQKKAIVDLLFKTNRKVTVKQLK EDYFKKIECFDSVEISGVEDRFNASLGTYHDLLKIIKDKDFLDNEE NEDILEDIVLTLTLFEDREMIEERLKTYAHLFDDKVMKQLKRRR YTGWGRLSRKLINGIRDKQSGKTILDFLKSDGFANRNFMQLIHD DSLTFKEDIQKAQVSGQGDSLHEHIANLAGSPAIKKGILQTVKVV DELVKVMGRHKPENIVIEMARENQTTQKGQKNSRERMKRIEEGI KELGSQILKEHPVENTQLQNEKLYLYYLQNGRDMYVDQELDINR LSDYDVDAIVPQSFLKDDSIDNKVLTRSDKNRGKSDNVPSEEVVK KMKNYWRQLLNAKLITQRKFDNLTKAERGGLSELDKAGFIKRQ LVETRQITKHVAQILDSRMNTKYDENDKLIREVKVITLKSKLVSD FRKDFQFYKVREINNYHHAHDAYLNAVVGTALIKKYPKLESEFV YGDYKVYDVRKMIAKSEQEIGKATAKYFFYSNIMNFFKTEITLAN GEIRKRPLIETNGETGEIVWDKGRDFATVRKVLSMPQVNIVKKT EVQTGGFSKESILPKRNSDKLIARKKDWDPKKYGGFDSPTVAYS VLVVAKVEKGKSKKLKSVKELLGITIMERSSFEKNPIDFLEAKGY KEVKKDLIIKLPKYSLFELENGRKRMLASAGELQKGNELALPSK YVNFLYLASHYEKLKGSPEDNEQKQLFVEQHKHYLDEIIEQISEF SKRVILADANLDKVLSAYNKHRDKPIREQAENIIHLFTLTNLGAP AAFKYFDTTIDRKRYTSTKEVLDATLIHQSITGLYETRIDLSQLGG DAYPYDVPDYASLGSGSPKKKRKVEDPKKKRKVDGSGSETPGTSES ATPES<RTLVTFKDVFVDFTREEWKLLDTAQQIVYRNVMLENYK NLVSLGYQLTKPDVILRLEKGEEP>* |
| 96 | 5xGCN 4-dCas9-KRAB | MPAAKRVKLDGSEELLSKNYHLENEVARLKKGSGSGGSGSGGSGSGSGS GGSGSGGSGSGEELLSKNYHLENEVARLKKGSGSGGSGSGGSGSGSGG SGSGGSGSGEELLSKNYHLENEVARLKKGSGSGGSGSGGSGSGSGGSG SGGSGSGEELLSKNYHLENEVARLKKGSGSGGSGSGGSGSGSGGSGSG GSGSGEELLSKNYHLENEVARLKKGSGSGGSGSGGSGSGSGGSGSGGS GSGPKKKRKVMDKKYSIGLAIGTNSVGWAVITDEYKVPSKKFKV LGNTDRHSIKKNLIGALLFDSGETAEATRLKRTARRRYTRRKNRI CYLQEIFSNEMAKVDDSFFHRLEESFLVEEDKKHERHPIFGNIVD EVAYHEKYPTIYHLRKKLVDSTDKADLRLIYLALAHMIKFRGHF LIEGDLNPDNSDVDKLFIQLVQTYNQLFEENPINASGVDAKAILSA RLSKSRRLENLIAQLPGEKKNGLFGNLIALSLGLTPNFKSNFDLA EDAKLQLSKDTYDDDLDNLLAQIGDQYADLFLAAKNLSDAILLSD ILRVNTEITKAPLSASMIKRYDEHHQDLTLLKALVRQQLPEKYKE IFFDQSKNGYAGYIDGGASQEEFYKFIKPILEKMDGTEELLVKLN REDLLRKQRTFDNGSIPHQIHLGELHAILRRQEDFYPFLKDNREK IEKILTFRIPYYVGPLARGNSRFAWMTRKSEETITPWNFEEVVDK GASAQSFIERMTNFDKNLPNEKVLPKHSLLYEYFTVYNELTKVK YVTEGMRKPAFLSGEQKKAIVDLLFKTNRKVTVKQLKEDYFKKI ECFDSVEISGVEDRFNASLGTYHDLLKIIKDKDFLDNEENEDILED IVLTLTLFEDREMIEERLKTYAHLFDDKVMKQLKRRRYTGWGR LSRKLINGIRDKQSGKTILDFLKSDGFANRNFMQLIHDDSLTFKE DIQKAQVSGQGDSLHEHIANLAGSPAIKKGILQTVKVVDELVKV MGRHKPENIVIEMARENQTTQKGQKNSRERMKRIEEGIKELGSQ ILKEHPVENTQLQNEKLYLYYLQNGRDMYVDQELDINRLSDYDV DAIVPQSFLKDDSIDNKVLTRSDKNRGKSDNVPSEEVVKKMKNY WRQLLNAKLITQRKFDNLTKAERGGLSELDKAGFIKRQLVETRQ ITKHVAQILDSRMNTKYDENDKLIREVKVITLKSKLVSDFRKDFQ FYKVREINNYHHAHDAYLNAVVGTALIKKYPKLESEFVYGDYKV YDVRKMIAKSEQEIGKATAKYFFYSNIMNFFKTEITLANGEIRKR PLIETNGETGEIVWDKGRDFATVRKVLSMPQVNIVKKTEVQTGG FSKESILPKRNSDKLIARKKDWDPKKYGGFDSPTVAYSVLVVAK VEKGKSKKLKSVKELLGITIMERSSFEKNPIDFLEAKGYKEVKKD LIIKLPKYSLFELENGRKRMLASAGELQKGNELALPSKYVNFLYL ASHYEKLKGSPEDNEQKQLFVEQHKHYLDEIIEQISEFSKRVILA DANLDKVLSAYNKHRDKPIREQAENIIHLFTLTNLGAPAAFKYFD TTIDRKRYTSTKEVLDATLIHQSITGLYETRIDLSQLGGDAYPYDV PDYASLGSGSPKKKRKVEDPKKKRKVDGSGSETPGTSESATPES<RT LVTFKDVFVDFTREEWKLLDTAQQIVYRNVMLENYKNLVSLGY QLTKPDVILRLEKGEEP>* |
| 97 | DNMT 3A-hDNM T3L-scFv | MPAAKRVKLDGSNHDQEFDPPKVYPPVPAEKRKPIRVLSLFDGIATG LLVLKDLGIQVDRYIASEVCEDSITVGMVRHQGKIMYVGDVRSVTQK HIQEWGPFDLVIGGSPCNDLSIVNPARKGLYEGTGRLFFEFYRLLHD ARPKEGDDRPFFWLFENVVAMGVSDKRDISRFLESNPVMIDAKEVS AAHRARYFWGNLPGMNRPLASTVNDKLELQECLEHGRIAKFSKVRT ITTRSNSIKQGKDQHFPVFMNEKEDILWCTEMERVFGFPVHYTDVS NMSRLARQRLLGRSWSVPVIRHLFAPLKEYFACVSSGNSNANSRGPS FSSGLVPLSLRGSHMFETVPVWRRQPVRVLSLFEDIKKELTSLGFLES GSDPGQLKHVVDVTDTVRKDVEEWGPFDLVYGATPPLGHTCDRPPS WYLFQFHRLLQYARPKPGSPRPFFWMFVDNLVLNKEDLDVASRFLE MEPVTIPDVHGGSLQNAVRVWSNIPAIRSSRHWALVSEEELSLLAQN KQSSKLAAKWPTKLVKNCFLPLREYFKYFSGGPSSGAPPPSGGSPAGS PTSTEEGTSESATPESGPGTSTEPSEGSAPGSPAGSPTSTEEGTSTEPSE GSAPGTSTEPSEPKKKRKVPSEGPDIVMTQSPSSLSASVGDRVTITCRSST GAVTTSNYASWVQEKPGKLFKGLIGGTNNRAPGVPSRFSGSLIGDKATLTI SSLQPEDFATYFCALWYSNHWVFGQGTKVELKRGGGGSGGGGSGGGGS SGGGSEVKLLESGGGLVQPGGSLKLSCAVSGFSLTDYGVNWVRQAPGRG LEWIGVIWGDGITDYNSALKDRFIISKDNGKNTVYLQMSKVRSDDTALYY CVTGLFDYWGQGTLVTVSSYPYDVPDYAGGGGGSGGGGSGGGGSGG GGSGSG* |
| 98 | 3xGCN 4-dCas9-ZIM3 | MPAAKRVKLDGSEELLSKNYHLENEVARLKKGSGSGGSGSGGSGSGS GGSGSGGSGSGEELLSKNYHLENEVARLKKGSGSGGSGSGGSGSGSGG SGSGGSGSGEELLSKNYHLENEVARLKKGSGSGGSGSGGSGSGSGGSG SGGSGSGPKKKRKVMDKKYSIGLAIGTNSVGWAVITDEYKVPSKK FKVLGNTDRHSIKKNLIGALLFDSGETAEATRLKRTARRRYTRR KNRICYLQEIFSNEMAKVDDSFFHRLEESFLVEEDKKHERHPIFG NIVDEVAYHEKYPTIYHLRKKLVDSTDKADLRLIYLALAHMIKFR GHFLIEGDLNPDNSDVDKLFIQLVQTYNQLFEENPINASGVDAKA ILSARLSKSRRLENLIAQLPGEKKNGLFGNLIALSLGLTPNFKSNF DLAEDAKLQLSKDTYDDDLDNLLAQIGDQYADLFLAAKNLSDAI LLSDILRVNTEITKAPLSASMIKRYDEHHQDLTLLKALVRQQLPE KYKEIFFDQSKNGYAGYIDGGASQEEFYKFIKPILEKMDGTEELL VKLNREDLLRKQRTFDNGSIPHQIHLGELHAILRRQEDFYPFLKD NREKIEKILTFRIPYYVGPLARGNSRFAWMTRKSEETITPWNFEE VVDKGASAQSFIERMTNFDKNLPNEKVLPKHSLLYEYFTVYNEL TKVKYVTEGMRKPAFLSGEQKKAIVDLLFKTNRKVTVKQLKED YFKKIECFDSVEISGVEDRFNASLGTYHDLLKIIKDKDFLDNEENE DILEDIVLTLTLFEDREMIEERLKTYAHLFDDKVMKQLKRRRYT GWGRLSRKLINGIRDKQSGKTILDFLKSDGFANRNFMQLIHDDSL TFKEDIQKAQVSGQGDSLHEHIANLAGSPAIKKGILQTVKVVDEL VKVMGRHKPENIVIEMARENQTTQKGQKNSRERMKRIEEGIKE LGSQILKEHPVENTQLQNEKLYLYYLQNGRDMYVDQELDINRLS DYDVDAIVPQSFLKDDSIDNKVLTRSDKNRGKSDNVPSEEVVKK MKNYWRQLLNAKLITQRKFDNLTKAERGGLSELDKAGFIKRQL VETRQITKHVAQILDSRMNTKYDENDKLIREVKVITLKSKLVSDF RKDFQFYKVREINNYHHAHDAYLNAVVGTALIKKYPKLESEFVY GDYKVYDVRKMIAKSEQEIGKATAKYFFYSNIMNFFKTEITLAN GEIRKRPLIETNGETGEIVWDKGRDFATVRKVLSMPQVNIVKKT EVQTGGFSKESILPKRNSDKLIARKKDWDPKKYGGFDSPTVAYS VLVVAKVEKGKSKKLKSVKELLGITIMERSSFEKNPIDFLEAKGY KEVKKDLIIKLPKYSLFELENGRKRMLASAGELQKGNELALPSK YVNFLYLASHYEKLKGSPEDNEQKQLFVEQHKHYLDEIIEQISEF SKRVILADANLDKVLSAYNKHRDKPIREQAENIIHLFTLTNLGAP AAFKYFDTTIDRKRYTSTKEVLDATLIHQSITGLYETRIDLSQLGG DAYPYDVPDYASLGSGSPKKKRKVEDPKKKRKVDGSGSETPGTSES ATPES<MNNSQGRVTFEDVTVNFTQGEWQRLNPEQRNLYRDVM LENYSNLVSVGQGETTKPDVILRLEQGKEPWLEEEEVLGSGRAE KNGDIGGQIWKPKDVKESL>* |
| 99 | 3xGCN 4-dCas12 a-ZIM3 | MPAAKRVKLDGSEELLSKNYHLENEVARLKKGSGSGGSGSGGSGSGS GGSGSGGSGSGEELLSKNYHLENEVARLKKGSGSGGSGSGGSGSGSGG SGSGGSGSGEELLSKNYHLENEVARLKKGSGSGGSGSGGSGSGSGGSG SGGSGSGPKKKRKVMSKLEKFTNCYSLSKTLRFKAIPVGKTQENI DNKRLLVEDEKRAEDYKGVKKLLDRYYLSFINDVLHSIKLKNLN NYISLFRKKTRTEKENKELENLEINLRKEIAKAFKGNEGYKSLFK KDIIETILPEFLDDKDEIALVNSFNGFTTAFTGFFDNRENMFSEEA KSTSIAFRCINENLTRYISNMDIFEKVDAIFDKHEVQEIKEKILNSD YDVEDFFEGEFFNFVLTQEGIDVYNAIIGGFVTESGEKIKGLNEYI NLYNQKTKQKLPKFKPLYKQVLSDRESLSFYGEGYTSDEEVLEV FRNTLNKNSEIFSSIKKLEKLFKNFDEYSSAGIFVKNGPAISTISKDI FGEWNVIRDKWNAEYDDIHLKKKAVVTEKYEDDRRKSFKKIGSF SLEQLQEYADADLSVVEKLKEIIIQKVDEIYKVYGSSEKLFDADFV LEKSLKKNDAVVAIMKDLLDSVKSFENYIKAFFGEGKETNRDESF YGDFVLAYDILLKVDHIYDAIRNYVTQKPYSKDKFKLYFQNPQF MGGWDKDKETDYRATILRYGSKYYLAIMDKKYAKCLQKIDKDD VNGNYEKINYKLLPGPNKMLPKVFFSKKWMAYYNPSEDIQKIYK NGTFKKGDMFNLNDCHKLIDFFKDSISRYPKWSNAYDFNFSETE KYKDIAGFYREVEEQGYKVSFESASKKEVDKLVEEGKLYMFQIY NKDFSDKSHGTPNLHTMYFKLLFDENNHGQIRLSGGAELFMRRA SLKKEELVVHPANSPIANKNPDNPKKTTTLSYDVYKDKRFSEDQY ELHIPIAINKCPKNIFKINTEVRVLLKHDDNPYVIGIARGERNLLYI VVVDGKGNIVEQYSLNEIINNFNGIRIKTDYHSLLDKKEKERFEA RQNWTSIENIKELKAGYISQVVHKICELVEKYDAVIALADLNSGF KNSRVKVEKQVYQKFEKMLIDKLNYMVDKKSNPCATGGALKG YQITNKFESFKSMSTQNGFIFYIPAWLTSKIDPSTGFVNLLKTKYT SIADSKKFISSFDRIMYVPEEDLFEFALDYKNFSRTDADYIKKWKL YSYGNRIRIFRNPKKNNVFDWEEVCLTSAYKELFNKYGINYQQG DIRALLCEQSDKAFYSSFMALMSLMLQMRNSITGRTDVAFLISPV KNSDGIFYDSRNYEAQENAILPKNADANGAYNIARKVLWAIGQF KKAEDEKLDKVKIAISNKEWLEYAQTSVKHAYPYDVPDYASLGS GSPKKKRKVEDPKKKRKVDGSGSETPGTSESATPES<MNNSQGRVT FEDVTVNFTQGEWQRLNPEQRNLYRDVMLENYSNLVSVGQGET TKPDVILRLEQGKEPWLEEEEVLGSGRAEKNGDIGGQIWKPKDV KESL>* |
| 100 | 3xGCN 4-dCas12 i-ZIM3 | MPAAKRVKLDGSEELLSKNYHLENEVARLKKGSGSGGSGSGGSGSGS GGSGSGGSGSGEELLSKNYHLENEVARLKKGSGSGGSGSGGSGSGSGG SGSGGSGSGEELLSKNYHLENEVARLKKGSGSGGSGSGGSGSGSGGSG SGGSGSGPKKKRKVMGITMSDTTFRPYKSKLNPNERKMPMVVDT MNWCDNACSVFFDLFVGLYGGVKIGDVTNFLTEKQDTTTDADL LCAINWFRAMPVSNPDAVVGPEQLVVRFRDYSGHNPSPMASMYL SAPVNPRYTWVDCRFLYQQMCQTLGANLDVDISTLVQTGLLPIQ KDTQLNAYSFCSNVFGEGVKEDPSAKVTSYTAAANALSGATNPII TYEDYRQILSSAITNRPSRKGAKDILAADLDSTKTGVLPGNYVSQ RIAMYQASAASKARGVVLPNRLRVKAYLQNLVGLNYICTTWAA PMLRAVGDIKSKNSNNYRFVGERLERQQEIDNLLAVSNIRSAQAI LQQFRANESNQFYIKKRHIGKGLPSLYDMWDLMDEKEGIDNYIQ SLKGDKTERLPIRELLQIIYPHRKSVSAELFLSAAELTDVEDRQNR CMVHPTVRGSLVINFGKSSTIFGAITPPDTLIKFRGEMVPAGQTG MIWFTMHLVDGGKWVKHHIPCHSSRYFEEIYAWKKDLPTLPFP RRSLYGYAIGNKISDTRKIDLRRKKASKQYLRTLENMTHNVEFD PTTQFMVDQELNVTITARVKKIPPVRDVPVGSRIMGLNKNQTIRD CYSIWERVEANTSGSFQHPVVQDVWLRLVADGHVTSTVNGPNG QIDQMSYSGPDVANCGNWMQERSNFITSVDDADQSIMKHFLWN NRVKLYAWNCVYAGTLMKAISKQMTTANRNLFRNEILSFVGGR FGCRLGSLAQVSLDLLRRMKSVISCYFSRNNAFTIEERSAFDKEL MELFESLDNKMNNKRKEKVNRTLSSIFQVAKQHNVVRLVVTKN LQTPDANVRSVMNQRSVDWCARKIEEKIVDLGNVIGIYVDLVEA LDLSHLDPFIYIAKGDVGKECQFDEVESSSLLPIHTSMFRDWHGL VNKGRTTTPIYHSALRSFASHYGLRFDDLARIKAAELAKMLPDQ KVFIPHRGGRFFMSTYPVTSTSRPVSYDGRQRFLNSGDMVAPVN TMLRGFRRARSAYPYDVPDYASLGSGSPKKKRKVEDPKKKRKVDG SGSETPGTSESATPES<MNNSQGRVTFEDVTVNFTQGEWQRLNPE QRNLYRDVMLENYSNLVSVGQGETTKPDVILRLEQGKEPWLEE EEVLGSGRAEKNGDIGGQIWKPKDVKESL>* |
| 101 | 10xGC N4-dCas9-ZIM3 | MPAAKRVKLDGSEELLSKNYHLENEVARLKKGSGSGGSGSGGSGSGS GGSGSGGSGSGEELLSKNYHLENEVARLKKGSGSGGSGSGGSGSGSGG SGSGGSGSGEELLSKNYHLENEVARLKKGSGSGGSGSGGSGSGSGGSG SGGSGSGEELLSKNYHLENEVARLKKGSGSGGSGSGGSGSGSGGSGSG GSGSGEELLSKNYHLENEVARLKKGSGSGGSGSGGSGSGSGGSGSGGS GSGEELLSKNYHLENEVARLKKGSGSGGSGSGGSGSGSGGSGSGGSGS GEELLSKNYHLENEVARLKKGSGSGGSGSGGSGSGSGGSGSGGSGSGE ELLSKNYHLENEVARLKKGSGSGGSGSGGSGSGSGGSGSGGSGSGEEL LSKNYHLENEVARLKKGSGSGGSGSGGSGSGSGGSGSGGSGSGEELLS KNYHLENEVARLKKGSGSGPKKKRKVMDKKYSIGLAIGTNSVGWAV ITDEYKVPSKKFKVLGNTDRHSIKKNLIGALLFDSGETAEATRLK RTARRRYTRRKNRICYLQEIFSNEMAKVDDSFFHRLEESFLVEED KKHERHPIFGNIVDEVAYHEKYPTIYHLRKKLVDSTDKADLRLIY LALAHMIKFRGHFLIEGDLNPDNSDVDKLFIQLVQTYNQLFEENP INASGVDAKAILSARLSKSRRLENLIAQLPGEKKNGLFGNLIALSL GLTPNFKSNFDLAEDAKLQLSKDTYDDDLDNLLAQIGDQYADLF LAAKNLSDAILLSDILRVNTEITKAPLSASMIKRYDEHHQDLTLLK ALVRQQLPEKYKEIFFDQSKNGYAGYIDGGASQEEFYKFIKPILE KMDGTEELLVKLNREDLLRKQRTFDNGSIPHQIHLGELHAILRR QEDFYPFLKDNREKIEKILTFRIPYYVGPLARGNSRFAWMTRKSE ETITPWNFEEVVDKGASAQSFIERMTNFDKNLPNEKVLPKHSLLY EYFTVYNELTKVKYVTEGMRKPAFLSGEQKKAIVDLLFKTNRKV TVKQLKEDYFKKIECFDSVEISGVEDRFNASLGTYHDLLKIIKDK DFLDNEENEDILEDIVLTLTLFEDREMIEERLKTYAHLFDDKVMK QLKRRRYTGWGRLSRKLINGIRDKQSGKTILDFLKSDGFANRNF MQLIHDDSLTFKEDIQKAQVSGQGDSLHEHIANLAGSPAIKKGIL QTVKVVDELVKVMGRHKPENIVIEMARENQTTQKGQKNSRERM KRIEEGIKELGSQILKEHPVENTQLQNEKLYLYYLQNGRDMYVD QELDINRLSDYDVDAIVPQSFLKDDSIDNKVLTRSDKNRGKSDNV PSEEVVKKMKNYWRQLLNAKLITQRKFDNLTKAERGGLSELDK AGFIKRQLVETRQITKHVAQILDSRMNTKYDENDKLIREVKVITL KSKLVSDFRKDFQFYKVREINNYHHAHDAYLNAVVGTALIKKYP KLESEFVYGDYKVYDVRKMIAKSEQEIGKATAKYFFYSNIMNFF KTEITLANGEIRKRPLIETNGETGEIVWDKGRDFATVRKVLSMPQ VNIVKKTEVQTGGFSKESILPKRNSDKLIARKKDWDPKKYGGFD SPTVAYSVLVVAKVEKGKSKKLKSVKELLGITIMERSSFEKNPID FLEAKGYKEVKKDLIIKLPKYSLFELENGRKRMLASAGELQKGN ELALPSKYVNFLYLASHYEKLKGSPEDNEQKQLFVEQHKHYLDE IIEQISEFSKRVILADANLDKVLSAYNKHRDKPIREQAENIIHLFTL TNLGAPAAFKYFDTTIDRKRYTSTKEVLDATLIHQSITGLYETRID LSQLGGDAYPYDVPDYASLGSGSPKKKRKVEDPKKKRKVDGSGSE TPGTSESATPES<MNNSQGRVTFEDVTVNFTQGEWQRLNPEQRNL YRDVMLENYSNLVSVGQGETTKPDVILRLEQGKEPWLEEEEVLG SGRAEKNGDIGGQIWKPKDVKESL>* |
| 102 | scFv-hDNM T3L-DNMT 3A | MPAAKRVKLDGSGPDIVMTQSPSSLSASVGDRVTITCRSSTGAVTTSNYA SWVQEKPGKLFKGLIGGTNNRAPGVPSRFSGSLIGDKATLTISSLQPEDF ATYFCALWYSNHWVFGQGTKVELKRGGGGSGGGGSGGGGSSGGGSEV KLLESGGGLVQPGGSLKLSCAVSGFSLTDYGVNWVRQAPGRGLEWIGVI WGDGITDYNSALKDRFIISKDNGKNTVYLQMSKVRSDDTALYYCVTGLFD YWGQGTLVTVSSYPYDVPDYAGGGGGSGGGGSGGGGSGGGGSMFET VPVWRRQPVRVLSLFEDIKKELTSLGFLESGSDPGQLKHVVDVTDTV RKDVEEWGPFDLVYGATPPLGHTCDRPPSWYLFQFHRLLQYARPKP GSPRPFFWMFVDNLVLNKEDLDVASRFLEMEPVTIPDVHGGSLQNA VRVWSNIPAIRSSRHWALVSEEELSLLAQNKQSSKLAAKWPTKLVKN CFLPLREYFKYFSTELTSSLSSGNSNANSRGPSFSSGLVPLSLRGSHM NHDQEFDPPKVYPPVPAEKRKPIRVLSLFDGIATGLLVLKDLGIQVD RYIASEVCEDSITVGMVRHQGKIMYVGDVRSVTQKHIQEWGPFDLVI GGSPCNDLSIVNPARKGLYEGTGRLFFEFYRLLHDARPKEGDDRPFF WLFENVVAMGVSDKRDISRFLESNPVMIDAKEVSAAHRARYFWGNL PGMNRPLASTVNDKLELQECLEHGRIAKFSKVRTITTRSNSIKQGKD QHFPVFMNEKEDILWCTEMERVFGFPVHYTDVSNMSRLARQRLLG RSWSVPVIRHLFAPLKEYFACV* |
| 103 | 1xGCN 4-ZIM3 | MPAAKRVKLDGS*EELLSKNYHLENEVARLKK*GSGSGGSGSGGSGSGS GGSGSGGSGSG<**MNNSQGRVTFEDVTVNFTQGEWQRLNPEQRNL** **YRDVMLENYSNLVSVGQGETTKPDVILRLEQGKEPWLEEEEVLG** **SGRAEKNGDIGGQIWKPKDVKESL**>* |
| 104 | DNMT 3A-hDNM T3L-dCas9-scFv | MPAAKRVKLDGSNHDQEFDPPKVYPPVPAEKRKPIRVLSLFDGIATG LLVLKDLGIQVDRYIASEVCEDSITVGMVRHQGKIMYVGDVRSVTQK HIQEWGPFDLVIGGSPCNDLSIVNPARKGLYEGTGRLFFEFYRLLHD ARPKEGDDRPFFWLFENVVAMGVSDKRDISRFLESNPVMIDAKEVS AAHRARYFWGNLPGMNRPLASTVNDKLELQECLEHGRIAKFSKVRT ITTRSNSIKQGKDQHFPVFMNEKEDILWCTEMERVFGFPVHYTDVS NMSRLARQRLLGRSWSVPVIRHLFAPLKEYFACVSSGNSNANSRGPS FSSGLVPLSLRGSHMFETVPVWRRQPVRVLSLFEDIKKELTSLGFLES GSDPGQLKHVVDVTDTVRKDVEEWGPFDLVYGATPPLGHTCDRPPS WYLFQFHRLLQYARPKPGSPRPFFWMFVDNLVLNKEDLDVASRFLE MEPVTIPDVHGGSLQNAVRVWSNIPAIRSSRHWALVSEEELSLLAQN KQSSKLAAKWPTKLVKNCFLPLREYFKYFSGGPSSGAPPPSGGSPAGS PTSTEEGTSESATPESGPGTSTEPSEGSAPGSPAGSPTSTEEGTSTEPSE GSAPGTSTEPSEPKKKRKVMDKKYSIGLAIGTNSVGWAVITDEYKV PSKKFKVLGNTDRHSIKKNLIGALLFDSGETAEATRLKRTARRRY TRRKNRICYLQEIFSNEMAKVDDSFFHRLEESFLVEEDKKHERHP IFGNIVDEVAYHEKYPTIYHLRKKLVDSTDKADLRLIYLALAHMI KFRGHFLIEGDLNPDNSDVDKLFIQLVQTYNQLFEENPINASGVD AKAILSARLSKSRRLENLIAQLPGEKKNGLFGNLIALSLGLTPNFK SNFDLAEDAKLQLSKDTYDDDLDNLLAQIGDQYADLFLAAKNLS DAILLSDILRVNTEITKAPLSASMIKRYDEHHQDLTLLKALVRQQ LPEKYKEIFFDQSKNGYAGYIDGGASQEEFYKFIKPILEKMDGTE ELLVKLNREDLLRKQRTFDNGSIPHQIHLGELHAILRRQEDFYPF LKDNREKIEKILTFRIPYYVGPLARGNSRFAWMTRKSEETITPWN FEEVVDKGASAQSFIERMTNFDKNLPNEKVLPKHSLLYEYFTVY NELTKVKYVTEGMRKPAFLSGEQKKAIVDLLFKTNRKVTVKQL KEDYFKKIECFDSVEISGVEDRFNASLGTYHDLLKIIKDKDFLDNE ENEDILEDIVLTLTLFEDREMIEERLKTYAHLFDDKVMKQLKRR RYTGWGRLSRKLINGIRDKQSGKTILDFLKSDGFANRNFMQLIH DDSLTFKEDIQKAQVSGQGDSLHEHIANLAGSPAIKKGILQTVKV VDELVKVMGRHKPENIVIEMARENQTTQKGQKNSRERMKRIEE GIKELGSQILKEHPVENTQLQNEKLYLYYLQNGRDMYVDQELDI NRLSDYDVDAIVPQSFLKDDSIDNKVLTRSDKNRGKSDNVPSEEV VKKMKNYWRQLLNAKLITQRKFDNLTKAERGGLSELDKAGFIK RQLVETRQITKHVAQILDSRMNTKYDENDKLIREVKVITLKSKLV SDFRKDFQFYKVREINNYHHAHDAYLNAVVGTALIKKYPKLESE FVYGDYKVYDVRKMIAKSEQEIGKATAKYFFYSNIMNFFKTEITL ANGEIRKRPLIETNGETGEIVWDKGRDFATVRKVLSMPQVNIVK KTEVQTGGFSKESILPKRNSDKLIARKKDWDPKKYGGFDSPTVA YSVLVVAKVEKGKSKKLKSVKELLGITIMERSSFEKNPIDFLEAK GYKEVKKDLIIKLPKYSLFELENGRKRMLASAGELQKGNELALP SKYVNFLYLASHYEKLKGSPEDNEQKQLFVEQHKHYLDEIIEQIS EFSKRVILADANLDKVLSAYNKHRDKPIREQAENIIHLFTLTNLG APAAFKYFDTTIDRKRYTSTKEVLDATLIHQSITGLYETRIDLSQL GGDAYPYDVPDYASLGSGSPKKKRKVEDPKKKRKVDGIGSGSGPDI VMTQSPSSLSASVGDRVTITCRSSTGAVTTSNYASWVQEKPGKLFKGLIGG TNNRAPGVPSRFSGSLIGDKATLTISSLQPEDFATYFCALWYSNHWVFGQ GTKVELKRGGGGSGGGGSGGGGSSGGGSEVKLLESGGGLVQPGGSLKL SCAVSGFSLTDYGVNWVRQAPGRGLEWIGVIWGDGITDYNSALKDRFIIS KDNGKNTVYLQMSKVRSDDTALYYCVTGLFDYWGQGTLVTVSSYPYDV PDYAGGGGGSGGGGSGGGGSGGGGS* |
| 105 | KRAB-1xGCN 4 | MPAAKRVKLDGS<RTLVTFKDVFVDFTREEWKLLDTAQQIVYRN VMLENYKNLVSLGYQLTKPDVILRLEKGEEP>GSGMEELLSKNYH LENEVARLKKGSGSGGSGSGGSGSGSGGSGSGGSGSGGSG* |
| 106 | DNMT 3A-DNMT 3L-1xGCN 4 | MPAAKRVKLDGSNHDQEFDPPKVYPPVPAEKRKPIRVLSLFDGIATG LLVLKDLGIQVDRYIASEVCEDSITVGMVRHQGKIMYVGDVRSVTQK HIQEWGPFDLVIGGSPCNDLSIVNPARKGLYEGTGRLFFEFYRLLHD ARPKEGDDRPFFWLFENVVAMGVSDKRDISRFLESNPVMIDAKEVS AAHRARYFWGNLPGMNRPLASTVNDKLELQECLEHGRIAKFSKVRT ITTRSNSIKQGKDQHFPVFMNEKEDILWCTEMERVFGFPVHYTDVS NMSRLARQRLLGRSWSVPVIRHLFAPLKEYFACVSSGNSNANSRGPS FSSGLVPLSLRGSHMGPMEIYKTVSAWKRQPVRVLSLFRNIDKVLKSL GFLESGSGSGGGTLKYVEDVTNVVRRDVEKWGPFDLVYGSTQPLGS SCDRCPGWYMFQFHRILQYALPRQESQRPFFWIFMDNLLLTEDDQE TTTRFLQTEAVTLQDVRGRDYQNAMRVWSNIPGLKSKHAPLTPKEE EYLQAQVRSRSKLDAPKVDLLVKNCLLPLREYFKYFSQNSLPLGGPSS GAPPPSGGSPAGSPTSTEEGTSESATGSGSGGSGSGGSGSGSGGSGSG GSGSGEELLSKNYHLENEVARLKKGSGSGGSGSGGSGSGSGGSGSGGS GSGPKKKRKV* |
| 107 | scFv-dCas9-KRAB | MPAAKRVKLDGSGPDIVMTQSPSSLSASVGDRVTITCRSSTGAVTTSNYA SWVQEKPGKLFKGLIGGTNNRAPGVPSRFSGSLIGDKATLTISSLQPEDF ATYFCALWYSNHWVFGQGTKVELKRGGGGSGGGGSGGGGSSGGGSEV KLLESGGGLVQPGGSLKLSCAVSGFSLTDYGVNWVRQAPGRGLEWIGVI WGDGITDYNSALKDRFIISKDNGKNTVYLQMSKVRSDDTALYYCVTGLFD YWGQGTLVTVSSYPYDVPDYAGGGGGSGGGGSGGGGSGGGGSGSGP KKKRKVMDKKYSIGLAIGTNSVGWAVITDEYKVPSKKFKVLGNT DRHSIKKNLIGALLFDSGETAEATRLKRTARRRYTRRKNRICYLQ EIFSNEMAKVDDSFFHRLEESFLVEEDKKHERHPIFGNIVDEVAY HEKYPTIYHLRKKLVDSTDKADLRLIYLALAHMIKFRGHFLIEGD LNPDNSDVDKLFIQLVQTYNQLFEENPINASGVDAKAILSARLSKS RRLENLIAQLPGEKKNGLFGNLIALSLGLTPNFKSNFDLAEDAKL QLSKDTYDDDLDNLLAQIGDQYADLFLAAKNLSDAILLSDILRVN TEITKAPLSASMIKRYDEHHQDLTLLKALVRQQLPEKYKEIFFDQ SKNGYAGYIDGGASQEEFYKFIKPILEKMDGTEELLVKLNREDLL RKQRTFDNGSIPHQIHLGELHAILRRQEDFYPFLKDNREKIEKILT FRIPYYVGPLARGNSRFAWMTRKSEETITPWNFEEVVDKGASAQ SFIERMTNFDKNLPNEKVLPKHSLLYEYFTVYNELTKVKYVTEG MRKPAFLSGEQKKAIVDLLFKTNRKVTVKQLKEDYFKKIECFDS VEISGVEDRFNASLGTYHDLLKIIKDKDFLDNEENEDILEDIVLTL TLFEDREMIEERLKTYAHLFDDKVMKQLKRRRYTGWGRLSRKL INGIRDKQSGKTILDFLKSDGFANRNFMQLIHDDSLTFKEDIQKA QVSGQGDSLHEHIANLAGSPAIKKGILQTVKVVDELVKVMGRHK PENIVIEMARENQTTQKGQKNSRERMKRIEEGIKELGSQILKEHP VENTQLQNEKLYLYYLQNGRDMYVDQELDINRLSDYDVDAIVPQ SFLKDDSIDNKVLTRSDKNRGKSDNVPSEEVVKKMKNYWRQLL NAKLITQRKFDNLTKAERGGLSELDKAGFIKRQLVETRQITKHV AQILDSRMNTKYDENDKLIREVKVITLKSKLVSDFRKDFQFYKVR EINNYHHAHDAYLNAVVGTALIKKYPKLESEFVYGDYKVYDVRK MIAKSEQEIGKATAKYFFYSNIMNFFKTEITLANGEIRKRPLIETN GETGEIVWDKGRDFATVRKVLSMPQVNIVKKTEVQTGGFSKESI LPKRNSDKLIARKKDWDPKKYGGFDSPTVAYSVLVVAKVEKGK SKKLKSVKELLGITIMERSSFEKNPIDFLEAKGYKEVKKDLIIKLP KYSLFELENGRKRMLASAGELQKGNELALPSKYVNFLYLASHYE KLKGSPEDNEQKQLFVEQHKHYLDEIIEQISEFSKRVILADANLD KVLSAYNKHRDKPIREQAENIIHLFTLTNLGAPAAFKYFDTTIDR KRYTSTKEVLDATLIHQSITGLYETRIDLSQLGGDAYPYDVPDYA SLGSGSPKKKRKVEDPKKKRKVDGSGSETPGTSESATPES<RTLVTF KDVFVDFTREEWKLLDTAQQIVYRNVMLENYKNLVSLGYQLTK PDVILRLEKGEEP>* |
| 108 | 1xGCN 4-DNMT 3L-DNMT 3A | MPAAKRVKLDGSGSGGSGSGEELLSKNYHLENEVARLKKGSGSGGSG SGGSGSGSGGSGSGGSGSGTPGTSEGGPSSGAPPPSGGSPAGSPTSTEE GTSESATPESGPGTSTEPSEGSAPGSPAGSPTSTEEGTSTEPSEGSAPGT STEPSESATPESMFETVPVWRRQPVRVLSLFEDIKKELTSLGFLESGS DPGQLKHVVDVTDTVRKDVEEWGPFDLVYGATPPLGHTCDRPPSW YLFQFHRLLQYARPKPGSPRPFFWMFVDNLVLNKEDLDVASRFLEM EPVTIPDVHGGSLQNAVRVWSNIPAIRSSRHWALVSEEELSLLAQNK QSSKLAAKWPTKLVKNCFLPLREYFKYFSTELTSSLSSGNSNANSRGP SFSSGLVPLSLRGSHMNHDQEFDPPKVYPPVPAEKRKPIRVLSLFDGI ATGLLVLKDLGIQVDRYIASEVCEDSITVGMVRHQGKIMYVGDVRSV TQKHIQEWGPFDLVIGGSPCNDLSIVNPARKGLYEGTGRLFFEFYRL LHDARPKEGDDRPFFWLFENVVAMGVSDKRDISRFLESNPVMIDAK EVSAAHRARYFWGNLPGMNRPLASTVNDKLELQECLEHGRIAKFSK VRTITTRSNSIKQGKDQHFPVFMNEKEDILWCTEMERVFGFPVHYT DVSNMSRLARQRLLGRSWSVPVIRHLFAPLKEYFACV* |
| 109 | scFv-dCas9-HPla | MPAAKRVKLDGSGPDIVMTQSPSSLSASVGDRVTITCRSSTGAVTTSNYA SWVQEKPGKLFKGLIGGTNNRAPGVPSRFSGSLIGDKATLTISSLQPEDF ATYFCALWYSNHWVFGQGTKVELKRGGGGSGGGGSGGGGSSGGGSEV KLLESGGGLVQPGGSLKLSCAVSGFSLTDYGVNWVRQAPGRGLEWIGVI WGDGITDYNSALKDRFIISKDNGKNTVYLQMSKVRSDDTALYYCVTGLFD YWGQGTLVTVSSYPYDVPDYAGGGGGSGGGGSGGGGSGGGGSGSGP KKKRKVMDKKYSIGLAIGTNSVGWAVITDEYKVPSKKFKVLGNT DRHSIKKNLIGALLFDSGETAEATRLKRTARRRYTRRKNRICYLQ EIFSNEMAKVDDSFFHRLEESFLVEEDKKHERHPIFGNIVDEVAY HEKYPTIYHLRKKLVDSTDKADLRLIYLALAHMIKFRGHFLIEGD LNPDNSDVDKLFIQLVQTYNQLFEENPINASGVDAKAILSARLSKS RRLENLIAQLPGEKKNGLFGNLIALSLGLTPNFKSNFDLAEDAKL QLSKDTYDDDLDNLLAQIGDQYADLFLAAKNLSDAILLSDILRVN TEITKAPLSASMIKRYDEHHQDLTLLKALVRQQLPEKYKEIFFDQ SKNGYAGYIDGGASQEEFYKFIKPILEKMDGTEELLVKLNREDLL RKQRTFDNGSIPHQIHLGELHAILRRQEDFYPFLKDNREKIEKILT FRIPYYVGPLARGNSRFAWMTRKSEETITPWNFEEVVDKGASAQ SFIERMTNFDKNLPNEKVLPKHSLLYEYFTVYNELTKVKYVTEG MRKPAFLSGEQKKAIVDLLFKTNRKVTVKQLKEDYFKKIECFDS VEISGVEDRFNASLGTYHDLLKIIKDKDFLDNEENEDILEDIVLTL TLFEDREMIEERLKTYAHLFDDKVMKQLKRRRYTGWGRLSRKL INGIRDKQSGKTILDFLKSDGFANRNFMQLIHDDSLTFKEDIQKA QVSGQGDSLHEHIANLAGSPAIKKGILQTVKVVDELVKVMGRHK PENIVIEMARENQTTQKGQKNSRERMKRIEEGIKELGSQILKEHP VENTQLQNEKLYLYYLQNGRDMYVDQELDINRLSDYDVDAIVPQ SFLKDDSIDNKVLTRSDKNRGKSDNVPSEEVVKKMKNYWRQLL NAKLITQRKFDNLTKAERGGLSELDKAGFIKRQLVETRQITKHV AQILDSRMNTKYDENDKLIREVKVITLKSKLVSDFRKDFQFYKVR EINNYHHAHDAYLNAVVGTALIKKYPKLESEFVYGDYKVYDVRK MIAKSEQEIGKATAKYFFYSNIMNFFKTEITLANGEIRKRPLIETN GETGEIVWDKGRDFATVRKVLSMPQVNIVKKTEVQTGGFSKESI LPKRNSDKLIARKKDWDPKKYGGFDSPTVAYSVLVVAKVEKGK SKKLKSVKELLGITIMERSSFEKNPIDFLEAKGYKEVKKDLIIKLP KYSLFELENGRKRMLASAGELQKGNELALPSKYVNFLYLASHYE KLKGSPEDNEQKQLFVEQHKHYLDEIIEQISEFSKRVILADANLD KVLSAYNKHRDKPIREQAENIIHLFTLTNLGAPAAFKYFDTTIDR KRYTSTKEVLDATLIHQSITGLYETRIDLSQLGGDAYPYDVPDYA SLGSGSPKKKRKVEDPKKKRKVDGSGSETPGTSESATPESRTL<GKK TKRTADSSSSEDEEEYVVEKVLDRRVVKGQVEYLLKWKGFSEEH NTWEPEKNLDCPELISEFMKKYKKMKEGENNKPREKSESNKRK SNFSNSADDIKSKKKREQSNDIARGFERGLEPEKIIGATDSCGDLM FLMKWKDTDEADLVLAKEANVKCPQIVIAFYEERLTWHAYPED AENKEKETAKSE>* |
| 110 | DNMT 3A-DNMT 3L-dCas9-7xGFP 11 | PAAKRVKLDGSNHDQEFDPPKVYPPVPAEKRKPIRVLSLFDGIATGL LVLKDLGIQVDRYIASEVCEDSITVGMVRHQGKIMYVGDVRSVTQKH IQEWGPFDLVIGGSPCNDLSIVNPARKGLYEGTGRLFFEFYRLLHDA RPKEGDDRPFFWLFENVVAMGVSDKRDISRFLESNPVMIDAKEVSA AHRARYFWGNLPGMNRPLASTVNDKLELQECLEHGRIAKFSKVRTI TTRSNSIKQGKDQHFPVFMNEKEDILWCTEMERVFGFPVHYTDVSN MSRLARQRLLGRSWSVPVIRHLFAPLKEYFACVSSGNSNANSRGPSF SSGLVPLSLRGSHMGPMEIYKTVSAWKRQPVRVLSLFRNIDKVLKSL GFLESGSGSGGGTLKYVEDVTNVVRRDVEKWGPFDLVYGSTQPLGS SCDRCPGWYMFQFHRILQYALPRQESQRPFFWIFMDNLLLTEDDQE TTTRFLQTEAVTLQDVRGRDYQNAMRVWSNIPGLKSKHAPLTPKEE EYLQAQVRSRSKLDAPKVDLLVKNCLLPLREYFKYFSQNSLPLGGPSS GAPPPSGGSPAGSPTSTEEGTSESATPESGPGTSTEPSEGSAPGSPAGSP TSTEEGTSTEPSEGSAPGTSTEPSEPKKKRKVMDKKYSIGLAIGTNSV GWAVITDEYKVPSKKFKVLGNTDRHSIKKNLIGALLFDSGETAE ATRLKRTARRRYTRRKNRICYLQEIFSNEMAKVDDSFFHRLEESF LVEEDKKHERHPIFGNIVDEVAYHEKYPTIYHLRKKLVDSTDKA DLRLIYLALAHMIKFRGHFLIEGDLNPDNSDVDKLFIQLVQTYNQ LFEENPINASGVDAKAILSARLSKSRRLENLIAQLPGEKKNGLFG NLIALSLGLTPNFKSNFDLAEDAKLQLSKDTYDDDLDNLLAQIGD QYADLFLAAKNLSDAILLSDILRVNTEITKAPLSASMIKRYDEHH QDLTLLKALVRQQLPEKYKEIFFDQSKNGYAGYIDGGASQEEFY KFIKPILEKMDGTEELLVKLNREDLLRKQRTFDNGSIPHQIHLGE LHAILRRQEDFYPFLKDNREKIEKILTFRIPYYVGPLARGNSRFA WMTRKSEETITPWNFEEVVDKGASAQSFIERMTNFDKNLPNEKV LPKHSLLYEYFTVYNELTKVKYVTEGMRKPAFLSGEQKKAIVDL LFKTNRKVTVKQLKEDYFKKIECFDSVEISGVEDRFNASLGTYHD LLKIIKDKDFLDNEENEDILEDIVLTLTLFEDREMIEERLKTYAHL FDDKVMKQLKRRRYTGWGRLSRKLINGIRDKQSGKTILDFLKSD GFANRNFMQLIHDDSLTFKEDIQKAQVSGQGDSLHEHIANLAGSP AIKKGILQTVKVVDELVKVMGRHKPENIVIEMARENQTTQKGQ KNSRERMKRIEEGIKELGSQILKEHPVENTQLQNEKLYLYYLQN GRDMYVDQELDINRLSDYDVDAIVPQSFLKDDSIDNKVLTRSDKN RGKSDNVPSEEVVKKMKNYWRQLLNAKLITQRKFDNLTKAERG GLSELDKAGFIKRQLVETRQITKHVAQILDSRMNTKYDENDKLIR EVKVITLKSKLVSDFRKDFQFYKVREINNYHHAHDAYLNAVVGT ALIKKYPKLESEFVYGDYKVYDVRKMIAKSEQEIGKATAKYFFY SNIMNFFKTEITLANGEIRKRPLIETNGETGEIVWDKGRDFATVR KVLSMPQVNIVKKTEVQTGGFSKESILPKRNSDKLIARKKDWDP KKYGGFDSPTVAYSVLVVAKVEKGKSKKLKSVKELLGITIMERS SFEKNPIDFLEAKGYKEVKKDLIIKLPKYSLFELENGRKRMLASA GELQKGNELALPSKYVNFLYLASHYEKLKGSPEDNEQKQLFVEQ HKHYLDEIIEQISEFSKRVILADANLDKVLSAYNKHRDKPIREQAE NIIHLFTLTNLGAPAAFKYFDTTIDRKRYTSTKEVLDATLIHQSIT GLYETRIDLSQLGGDAYPYDVPDYASLGSGSPKKKRKVEDPKKKR KVDGSGSETPGTSESATPESGSGSNGSSGSMRDHMVLHEYVNAAGITG GSGGRDHMVLHEYVNAAGITGGSGGRDHMVLHEYVNAAGITGGSGGR DHMVLHEYVNAAGITGGSGGRDHMVLHEYVNAAGITGGSGGRDHMVL HEYVNAAGITGGSGGRDHMVLHEYVNAAGITAAARSGSGQRGSKRTA DGSEFESPKKKRKV* |
| 111 | GFP1-10-ZIM3 | MSKGEELFTGVVPILVELDGDVNGHKFSVRGEGEGDATIGKLTLKFICTT GKLPVPWPTLVTTLTYGVQCFSRYPDHMKRHDFFKSAMPEGYVQERTIS FKDDGKYKTRAVVKFEGDTLVNRIELKGTDFKEDGNILGHKLEYNFNSH NVYITADKQKNGIKANFTVRHNVEDGSVQLADHYQQNTPIGDGPVLLPD NHYLSTQTVLSKDPNEKLDPGGGGSG<MNNSQGRVTFEDVTVNFTQ GEWQRLNPEQRNLYRDVMLENYSNLVSVGQGETTKPDVILRLE QGKEPWLEEEEVLGSGRAEKNGDIGGQIWKPKDVKESL>* |
| 112 | DNMT 3A-hDNM T3L-dCas9-1xGFP 11 | MPAAKRVKLDGSNHDQEFDPPKVYPPVPAEKRKPIRVLSLFDGIATG LLVLKDLGIQVDRYIASEVCEDSITVGMVRHQGKIMYVGDVRSVTQK HIQEWGPFDLVIGGSPCNDLSIVNPARKGLYEGTGRLFFEFYRLLHD ARPKEGDDRPFFWLFENVVAMGVSDKRDISRFLESNPVMIDAKEVS AAHRARYFWGNLPGMNRPLASTVNDKLELQECLEHGRIAKFSKVRT ITTRSNSIKQGKDQHFPVFMNEKEDILWCTEMERVFGFPVHYTDVS NMSRLARQRLLGRSWSVPVIRHLFAPLKEYFACVSSGNSNANSRGPS FSSGLVPLSLRGSHMFETVPVWRRQPVRVLSLFEDIKKELTSLGFLES GSDPGQLKHVVDVTDTVRKDVEEWGPFDLVYGATPPLGHTCDRPPS WYLFQFHRLLQYARPKPGSPRPFFWMFVDNLVLNKEDLDVASRFLE MEPVTIPDVHGGSLQNAVRVWSNIPAIRSSRHWALVSEEELSLLAQN KQSSKLAAKWPTKLVKNCFLPLREYFKYFSGGPSSGAPPPSGGSPAGS PTSTEEGTSESATPESGPGTSTEPSEGSAPGSPAGSPTSTEEGTSTEPSE GSAPGTSTEPSEPKKKRKVMDKKYSIGLAIGTNSVGWAVITDEYKV PSKKFKVLGNTDRHSIKKNLIGALLFDSGETAEATRLKRTARRRY TRRKNRICYLQEIFSNEMAKVDDSFFHRLEESFLVEEDKKHERHP IFGNIVDEVAYHEKYPTIYHLRKKLVDSTDKADLRLIYLALAHMI KFRGHFLIEGDLNPDNSDVDKLFIQLVQTYNQLFEENPINASGVD AKAILSARLSKSRRLENLIAQLPGEKKNGLFGNLIALSLGLTPNFK SNFDLAEDAKLQLSKDTYDDDLDNLLAQIGDQYADLFLAAKNLS DAILLSDILRVNTEITKAPLSASMIKRYDEHHQDLTLLKALVRQQ LPEKYKEIFFDQSKNGYAGYIDGGASQEEFYKFIKPILEKMDGTE ELLVKLNREDLLRKQRTFDNGSIPHQIHLGELHAILRRQEDFYPF LKDNREKIEKILTFRIPYYVGPLARGNSRFAWMTRKSEETITPWN FEEVVDKGASAQSFIERMTNFDKNLPNEKVLPKHSLLYEYFTVY NELTKVKYVTEGMRKPAFLSGEQKKAIVDLLFKTNRKVTVKQL KEDYFKKIECFDSVEISGVEDRFNASLGTYHDLLKIIKDKDFLDNE ENEDILEDIVLTLTLFEDREMIEERLKTYAHLFDDKVMKQLKRR RYTGWGRLSRKLINGIRDKQSGKTILDFLKSDGFANRNFMQLIH DDSLTFKEDIQKAQVSGQGDSLHEHIANLAGSPAIKKGILQTVKV VDELVKVMGRHKPENIVIEMARENQTTQKGQKNSRERMKRIEE GIKELGSQILKEHPVENTQLQNEKLYLYYLQNGRDMYVDQELDI NRLSDYDVDAIVPQSFLKDDSIDNKVLTRSDKNRGKSDNVPSEEV VKKMKNYWRQLLNAKLITQRKFDNLTKAERGGLSELDKAGFIK RQLVETRQITKHVAQILDSRMNTKYDENDKLIREVKVITLKSKLV SDFRKDFQFYKVREINNYHHAHDAYLNAVVGTALIKKYPKLESE FVYGDYKVYDVRKMIAKSEQEIGKATAKYFFYSNIMNFFKTEITL ANGEIRKRPLIETNGETGEIVWDKGRDFATVRKVLSMPQVNIVK KTEVQTGGFSKESILPKRNSDKLIARKKDWDPKKYGGFDSPTVA YSVLVVAKVEKGKSKKLKSVKELLGITIMERSSFEKNPIDFLEAK GYKEVKKDLIIKLPKYSLFELENGRKRMLASAGELQKGNELALP SKYVNFLYLASHYEKLKGSPEDNEQKQLFVEQHKHYLDEIIEQIS EFSKRVILADANLDKVLSAYNKHRDKPIREQAENIIHLFTLTNLG APAAFKYFDTTIDRKRYTSTKEVLDATLIHQSITGLYETRIDLSQL GGDAYPYDVPDYASLGSGSPKKKRKVEDPKKKRKVDGSGSETPGTS ESATPESGSGSNGSSGSMRDHMVLHEYVNAAGITAAARSGSGQRGSK RTADGSEFESPKKKRKV* |
| 113 | GFP1-10-HDAC 1 | MSKGEELFTGVVPILVELDGDVNGHKFSVRGEGEGDATIGKLTLKFICTT GKLPVPWPTLVTTLTYGVQCFSRYPDHMKRHDFFKSAMPEGYVQERTIS FKDDGKYKTRAVVKFEGDTLVNRIELKGTDFKEDGNILGHKLEYNFNSH NVYITADKQKNGIKANFTVRHNVEDGSVQLADHYQQNTPIGDGPVLLPD NHYLSTQTVLSKDPNEKLDPGGGGSG<MAQTQGTRRKVCYYYDGD VGNYYYGQGHPMKPHRIRMTHNLLLNYGLYRKMEIYRPHKAN AEEMTKYHSDDYIKFLRSIRPDNMSEYSKQMQRFNVGEDCPVFD GLFEFCQLSTGGSVASAVKLNKQQTDIAVNWAGGLHHAKKSEA SGFCYVNDIVLAILELLKYHQRVLYIDIDIHHGDGVEEAFYTTDR VMTVSFHKYGEYFPGTGDLRDIGAGKGKYYAVNYPLRDGIDDES YEAIFKPVMSKVMEMFQPSAVVLQCGSDSLSGDRLGCFNLTIKG HAKCVEFVKSFNLPMLMLGGGGYTIRNVARCWTYETAVALDTE IPNELPYNDYFEYFGPDFKLHISPSNMTNQNTNEYLEKIKQRLFE NLRMLPHAPGVQMQAIPEDAIPEESGDEDEDDPDKRISICSSDKRI ACEEEFSDSEEEGEGGRKNSSNFKKAKRVKTEDEKEKDPEEKKE VTEEEKTKEEKPEAKGVKEEVKLA>* |
| 114 | DNMT 3A-hDNM T3L-dCas12 a-1xGFP 11 | MPAAKRVKLDGSNHDQEFDPPKVYPPVPAEKRKPIRVLSLFDGIATG LLVLKDLGIQVDRYIASEVCEDSITVGMVRHQGKIMYVGDVRSVTQK HIQEWGPFDLVIGGSPCNDLSIVNPARKGLYEGTGRLFFEFYRLLHD ARPKEGDDRPFFWLFENVVAMGVSDKRDISRFLESNPVMIDAKEVS AAHRARYFWGNLPGMNRPLASTVNDKLELQECLEHGRIAKFSKVRT ITTRSNSIKQGKDQHFPVFMNEKEDILWCTEMERVFGFPVHYTDVS NMSRLARQRLLGRSWSVPVIRHLFAPLKEYFACVSSGNSNANSRGPS FSSGLVPLSLRGSHMFETVPVWRRQPVRVLSLFEDIKKELTSLGFLES GSDPGQLKHVVDVTDTVRKDVEEWGPFDLVYGATPPLGHTCDRPPS WYLFQFHRLLQYARPKPGSPRPFFWMFVDNLVLNKEDLDVASRFLE MEPVTIPDVHGGSLQNAVRVWSNIPAIRSSRHWALVSEEELSLLAQN KQSSKLAAKWPTKLVKNCFLPLREYFKYFSGGPSSGAPPPSGGSPAGS PTSTEEGTSESATPESGPGTSTEPSEGSAPGSPAGSPTSTEEGTSTEPSE GSAPGTSTEPSEPKKKRKVMSKLEKFTNCYSLSKTLRFKAIPVGKT QENIDNKRLLVEDEKRAEDYKGVKKLLDRYYLSFINDVLHSIKL KNLNNYISLFRKKTRTEKENKELENLEINLRKEIAKAFKGNEGYK SLFKKDIIETILPEFLDDKDEIALVNSFNGFTTAFTGFFDNRENMFS EEAKSTSIAFRCINENLTRYISNMDIFEKVDAIFDKHEVQEIKEKIL NSDYDVEDFFEGEFFNFVLTQEGIDVYNAIIGGFVTESGEKIKGLN EYINLYNQKTKQKLPKFKPLYKQVLSDRESLSFYGEGYTSDEEVL EVFRNTLNKNSEIFSSIKKLEKLFKNFDEYSSAGIFVKNGPAISTIS KDIFGEWNVIRDKWNAEYDDIHLKKKAVVTEKYEDDRRKSFKKI GSFSLEQLQEYADADLSVVEKLKEIIIQKVDEIYKVYGSSEKLFDA DFVLEKSLKKNDAVVAIMKDLLDSVKSFENYIKAFFGEGKETNR DESFYGDFVLAYDILLKVDHIYDAIRNYVTQKPYSKDKFKLYFQN PQFMGGWDKDKETDYRATILRYGSKYYLAIMDKKYAKCLQKID KDDVNGNYEKINYKLLPGPNKMLPKVFFSKKWMAYYNPSEDIQ KIYKNGTFKKGDMFNLNDCHKLIDFFKDSISRYPKWSNAYDFNFS ETEKYKDIAGFYREVEEQGYKVSFESASKKEVDKLVEEGKLYMF QIYNKDFSDKSHGTPNLHTMYFKLLFDENNHGQIRLSGGAELFM RRASLKKEELVVHPANSPIANKNPDNPKKTTTLSYDVYKDKRFSE DQYELHIPIAINKCPKNIFKINTEVRVLLKHDDNPYVIGIARGERN LLYIVVVDGKGNIVEQYSLNEIINNFNGIRIKTDYHSLLDKKEKER FEARQNWTSIENIKELKAGYISQVVHKICELVEKYDAVIALADLN SGFKNSRVKVEKQVYQKFEKMLIDKLNYMVDKKSNPCATGGAL KGYQITNKFESFKSMSTQNGFIFYIPAWLTSKIDPSTGFVNLLKTK YTSIADSKKFISSFDRIMYVPEEDLFEFALDYKNFSRTDADYIKKW KLYSYGNRIRIFRNPKKNNVFDWEEVCLTSAYKELFNKYGINYQ QGDIRALLCEQSDKAFYSSFMALMSLMLQMRNSITGRTDVAFLIS PVKNSDGIFYDSRNYEAQENAILPKNADANGAYNIARKVLWAIG QFKKAEDEKLDKVKIAISNKEWLEYAQTSVKHAYPYDVPDYASL GSGSPKKKRKVEDPKKKRKVDGSGSETPGTSESATPESGSGSNGSSG SMRDHMVLHEYVNAAGITAAARSGSGQRGSKRTADGSEFESPKKKRK V* |
| 115 | DNMT 3A-hDNM T3L-dCas12 i-1xGFP 11 | MPAAKRVKLDGSNHDQEFDPPKVYPPVPAEKRKPIRVLSLFDGIATG LLVLKDLGIQVDRYIASEVCEDSITVGMVRHQGKIMYVGDVRSVTQK HIQEWGPFDLVIGGSPCNDLSIVNPARKGLYEGTGRLFFEFYRLLHD ARPKEGDDRPFFWLFENVVAMGVSDKRDISRFLESNPVMIDAKEVS AAHRARYFWGNLPGMNRPLASTVNDKLELQECLEHGRIAKFSKVRT ITTRSNSIKQGKDQHFPVFMNEKEDILWCTEMERVFGFPVHYTDVS NMSRLARQRLLGRSWSVPVIRHLFAPLKEYFACVSSGNSNANSRGPS FSSGLVPLSLRGSHMFETVPVWRRQPVRVLSLFEDIKKELTSLGFLES GSDPGQLKHVVDVTDTVRKDVEEWGPFDLVYGATPPLGHTCDRPPS WYLFQFHRLLQYARPKPGSPRPFFWMFVDNLVLNKEDLDVASRFLE MEPVTIPDVHGGSLQNAVRVWSNIPAIRSSRHWALVSEEELSLLAQN KQSSKLAAKWPTKLVKNCFLPLREYFKYFSGGPSSGAPPPSGGSPAGS PTSTEEGTSESATPESGPGTSTEPSEGSAPGSPAGSPTSTEEGTSTEPSE GSAPGTSTEPSEPKKKRKVMGITMSDTTFRPYKSKLNPNERKMPM VVDTMNWCDNACSVFFDLFVGLYGGVKIGDVTNFLTEKQDTTT DADLLCAINWFRAMPVSNPDAVVGPEQLVVRFRDYSGHNPSPMA SMYLSAPVNPRYTWVDCRFLYQQMCQTLGANLDVDISTLVQTG LLPIQKDTQLNAYSFCSNVFGEGVKEDPSAKVTSYTAAANALSGA TNPIITYEDYRQILSSAITNRPSRKGAKDILAADLDSTKTGVLPGN YVSQRIAMYQASAASKARGVVLPNRLRVKAYLQNLVGLNYICTT WAAPMLRAVGDIKSKNSNNYRFVGERLERQQEIDNLLAVSNIRS AQAILQQFRANESNQFYIKKRHIGKGLPSLYDMWDLMDEKEGID NYIQSLKGDKTERLPIRELLQIIYPHRKSVSAELFLSAAELTDVED RQNRCMVHPTVRGSLVINFGKSSTIFGAITPPDTLIKFRGEMVPA GQTGMIWFTMHLVDGGKWVKHHIPCHSSRYFEEIYAWKKDLPT LPFPRRSLYGYAIGNKISDTRKIDLRRKKASKQYLRTLENMTHNV EFDPTTQFMVDQELNVTITARVKKIPPVRDVPVGSRIMGLNKNQ TIRDCYSIWERVEANTSGSFQHPVVQDVWLRLVADGHVTSTVNG PNGQIDQMSYSGPDVANCGNWMQERSNFITSVDDADQSIMKHFL WNNRVKLYAWNCVYAGTLMKAISKQMTTANRNLFRNEILSFVG GRFGCRLGSLAQVSLDLLRRMKSVISCYFSRNNAFTIEERSAFDK ELMELFESLDNKMNNKRKEKVNRTLSSIFQVAKQHNVVRLVVT KNLQTPDANVRSVMNQRSVDWCARKIEEKIVDLGNVIGIYVDLV EALDLSHLDPFIYIAKGDVGKECQFDEVESSSLLPIHTSMFRDWH GLVNKGRTTTPIYHSALRSFASHYGLRFDDLARIKAAELAKMLP DQKVFIPHRGGRFFMSTYPVTSTSRPVSYDGRQRFLNSGDMVAP VNTMLRGFRRARSAYPYDVPDYASLGSGSPKKKRKVEDPKKKRKV DGSGSETPGTSESATPESGSGSNGSSGSMRDHMVLHEYVNAAGITAAA RSGSGQRGSKRTADGSEFESPKKKRKV* |
| 116 | DNMT 3A-DNMT 3L-GFP1-10 | MPAAKRVKLDGSNHDQEFDPPKVYPPVPAEKRKPIRVLSLFDGIATG LLVLKDLGIQVDRYIASEVCEDSITVGMVRHQGKIMYVGDVRSVTQK HIQEWGPFDLVIGGSPCNDLSIVNPARKGLYEGTGRLFFEFYRLLHD ARPKEGDDRPFFWLFENVVAMGVSDKRDISRFLESNPVMIDAKEVS AAHRARYFWGNLPGMNRPLASTVNDKLELQECLEHGRIAKFSKVRT ITTRSNSIKQGKDQHFPVFMNEKEDILWCTEMERVFGFPVHYTDVS NMSRLARQRLLGRSWSVPVIRHLFAPLKEYFACVSSGNSNANSRGPS FSSGLVPLSLRGSHMGPMEIYKTVSAWKRQPVRVLSLFRNIDKVLKSL GFLESGSGSGGGTLKYVEDVTNVVRRDVEKWGPFDLVYGSTQPLGS SCDRCPGWYMFQFHRILQYALPRQESQRPFFWIFMDNLLLTEDDQE TTTRFLQTEAVTLQDVRGRDYQNAMRVWSNIPGLKSKHAPLTPKEE EYLQAQVRSRSKLDAPKVDLLVKNCLLPLREYFKYFSQNSLPLGGPSS GAPPPSGGSPAGSPTSTEEGTSESATMSKGEELFTGVVPILVELDGDVN GHKFSVRGEGEGDATIGKLTLKFICTTGKLPVPWPTLVTTLTYGVQCFSR YPDHMKRHDFFKSAMPEGYVQERTISFKDDGKYKTRAVVKFEGDTLVN RIELKGTDFKEDGNILGHKLEYNFNSHNVYITADKQKNGIKANFTVRHNV EDGSVQLADHYQQNTPIGDGPVLLPDNHYLSTQTVLSKDPNEK* |
| 117 | 1xGFP 11-dCas9-KRAB | MPAAKRVKLDGSGPDIVIGSGGRDHMVLHEYVNAAGITAAARSGSGQ RGSKRTADGSEFESPKKKRKVGGSGSGPKKKRKVMDKKYSIGLAIG TNSVGWAVITDEYKVPSKKFKVLGNTDRHSIKKNLIGALLFDSGE TAEATRLKRTARRRYTRRKNRICYLQEIFSNEMAKVDDSFFHRL EESFLVEEDKKHERHPIFGNIVDEVAYHEKYPTIYHLRKKLVDST DKADLRLIYLALAHMIKFRGHFLIEGDLNPDNSDVDKLFIQLVQT YNQLFEENPINASGVDAKAILSARLSKSRRLENLIAQLPGEKKNG LFGNLIALSLGLTPNFKSNFDLAEDAKLQLSKDTYDDDLDNLLAQ IGDQYADLFLAAKNLSDAILLSDILRVNTEITKAPLSASMIKRYDE HHQDLTLLKALVRQQLPEKYKEIFFDQSKNGYAGYIDGGASQEE FYKFIKPILEKMDGTEELLVKLNREDLLRKQRTFDNGSIPHQIHL GELHAILRRQEDFYPFLKDNREKIEKILTFRIPYYVGPLARGNSRF AWMTRKSEETITPWNFEEVVDKGASAQSFIERMTNFDKNLPNEK VLPKHSLLYEYFTVYNELTKVKYVTEGMRKPAFLSGEQKKAIVD LLFKTNRKVTVKQLKEDYFKKIECFDSVEISGVEDRFNASLGTYH DLLKIIKDKDFLDNEENEDILEDIVLTLTLFEDREMIEERLKTYAH LFDDKVMKQLKRRRYTGWGRLSRKLINGIRDKQSGKTILDFLKS DGFANRNFMQLIHDDSLTFKEDIQKAQVSGQGDSLHEHIANLAG SPAIKKGILQTVKVVDELVKVMGRHKPENIVIEMARENQTTQKG QKNSRERMKRIEEGIKELGSQILKEHPVENTQLQNEKLYLYYLQ NGRDMYVDQELDINRLSDYDVDAIVPQSFLKDDSIDNKVLTRSDK NRGKSDNVPSEEVVKKMKNYWRQLLNAKLITQRKFDNLTKAER GGLSELDKAGFIKRQLVETRQITKHVAQILDSRMNTKYDENDKL IREVKVITLKSKLVSDFRKDFQFYKVREINNYHHAHDAYLNAVV GTALIKKYPKLESEFVYGDYKVYDVRKMIAKSEQEIGKATAKYF FYSNIMNFFKTEITLANGEIRKRPLIETNGETGEIVWDKGRDFAT VRKVLSMPQVNIVKKTEVQTGGFSKESILPKRNSDKLIARKKDW DPKKYGGFDSPTVAYSVLVVAKVEKGKSKKLKSVKELLGITIME RSSFEKNPIDFLEAKGYKEVKKDLIIKLPKYSLFELENGRKRMLA SAGELQKGNELALPSKYVNFLYLASHYEKLKGSPEDNEQKQLFV EQHKHYLDEIIEQISEFSKRVILADANLDKVLSAYNKHRDKPIREQ AENIIHLFTLTNLGAPAAFKYFDTTIDRKRYTSTKEVLDATLIHQS ITGLYETRIDLSQLGGDAYPYDVPDYASLGSGSPKKKRKVEDPKKK RKVDGSGSETPGTSESATPES<RTLVTFKDVFVDFTREEWKLLDTA QQIVYRNVMLENYKNLVSLGYQLTKPDVILRLEKGEEP>* |
| 118 | 7xGFP 11-dCas9-ZIM3 | MPAAKRVKLDGSMRDHMVLHEYVNAAGITGGSGGRDHMVLHEYVNA AGITGGSGGRDHMVLHEYVNAAGITGGSGGRDHMVLHEYVNAAGITGG SGGRDHMVLHEYVNAAGITGGSGGRDHMVLHEYVNAAGITGGSGGRD HMVLHEYVNAAGITAAARSGSGQRGSKRTADGSEFESPKKKRKVGGS GSGPKKKRKVMDKKYSIGLAIGTNSVGWAVITDEYKVPSKKFKV LGNTDRHSIKKNLIGALLFDSGETAEATRLKRTARRRYTRRKNRI CYLQEIFSNEMAKVDDSFFHRLEESFLVEEDKKHERHPIFGNIVD EVAYHEKYPTIYHLRKKLVDSTDKADLRLIYLALAHMIKFRGHF LIEGDLNPDNSDVDKLFIQLVQTYNQLFEENPINASGVDAKAILSA RLSKSRRLENLIAQLPGEKKNGLFGNLIALSLGLTPNFKSNFDLA EDAKLQLSKDTYDDDLDNLLAQIGDQYADLFLAAKNLSDAILLSD ILRVNTEITKAPLSASMIKRYDEHHQDLTLLKALVRQQLPEKYKE IFFDQSKNGYAGYIDGGASQEEFYKFIKPILEKMDGTEELLVKLN REDLLRKQRTFDNGSIPHQIHLGELHAILRRQEDFYPFLKDNREK IEKILTFRIPYYVGPLARGNSRFAWMTRKSEETITPWNFEEVVDK GASAQSFIERMTNFDKNLPNEKVLPKHSLLYEYFTVYNELTKVK YVTEGMRKPAFLSGEQKKAIVDLLFKTNRKVTVKQLKEDYFKKI ECFDSVEISGVEDRFNASLGTYHDLLKIIKDKDFLDNEENEDILED IVLTLTLFEDREMIEERLKTYAHLFDDKVMKQLKRRRYTGWGR LSRKLINGIRDKQSGKTILDFLKSDGFANRNFMQLIHDDSLTFKE DIQKAQVSGQGDSLHEHIANLAGSPAIKKGILQTVKVVDELVKV MGRHKPENIVIEMARENQTTQKGQKNSRERMKRIEEGIKELGSQ ILKEHPVENTQLQNEKLYLYYLQNGRDMYVDQELDINRLSDYDV DAIVPQSFLKDDSIDNKVLTRSDKNRGKSDNVPSEEVVKKMKNY WRQLLNAKLITQRKFDNLTKAERGGLSELDKAGFIKRQLVETRQ ITKHVAQILDSRMNTKYDENDKLIREVKVITLKSKLVSDFRKDFQ FYKVREINNYHHAHDAYLNAVVGTALIKKYPKLESEFVYGDYKV YDVRKMIAKSEQEIGKATAKYFFYSNIMNFFKTEITLANGEIRKR PLIETNGETGEIVWDKGRDFATVRKVLSMPQVNIVKKTEVQTGG FSKESILPKRNSDKLIARKKDWDPKKYGGFDSPTVAYSVLVVAK VEKGKSKKLKSVKELLGITIMERSSFEKNPIDFLEAKGYKEVKKD LIIKLPKYSLFELENGRKRMLASAGELQKGNELALPSKYVNFLYL ASHYEKLKGSPEDNEQKQLFVEQHKHYLDEIIEQISEFSKRVILA DANLDKVLSAYNKHRDKPIREQAENIIHLFTLTNLGAPAAFKYFD TTIDRKRYTSTKEVLDATLIHQSITGLYETRIDLSQLGGDAYPYDV PDYASLGSGSPKKKRKVEDPKKKRKVDGSGSETPGTSESATPES<MN NSQGRVTFEDVTVNFTQGEWQRLNPEQRNLYRDVMLENYSNLV SVGQGETTKPDVILRLEQGKEPWLEEEEVLGSGRAEKNGDIGGQ IWKPKDVKESL>* |
| 119 | DNMT 3A-hDNM T3L-GFP1-10 | MPAAKRVKLDGSNHDQEFDPPKVYPPVPAEKRKPIRVLSLFDGIATG LLVLKDLGIQVDRYIASEVCEDSITVGMVRHQGKIMYVGDVRSVTQK HIQEWGPFDLVIGGSPCNDLSIVNPARKGLYEGTGRLFFEFYRLLHD ARPKEGDDRPFFWLFENVVAMGVSDKRDISRFLESNPVMIDAKEVS AAHRARYFWGNLPGMNRPLASTVNDKLELQECLEHGRIAKFSKVRT ITTRSNSIKQGKDQHFPVFMNEKEDILWCTEMERVFGFPVHYTDVS NMSRLARQRLLGRSWSVPVIRHLFAPLKEYFACVSSGNSNANSRGPS FSSGLVPLSLRGSHMFETVPVWRRQPVRVLSLFEDIKKELTSLGFLES GSDPGQLKHVVDVTDTVRKDVEEWGPFDLVYGATPPLGHTCDRPPS WYLFQFHRLLQYARPKPGSPRPFFWMFVDNLVLNKEDLDVASRFLE MEPVTIPDVHGGSLQNAVRVWSNIPAIRSSRHWALVSEEELSLLAQN KQSSKLAAKWPTKLVKNCFLPLREYFKYFSGGPSSGAPPPSGGSPAGS PTSTEEGTSESATPESGPGTSTEPSEGSAPGSPAGSPTSTEEGTSTEPSE GSAPGTSTEPSEPKKKRKVPSEMSKGEELFTGVVPILVELDGDVNGHK FSVRGEGEGDATIGKLTLKFICTTGKLPVPWPTLVTTLTYGVQCFSRYPD HMKRHDFFKSAMPEGYVQERTISFKDDGKYKTRAVVKFEGDTLVNRIEL KGTDFKEDGNILGHKLEYNFNSHNVYITADKQKNGIKANFTVRHNVEDG SVQLADHYQQNTPIGDGPVLLPDNHYLSTQTVLSKDPNEK* |
| 120 | DNMT 3A-DNMT 3L-1xGFP 11 | MPAAKRVKLDGSNHDQEFDPPKVYPPVPAEKRKPIRVLSLFDGIATG LLVLKDLGIQVDRYIASEVCEDSITVGMVRHQGKIMYVGDVRSVTQK HIQEWGPFDLVIGGSPCNDLSIVNPARKGLYEGTGRLFFEFYRLLHD ARPKEGDDRPFFWLFENVVAMGVSDKRDISRFLESNPVMIDAKEVS AAHRARYFWGNLPGMNRPLASTVNDKLELQECLEHGRIAKFSKVRT ITTRSNSIKQGKDQHFPVFMNEKEDILWCTEMERVFGFPVHYTDVS NMSRLARQRLLGRSWSVPVIRHLFAPLKEYFACVSSGNSNANSRGPS FSSGLVPLSLRGSHMGPMEIYKTVSAWKRQPVRVLSLFRNIDKVLKSL GFLESGSGSGGGTLKYVEDVTNVVRRDVEKWGPFDLVYGSTQPLGS SCDRCPGWYMFQFHRILQYALPRQESQRPFFWIFMDNLLLTEDDQE TTTRFLQTEAVTLQDVRGRDYQNAMRVWSNIPGLKSKHAPLTPKEE EYLQAQVRSRSKLDAPKVDLLVKNCLLPLREYFKYFSQNSLPLGGPSS GAPPPSGGSPAGSPTSTEEGTSESATGGSGGRDHMVLHEYVNAAGITA AARSGSGQRGSKRTADGSEFESPKKKRKV* |
| 121 | GFP1-10-dCas9-KRAB | MPAAKRVKLDGSGPDIVMSKGEELFTGVVPILVELDGDVNGHKFSVRG EGEGDATIGKLTLKFICTTGKLPVPWPTLVTTLTYGVQCFSRYPDHMKRH DFFKSAMPEGYVQERTISFKDDGKYKTRAVVKFEGDTLVNRIELKGTDF KEDGNILGHKLEYNFNSHNVYITADKQKNGIKANFTVRHNVEDGSVQLA DHYQQNTPIGDGPVLLPDNHYLSTQTVLSKDPNEKLDPGGGGSGSGPK KKRKVMDKKYSIGLAIGTNSVGWAVITDEYKVPSKKFKVLGNTD RHSIKKNLIGALLFDSGETAEATRLKRTARRRYTRRKNRICYLQE IFSNEMAKVDDSFFHRLEESFLVEEDKKHERHPIFGNIVDEVAYH EKYPTIYHLRKKLVDSTDKADLRLIYLALAHMIKFRGHFLIEGDL NPDNSDVDKLFIQLVQTYNQLFEENPINASGVDAKAILSARLSKSR RLENLIAQLPGEKKNGLFGNLIALSLGLTPNFKSNFDLAEDAKLQ LSKDTYDDDLDNLLAQIGDQYADLFLAAKNLSDAILLSDILRVNT EITKAPLSASMIKRYDEHHQDLTLLKALVRQQLPEKYKEIFFDQS KNGYAGYIDGGASQEEFYKFIKPILEKMDGTEELLVKLNREDLL RKQRTFDNGSIPHQIHLGELHAILRRQEDFYPFLKDNREKIEKILT FRIPYYVGPLARGNSRFAWMTRKSEETITPWNFEEVVDKGASAQ SFIERMTNFDKNLPNEKVLPKHSLLYEYFTVYNELTKVKYVTEG MRKPAFLSGEQKKAIVDLLFKTNRKVTVKQLKEDYFKKIECFDS VEISGVEDRFNASLGTYHDLLKIIKDKDFLDNEENEDILEDIVLTL TLFEDREMIEERLKTYAHLFDDKVMKQLKRRRYTGWGRLSRKL INGIRDKQSGKTILDFLKSDGFANRNFMQLIHDDSLTFKEDIQKA QVSGQGDSLHEHIANLAGSPAIKKGILQTVKVVDELVKVMGRHK PENIVIEMARENQTTQKGQKNSRERMKRIEEGIKELGSQILKEHP VENTQLQNEKLYLYYLQNGRDMYVDQELDINRLSDYDVDAIVPQ SFLKDDSIDNKVLTRSDKNRGKSDNVPSEEVVKKMKNYWRQLL NAKLITQRKFDNLTKAERGGLSELDKAGFIKRQLVETRQITKHV AQILDSRMNTKYDENDKLIREVKVITLKSKLVSDFRKDFQFYKVR EINNYHHAHDAYLNAVVGTALIKKYPKLESEFVYGDYKVYDVRK MIAKSEQEIGKATAKYFFYSNIMNFFKTEITLANGEIRKRPLIETN GETGEIVWDKGRDFATVRKVLSMPQVNIVKKTEVQTGGFSKESI LPKRNSDKLIARKKDWDPKKYGGFDSPTVAYSVLVVAKVEKGK SKKLKSVKELLGITIMERSSFEKNPIDFLEAKGYKEVKKDLIIKLP KYSLFELENGRKRMLASAGELQKGNELALPSKYVNFLYLASHYE KLKGSPEDNEQKQLFVEQHKHYLDEIIEQISEFSKRVILADANLD KVLSAYNKHRDKPIREQAENIIHLFTLTNLGAPAAFKYFDTTIDR KRYTSTKEVLDATLIHQSITGLYETRIDLSQLGGDAYPYDVPDYA SLGSGSPKKKRKVEDPKKKRKVDGSGSETPGTSESATPES<RTLVTF KDVFVDFTREEWKLLDTAQQIVYRNVMLENYKNLVSLGYQLTK PDVILRLEKGEEP>* |
| 122 | 10xGF P11-dCas9-ZIM3 | MPAAKRVKLDGSMRDHMVLHEYVNAAGITGGSGGRDHMVLHEYVNA AGITGGSGGRDHMVLHEYVNAAGITGGSGGRDHMVLHEYVNAAGITGG SGGRDHMVLHEYVNAAGITGGSGGRDHMVLHEYVNAAGITGGSGGRD HMVLHEYVNAAGITAAAGGSGGRDHMVLHEYVNAAGITGGSGGRDHM VLHEYVNAAGITGGSGGRDHMVLHEYVNAAGITGGSGGRSGSGQRGSK RTADGSEFESPKKKRKVGGSGSGPKKKRKVMDKKYSIGLAIGTNSV GWAVITDEYKVPSKKFKVLGNTDRHSIKKNLIGALLFDSGETAE ATRLKRTARRRYTRRKNRICYLQEIFSNEMAKVDDSFFHRLEESF LVEEDKKHERHPIFGNIVDEVAYHEKYPTIYHLRKKLVDSTDKA DLRLIYLALAHMIKFRGHFLIEGDLNPDNSDVDKLFIQLVQTYNQ LFEENPINASGVDAKAILSARLSKSRRLENLIAQLPGEKKNGLFG NLIALSLGLTPNFKSNFDLAEDAKLQLSKDTYDDDLDNLLAQIGD QYADLFLAAKNLSDAILLSDILRVNTEITKAPLSASMIKRYDEHH QDLTLLKALVRQQLPEKYKEIFFDQSKNGYAGYIDGGASQEEFY KFIKPILEKMDGTEELLVKLNREDLLRKQRTFDNGSIPHQIHLGE LHAILRRQEDFYPFLKDNREKIEKILTFRIPYYVGPLARGNSRFA WMTRKSEETITPWNFEEVVDKGASAQSFIERMTNFDKNLPNEKV LPKHSLLYEYFTVYNELTKVKYVTEGMRKPAFLSGEQKKAIVDL LFKTNRKVTVKQLKEDYFKKIECFDSVEISGVEDRFNASLGTYHD LLKIIKDKDFLDNEENEDILEDIVLTLTLFEDREMIEERLKTYAHL FDDKVMKQLKRRRYTGWGRLSRKLINGIRDKQSGKTILDFLKSD GFANRNFMQLIHDDSLTFKEDIQKAQVSGQGDSLHEHIANLAGSP AIKKGILQTVKVVDELVKVMGRHKPENIVIEMARENQTTQKGQ KNSRERMKRIEEGIKELGSQILKEHPVENTQLQNEKLYLYYLQN GRDMYVDQELDINRLSDYDVDAIVPQSFLKDDSIDNKVLTRSDKN RGKSDNVPSEEVVKKMKNYWRQLLNAKLITQRKFDNLTKAERG GLSELDKAGFIKRQLVETRQITKHVAQILDSRMNTKYDENDKLIR EVKVITLKSKLVSDFRKDFQFYKVREINNYHHAHDAYLNAVVGT ALIKKYPKLESEFVYGDYKVYDVRKMIAKSEQEIGKATAKYFFY SNIMNFFKTEITLANGEIRKRPLIETNGETGEIVWDKGRDFATVR KVLSMPQVNIVKKTEVQTGGFSKESILPKRNSDKLIARKKDWDP KKYGGFDSPTVAYSVLVVAKVEKGKSKKLKSVKELLGITIMERS SFEKNPIDFLEAKGYKEVKKDLIIKLPKYSLFELENGRKRMLASA GELQKGNELALPSKYVNFLYLASHYEKLKGSPEDNEQKQLFVEQ HKHYLDEIIEQISEFSKRVILADANLDKVLSAYNKHRDKPIREQAE NIIHLFTLTNLGAPAAFKYFDTTIDRKRYTSTKEVLDATLIHQSIT GLYETRIDLSQLGGDAYPYDVPDYASLGSGSPKKKRKVEDPKKKR KVDGSGSETPGTSESATPES<MNNSQGRVTFEDVTVNFTQGEWQR LNPEQRNLYRDVMLENYSNLVSVGQGETTKPDVILRLEQGKEP WLEEEEVLGSGRAEKNGDIGGQIWKPKDVKESL>* |
| 123 | scFv-ZIM3 | MVPAAKRVKLDGSGPDIVMTQSPSSLSASVGDRVTITCRSSTGAVTTSNY ASWVQEKPGKLFKGLIGGTNNRAPGVPSRFSGSLIGDKATLTISSLQPED FATYFCALWYSNHWVFGQGTKVELKRGGGGSGGGGSGGGGSSGGGSE VKLLESGGGLVQPGGSLKLSCAVSGFSLTDYGVNWVRQAPGRGLEWIGV IWGDGITDYNSALKDRFIISKDNGKNTVYLQMSKVRSDDTALYYCVTGLF DYWGQGTLVTVSSYPYDVPDYAGGGGGSGGGGSGGGGSGGGGSLDP GGGGSG<MNNSQGRVTFEDVTVNFTQGEWQRLNPEQRNLYRDV MLENYSNLVSVGQGETTKPDVILRLEQGKEPWLEEEEVLGSGRA EKNGDIGGQIWKPKDVKESL>* |
| 124 | DNMT 3A-hDNM T3L-dCas9-1xGCN 4 | MPAAKRVKLDGSNHDQEFDPPKVYPPVPAEKRKPIRVLSLFDGIATG LLVLKDLGIQVDRYIASEVCEDSITVGMVRHQGKIMYVGDVRSVTQK HIQEWGPFDLVIGGSPCNDLSIVNPARKGLYEGTGRLFFEFYRLLHD ARPKEGDDRPFFWLFENVVAMGVSDKRDISRFLESNPVMIDAKEVS AAHRARYFWGNLPGMNRPLASTVNDKLELQECLEHGRIAKFSKVRT ITTRSNSIKQGKDQHFPVFMNEKEDILWCTEMERVFGFPVHYTDVS NMSRLARQRLLGRSWSVPVIRHLFAPLKEYFACVSSGNSNANSRGPS FSSGLVPLSLRGSHMFETVPVWRRQPVRVLSLFEDIKKELTSLGFLES GSDPGQLKHVVDVTDTVRKDVEEWGPFDLVYGATPPLGHTCDRPPS WYLFQFHRLLQYARPKPGSPRPFFWMFVDNLVLNKEDLDVASRFLE MEPVTIPDVHGGSLQNAVRVWSNIPAIRSSRHWALVSEEELSLLAQN KQSSKLAAKWPTKLVKNCFLPLREYFKYFSGGPSSGAPPPSGGSPAGS PTSTEEGTSESATPESGPGTSTEPSEGSAPGSPAGSPTSTEEGTSTEPSE GSAPGTSTEPSEPKKKRKVGSDKKYSIGLAIGTNSVGWAVITDEYK VPSKKFKVLGNTDRHSIKKNLIGALLFDSGETAEATRLKRTARRR YTRRKNRICYLQEIFSNEMAKVDDSFFHRLEESFLVEEDKKHERH PIFGNIVDEVAYHEKYPTIYHLRKKLVDSTDKADLRLIYLALAHM IKFRGHFLIEGDLNPDNSDVDKLFIQLVQTYNQLFEENPINASGVD AKAILSARLSKSRRLENLIAQLPGEKKNGLFGNLIALSLGLTPNFK SNFDLAEDAKLQLSKDTYDDDLDNLLAQIGDQYADLFLAAKNLS DAILLSDILRVNTEITKAPLSASMIKRYDEHHQDLTLLKALVRQQ LPEKYKEIFFDQSKNGYAGYIDGGASQEEFYKFIKPILEKMDGTE ELLVKLNREDLLRKQRTFDNGSIPHQIHLGELHAILRRQEDFYPF LKDNREKIEKILTFRIPYYVGPLARGNSRFAWMTRKSEETITPWN FEEVVDKGASAQSFIERMTNFDKNLPNEKVLPKHSLLYEYFTVY NELTKVKYVTEGMRKPAFLSGEQKKAIVDLLFKTNRKVTVKQL KEDYFKKIECFDSVEISGVEDRFNASLGTYHDLLKIIKDKDFLDNE ENEDILEDIVLTLTLFEDREMIEERLKTYAHLFDDKVMKQLKRR RYTGWGRLSRKLINGIRDKQSGKTILDFLKSDGFANRNFMQLIH DDSLTFKEDIQKAQVSGQGDSLHEHIANLAGSPAIKKGILQTVKV VDELVKVMGRHKPENIVIEMARENQTTQKGQKNSRERMKRIEE GIKELGSQILKEHPVENTQLQNEKLYLYYLQNGRDMYVDQELDI NRLSDYDVDAIVPQSFLKDDSIDNKVLTRSDKNRGKSDNVPSEEV VKKMKNYWRQLLNAKLITQRKFDNLTKAERGGLSELDKAGFIK RQLVETRQITKHVAQILDSRMNTKYDENDKLIREVKVITLKSKLV SDFRKDFQFYKVREINNYHHAHDAYLNAVVGTALIKKYPKLESE FVYGDYKVYDVRKMIAKSEQEIGKATAKYFFYSNIMNFFKTEITL ANGEIRKRPLIETNGETGEIVWDKGRDFATVRKVLSMPQVNIVK KTEVQTGGFSKESILPKRNSDKLIARKKDWDPKKYGGFDSPTVA YSVLVVAKVEKGKSKKLKSVKELLGITIMERSSFEKNPIDFLEAK GYKEVKKDLIIKLPKYSLFELENGRKRMLASAGELQKGNELALP SKYVNFLYLASHYEKLKGSPEDNEQKQLFVEQHKHYLDEIIEQIS EFSKRVILADANLDKVLSAYNKHRDKPIREQAENIIHLFTLTNLG APAAFKYFDTTIDRKRYTSTKEVLDATLIHQSITGLYETRIDLSQL GGDAYPYDVPDYASLGSGSPKKKRKVEDPKKKRKVDGIGSGSNGSS GEELLSKNYHLENEVARLKKGSGSGGSGSGGSGSGSGGSGSGGSGSGE ELLSKNYHGSGSGQRGSKRTADGSEFESPKKKRKV* |
| 361 | DNMT 3A-hDNM T3L-XTEN8 0-TALE HPT8-1xGCN 4 | MPAAKRVKLDGSNHDQEFDPPKVYPPVPAEKRKPIRVLSLFDGIATG LLVLKDLGIQVDRYIASEVCEDSITVGMVRHQGKIMYVGDVRSVTQK HIQEWGPFDLVIGGSPCNDLSIVNPARKGLYEGTGRLFFEFYRLLHD ARPKEGDDRPFFWLFENVVAMGVSDKRDISRFLESNPVMIDAKEVS AAHRARYFWGNLPGMNRPLASTVNDKLELQECLEHGRIAKFSKVRT ITTRSNSIKQGKDQHFPVFMNEKEDILWCTEMERVFGFPVHYTDVS NMSRLARQRLLGRSWSVPVIRHLFAPLKEYFACVSSGNSNANSRGPS FSSGLVPLSLRGSHMFETVPVWRRQPVRVLSLFEDIKKELTSLGFLES GSDPGQLKHVVDVTDTVRKDVEEWGPFDLVYGATPPLGHTCDRPPS WYLFQFHRLLQYARPKPGSPRPFFWMFVDNLVLNKEDLDVASRFLE MEPVTIPDVHGGSLQNAVRVWSNIPAIRSSRHWALVSEEELSLLAQN KQSSKLAAKWPTKLVKNCFLPLREYFKYFSGGPSSGAPPPSGGSPAGS PTSTEEGTSESATPESGPGTSTEPSEGSAPGSPAGSPTSTEEGTSTEPSE GSAPGTSTEPSEPKKKRKVMVDLRTLGYSQQQQEKIKPKVRSTVA QHHEALVGHGFTHAHIVALSQHPAALGTVAVKYQDMIAALPEA THEAIVGVGKQWSGARALEALLTVAGELRGPPLQLDTGQLLKIA KRGGVTAVEAVHAWRNALTGAPLNLTPDQVVAIASNGGGKQAL ETVQRLLPVLCQDHGLTPDQVVAIASHDGGKQALETVQRLLPVL CQDHGLTPDQVVAIASNIGGKQALETVQRLLPVLCQDHGLTPDQ VVAIASNNGGKQALETVQRLLPVLCQDHGLTPDQVVAIASNGGG KQALETVQRLLPVLCQDHGLTPDQVVAIASNGGGKQALETVQR LLPVLCQDHGLTPDQVVAIASHDGGKQALETVQRLLPVLCQDH GLTPDQVVAIASNIGGKQALETVQRLLPVLCQDHGLTPDQVVAI ASNNGGKQALETVQRLLPVLCQDHGLTPDQVVAIASNNGGKQA LETVQRLLPVLCQDHGLTPDQVVAIASNNGGKQALETVQRLLPV LCQDHGLTPDQVVAIASNGGGKQALETVQRLLPVLCQDHGLTP DQVVAIASHDGGKQALETVQRLLPVLCQDHGLTPDQVVAIASNG GGKQALETVQRLLPVLCQDHGLTPDQVVAIASNNGGKQALETV QRLLPVLCQDHGLTPDQVVAIASNIGGKQALETVQRLLPVLCQD HGLTPDQVVAIASNNGGKQALETVQRLLPVLCQDHGLTPDQVV AIASHDGGKQALESIVAQLSRPDPALAALTNDHLVALACLGGRP AMDAVKKGLPHAPELIRRVNRRIGERTSHRVAYPYDVPDYASLGS GSPKKKRKVEDPKKKRKVDGIGSGSNGSSGEELLSKNYHLENEVARLK KGSGSGGSGSGGSGSGSGGSGSGGSGSGEELLSKNYHGSGSGQRGSK RTADGSEFESPKKKRKV* |
| 362 | DNMT 3A-hDNM T3L-XTEN8 0-TALE HPT12-1xGCN 4 | MPAAKRVKLDGSNHDQEFDPPKVYPPVPAEKRKPIRVLSLFDGIATG LLVLKDLGIQVDRYIASEVCEDSITVGMVRHQGKIMYVGDVRSVTQK HIQEWGPFDLVIGGSPCNDLSIVNPARKGLYEGTGRLFFEFYRLLHD ARPKEGDDRPFFWLFENVVAMGVSDKRDISRFLESNPVMIDAKEVS AAHRARYFWGNLPGMNRPLASTVNDKLELQECLEHGRIAKFSKVRT ITTRSNSIKQGKDQHFPVFMNEKEDILWCTEMERVFGFPVHYTDVS NMSRLARQRLLGRSWSVPVIRHLFAPLKEYFACVSSGNSNANSRGPS FSSGLVPLSLRGSHMFETVPVWRRQPVRVLSLFEDIKKELTSLGFLES GSDPGQLKHVVDVTDTVRKDVEEWGPFDLVYGATPPLGHTCDRPPS WYLFQFHRLLQYARPKPGSPRPFFWMFVDNLVLNKEDLDVASRFLE MEPVTIPDVHGGSLQNAVRVWSNIPAIRSSRHWALVSEEELSLLAQN KQSSKLAAKWPTKLVKNCFLPLREYFKYFSGGPSSGAPPPSGGSPAGS PTSTEEGTSESATPESGPGTSTEPSEGSAPGSPAGSPTSTEEGTSTEPSE GSAPGTSTEPSEPKKKRKVMVDLRTLGYSQQQQEKIKPKVRSTVA QHHEALVGHGFTHAHIVALSQHPAALGTVAVKYQDMIAALPEA THEAIVGVGKQWSGARALEALLTVAGELRGPPLQLDTGQLLKIA KRGGVTAVEAVHAWRNALTGAPLNLTPDQVVAIASNNGGKQAL ETVQRLLPVLCQDHGLTPDQVVAIASNNGGKQALETVQRLLPVL CQDHGLTPDQVVAIASNIGGKQALETVQRLLPVLCQDHGLTPDQ VVAIASNNGGKQALETVQRLLPVLCQDHGLTPDQVVAIASNNGG KQALETVQRLLPVLCQDHGLTPDQVVAIASNIGGKQALETVQRL LPVLCQDHGLTPDQVVAIASNNGGKQALETVQRLLPVLCQDHG LTPDQVVAIASNGGGKQALETVQRLLPVLCQDHGLTPDQVVAIA SNNGGKQALETVQRLLPVLCQDHGLTPDQVVAIASNIGGKQALE TVQRLLPVLCQDHGLTPDQVVAIASNNGGKQALETVQRLLPVLC QDHGLTPDQVVAIASHDGGKQALETVQRLLPVLCQDHGLTPDQ VVAIASHDGGKQALETVQRLLPVLCQDHGLTPDQVVAIASNIGG KQALETVQRLLPVLCQDHGLTPDQVVAIASNNGGKQALETVQR LLPVLCQDHGLTPDQVVAIASNNGGKQALETVQRLLPVLCQDH GLTPDQVVAIASHDGGKQALETVQRLLPVLCQDHGLTPDQVVAI ASNIGGKQALESIVAQLSRPDPALAALTNDHLVALACLGGRPAM DAVKKGLPHAPELIRRVNRRIGERTSHRVAYPYDVPDYASLGSGS PKKKRKVEDPKKKRKVDGIGSGSNGSSGEELLSKNYHLENEVARLKKG SGSGGSGSGGSGSGSGGSGSGGSGSGEELLSKNYHGSGSGQRGSKRT ADGSEFESPKKKRKV* |
| 363 | 3xGCN 4-TALE HPT8-ZIM3 | MPAAKRVKLDGSEELLSKNYHLENEVARLKKGSGSGGSGSGGSGSGS GGSGSGGSGSGEELLSKNYHLENEVARLKKGSGSGGSGSGGSGSGSGG SGSGGSGSGEELLSKNYHLENEVARLKKGSGSGGSGSGGSGSGSGGSG SGGSGSGPKKKRKVMVDLRTLGYSQQQQEKIKPKVRSTVAQHHE ALVGHGFTHAHIVALSQHPAALGTVAVKYQDMIAALPEATHEAI VGVGKQWSGARALEALLTVAGELRGPPLQLDTGQLLKIAKRGG VTAVEAVHAWRNALTGAPLNLTPDQVVAIASNGGGKQALETVQ RLLPVLCQDHGLTPDQVVAIASHDGGKQALETVQRLLPVLCQD HGLTPDQVVAIASNIGGKQALETVQRLLPVLCQDHGLTPDQVVA IASNNGGKQALETVQRLLPVLCQDHGLTPDQVVAIASNGGGKQA LETVQRLLPVLCQDHGLTPDQVVAIASNGGGKQALETVQRLLPV LCQDHGLTPDQVVAIASHDGGKQALETVQRLLPVLCQDHGLTP DQVVAIASNIGGKQALETVQRLLPVLCQDHGLTPDQVVAIASNN GGKQALETVQRLLPVLCQDHGLTPDQVVAIASNNGGKQALETV QRLLPVLCQDHGLTPDQVVAIASNNGGKQALETVQRLLPVLCQD HGLTPDQVVAIASNGGGKQALETVQRLLPVLCQDHGLTPDQVV AIASHDGGKQALETVQRLLPVLCQDHGLTPDQVVAIASNGGGKQ ALETVQRLLPVLCQDHGLTPDQVVAIASNNGGKQALETVQRLLP VLCQDHGLTPDQVVAIASNIGGKQALETVQRLLPVLCQDHGLTP DQVVAIASNNGGKQALETVQRLLPVLCQDHGLTPDQVVAIASHD GGKQALESIVAQLSRPDPALAALTNDHLVALACLGGRPAMDAV KKGLPHAPELIRRVNRRIGERTSHRVAYPYDVPDYASLGSGSPKK KRKVEDPKKKRKVDGSGSETPGTSESATPES<MNNSQGRVTFEDVT VNFTQGEWQRLNPEQRNLYRDVMLENYSNLVSVGQGETTKPDV ILRLEQGKEPWLEEEEVLGSGRAEKNGDIGGQIWKPKDVKESL> * |
| 364 | 3xGCN 4-TALE HPT12-ZIM3 | MPAAKRVKLDGSEELLSKNYHLENEVARLKKGSGSGGSGSGGSGSGS GGSGSGGSGSGEELLSKNYHLENEVARLKKGSGSGGSGSGGSGSGSGG SGSGGSGSGEELLSKNYHLENEVARLKKGSGSGGSGSGGSGSGSGGSG SGGSGSGPKKKRKVMVDLRTLGYSQQQQEKIKPKVRSTVAQHHE ALVGHGFTHAHIVALSQHPAALGTVAVKYQDMIAALPEATHEAI VGVGKQWSGARALEALLTVAGELRGPPLQLDTGQLLKIAKRGG VTAVEAVHAWRNALTGAPLNLTPDQVVAIASNNGGKQALETVQ RLLPVLCQDHGLTPDQVVAIASNNGGKQALETVQRLLPVLCQDH GLTPDQVVAIASNIGGKQALETVQRLLPVLCQDHGLTPDQVVAI ASNNGGKQALETVQRLLPVLCQDHGLTPDQVVAIASNNGGKQA LETVQRLLPVLCQDHGLTPDQVVAIASNIGGKQALETVQRLLPV LCQDHGLTPDQVVAIASNNGGKQALETVQRLLPVLCQDHGLTP DQVVAIASNGGGKQALETVQRLLPVLCQDHGLTPDQVVAIASNN GGKQALETVQRLLPVLCQDHGLTPDQVVAIASNIGGKQALETVQ RLLPVLCQDHGLTPDQVVAIASNNGGKQALETVQRLLPVLCQDH GLTPDQVVAIASHDGGKQALETVQRLLPVLCQDHGLTPDQVVAI ASHDGGKQALETVQRLLPVLCQDHGLTPDQVVAIASNIGGKQAL ETVQRLLPVLCQDHGLTPDQVVAIASNNGGKQALETVQRLLPVL CQDHGLTPDQVVAIASNNGGKQALETVQRLLPVLCQDHGLTPD QVVAIASHDGGKQALETVQRLLPVLCQDHGLTPDQVVAIASNIG GKQALESIVAQLSRPDPALAALTNDHLVALACLGGRPAMDAVK KGLPHAPELIRRVNRRIGERTSHRVAYPYDVPDYASLGSGSPKKK RKVEDPKKKRKVDGSGSETPGTSESATPES<MNNSQGRVTFEDVTV NFTQGEWQRLNPEQRNLYRDVMLENYSNLVSVGQGETTKPDVI LRLEQGKEPWLEEEEVLGSGRAEKNGDIGGQIWKPKDVKESL>* |

Without intending to be limited by any theory, the following examples are only for illustrating the complexes, preparation methods, uses, etc. of the present application, and are not intended to limit the scope of the invention of the present application.

### EXAMPLES

### Example 1

### Design and construction of plasmids comprising the complex of the present application

The amino acid sequences of the epigenetic modification recruitment system with HA epitope, P2A and the recruited elements (including Dnmt3a CD, Dnmt3l CD, dSpCas9 or TALE, KRAB) were optimized by Genscript company into nucleic acid sequences suitable for mammalian expression and synthesized, and then cloned into pLV-CAG vector with CAG promoter and WPRE, and the recruited elements and self-cleaved recruitment system fusion proteins were expressed by the CAG promoter.

When optimizing different functional elements, the different functional elements were optimized by Genscript company into nucleic acid sequences suitable for mammalian expression and synthesized. First, a vector other than the elements to be replaced was amplified by PCR, and then the elements to be replaced were amplified from the sequences synthesized by the company, and homologous arm sequences were introduced at the same time, and finally, different components were recombined into the vector using NEBuilder reagent to construct the final expression plasmid.

### Example 2

### Inhibitory effect of the complex of the present application on PTP1b gene expression

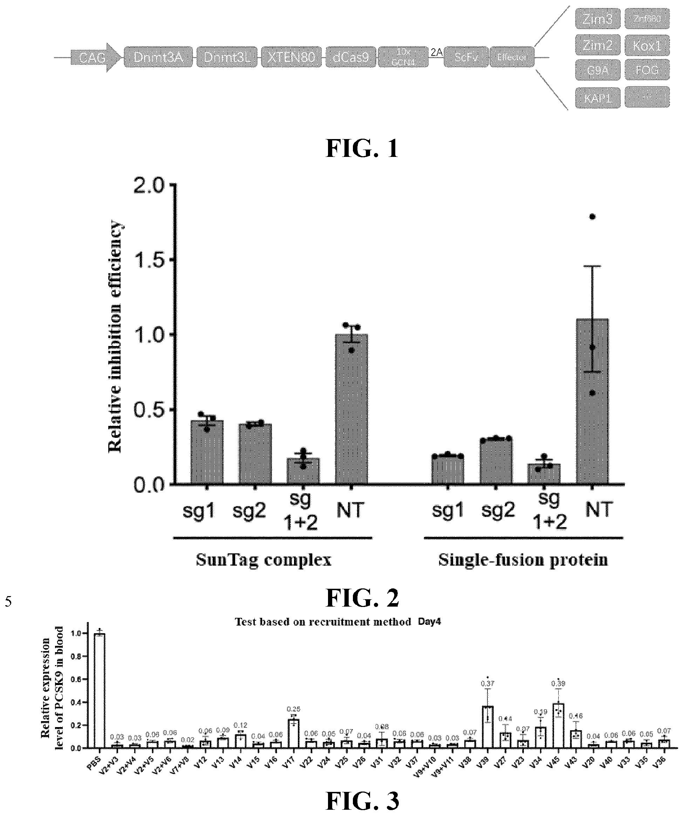

Editing tools comprising the complex of the present application (a complex formed by combining a first fusion having an amino acid sequence as set forth in SEQ ID NO: 51 with a second fusion having an amino acid sequence as set forth in SEQ ID NO: 52), and gRNAs differently targeted to mouse PTP1b gene (sg1, sg2, or an equal mixture of sg1 and sg2), complementary nucleotide sequences of the gRNA targeting sequences as set forth in SEQ ID NOs: 336 and 337, and control gRNA (NT gRNA, the complementary nucleotide sequence of its targeting sequence is as set forth in SEQ IF NO:365) were co-transfected into mouse N2a cell line (700 ng editor + 300 ng gRNA, 24-well plate), and the transfected positive cells were sorted after 72 hours of transfection, total RNA was extracted with Trizol, the relative expression of PTP1b was quantified by qPCR, and the relative inhibition efficiencies of the different tools and different gRNAs were calculated (Fig. 2). The results showed that compared with the control group (Fig. 2, right) embodiment (DNMT3A-DNMT3L-dCas9-KRAB) in which the nucleic acid binding domain dCas9, methylation factors DNMT3A and DNMT3L, and the transcriptional repressor KRAB were directly fused together, the complex of the present application achieved higher or substantial inhibition efficiency for gRNAs targeting PTP1b gene, that is, sg1 and/or sg2.

### Example 3

### Inhibitory effect of the complex of the present application on PCSK9 gene expression

Different versions of tools were in vitro transcribed into mRNA (see Table below for the information on the tools to be tested), and then mixed with chemically synthesized sgRNA at a mass ratio of 1:1 (the mass ratio of mRNA to sgRNA of the first fusion and the second fusion was 0.5:0.5:1; the complementary nucleotide sequence of the sgRNA targeting sequence was as set forth in SEQ ID NO: 341 and/or 342) to prepare LNP (LNP citation: Musunuru, K., Chadwick, A.C., Mizoguchi, T. et al. In vivo CRISPR base editing of PCSK9 durably lowers cholesterol in primates. Nature 593, 429-434 (2021).). The prepared LNP was injected into mice through tail vein at an injection dose of 4.5 mg per kg body weight, and 4-10 days after injection, blood was collected from the cheeks of the mice, and the content of PCSK9 protein in the blood was determined by Elisa. The PBS group was the control group injected with equal volume PBS. The test results of each tool on Day 4 after injection were shown in Fig. 3. Compared with the PBS control group, each group of tools in the present application showed an extremely prominent inhibitory effect on PCSK9 gene expression.

| **Tool number** | **Domain Information** | **Amino acid sequence (SEQ ID NO:)** | **mRNA sequence (SEQ ID NO:)** |
|---|---|---|---|
| V2+V3 | scFv-KRAB, DNMT3A-DNMT3L-dCas9-1xGCN4 | 78, 79 | 243, 244 |
| V2+V4 | scFv-KRAB, DNMT3A-DNMT3L-dCas9-2xGCN4 | 78, 80 | 243, 245 |
| V2+V5 | scFv-KRAB, DNMT3A-DNMT3L-dCas9-4xGCN4 | 78, 81 | 243, 246 |
| V2+V6 | scFv-KRAB, DNMT3A-DNMT3L-dCas9-10xGCN4 | 78, 82 | 243, 247 |
| V7+V8 | DNMT3A-DNMT3L-scFv, 3xGCN4-dCas9-KRAB | 83, 84 | 248, 249 |
| V12 | scFv-KRAB-2a-DNMT3A-DNMT3L-dCas9-1xGCN4 | 133 | 253 |
| V13 | scFv-KRAB-2a-DNMT3A-DNMT3L-dCas9-2xGCN4 | 134 | 254 |
| V14 | scFv-KRAB-2a-DNMT3A-DNMT3L-dCas9-10xGCN4 | 135 | 255 |
| V15 | scFv-ZIM3-2a-DNMT3A-hDNMT3L-dCas9-1xGCN4 | 136 | 256 |
| V16 | scFv-ZIM3-2a-DNMT3A-hDNMT3L-dCas9-2xGCN4 | 137 | 257 |
| V17 | scFv-ZIM3-2a-DNMT3A-hDNMT3L-dCas9-10xGCN4 | 138 | 258 |
| V22 | DNMT3A-DNMT3L-scFv-2a-1xGCN4-dCas9-KRAB | 143 | 263 |
| V24 | DNMT3A-DNMT3L-scFv-2a-3xGCN4-dCas9-KRAB | 145 | 265 |
| V25 | DNMT3A-DNMT3L-scFv-2a-5xGCN4-dCas9-KRAB | 146 | 266 |
| V26 | DNMT3A-hDNMT3L-scFv-2a-3xGCN4-dCas9-ZIM3 | 147 | 267 |
| V31 | scFV-hDNMT3L-DNMT3A-2a-3xGCN4-dCas9-KRAB | 152 | 272 |
| V32 | 1xGCN4-ZIM3-2a-DNMT3A-hDNMT3L-dCas9-scFv | 153 | 273 |
| V37 | GFP1-10-KRAB-2a-DNMT3A-DNMT3L-dCas9-7xGFP11 | 158 | 278 |
| V9+V10 | GFP1-10-KRAB, DNMT3A-DNMT3L-dCas9-1xGFP11 | 85, 86 | 250, 251 |
| V9+V 11 | GFP1-10-KRAB, DNMT3A-DNMT3L-dCas9-8xGFP11 | 85, 87 | 250, 252 |
| V38 | GFP1-10-KRAB-2a-DNMT3A-DNMT3L-dCas9-1xGFP11 | 159 | 279 |
| V39 | GFP1-10-ZIM3-2a-DNMT3A-hDNMT3L-dCas9-1xGFP11 | 160 | 280 |
| V27 | 3xGCN4-dCas9-ZIM3-2a-DNMT3A-hDNMT3L-scFv | 148 | 268 |
| V23 | DNMT3A-DNMT3L-scFv-2a-2xGCN4-dCas9-KRAB | 144 | 264 |
| V34 | DNMT3A-DNMT3L-1xGCN4-2a-scFv-dCas9-KRAB | 155 | 275 |
| V45 | DNMT3A-DNMT3L-1xGFP11-2a-GFP1-10-dCas9-KRAB | 166 | 286 |
| V43 | DNMT3A-DNMT3L-GFP1-10-2a-1xGFP11-dCas9-KRAB | 164 | 284 |
| V20 | KRAB-scFv-2a-DNMT3A-DNMT3L-dCas9-2xGCN4 | 141 | 261 |
| V40 | GFP1-10-HDAC1-2a-DNMT3A-hDNMT3L-dCas9-1xGFP11 | 161 | 281 |
| V33 | KRAB-1xGCN4-2a-DNMT3A-DNMT3L-dCas9-scFv | 154 | 274 |
| V35 | scFv-dCas9-KRAB-2a-1xGCN4-DNMT3L-DNMT3A | 156 | 276 |
| V36 | scFv-dCas9-HP1a-2a-1xGCN4-DNMT3L-DNMT3A | 157 | 277 |

### Example 4

### Inhibitory effect of the complex of the present application on PCSK9 gene expression

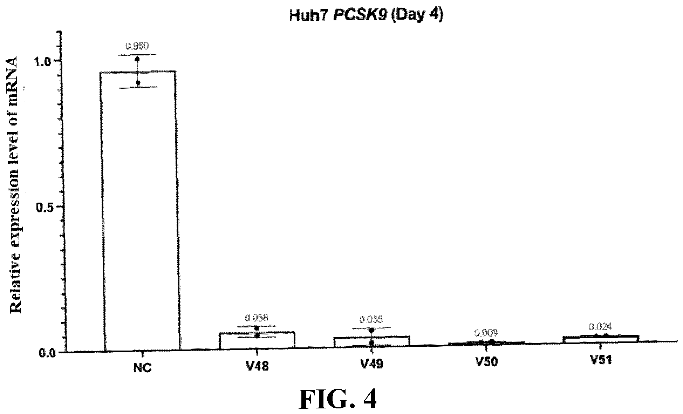

According to the in vitro transcribed mRNA in Example 3 (see Table below for information on the tools used), the chemically synthesized sgRNA/mRNA mixture with a mass ratio of 1:1 (sgRNA sequence was the same as in Example 3), and the method for preparing LNP, the prepared LNP was added to Huh7 cells (1.25 ug/mL dose). 4 days after the addition of LNP, all cells were collected, total RNA was extracted with Trizol, the relative expression of PCSK9 was quantified by qPCR, and the relative inhibition efficiencies of the different tools were calculated. The results were shown in Fig. 4, wherein the NC group was a control group without adding LNP. It can be seen that the fusion provided by the present application has a significant inhibitory effect on PCSK9 gene expression.

| **Tool number** | **Domain Information** | **Amino acid sequence (SEQ ID NO:)** | **mRNA sequence (SEQ ID NO:)** |
|---|---|---|---|
| V48 | scFv-ZIM3-P2A-DNMT3A-hDNMT3L-XTEN80-TALE HPT8-1x GCN4 | 345 | 353 |
| V49 | scFv-ZIM3-P2A-DNMT3A-hDNMT3L-XTEN80-TALE HPT12-1x GCN4 | 346 | 354 |
| V50 | 3xGCN4-TALE HPT8-ZIM3-P2A-DNMT3A-hDNMT3L-XTEN80-scFv | 347 | 355 |
| V51 | 3xGCN4-TALE HPT12-ZIM3-P2A-DNMT3A-hDNMT3L-XTEN80-scFv | 348 | 356 |

## Claims

1. A complex comprising a first fusion and a second fusion, wherein the fusion of one of the first fusion and the second fusion comprises a DNA methylation domain and at least one recruitment domain A, and wherein the other fusion comprises a transcriptional repressor domain and at least one recruitment domain A'; wherein the recruitment domain A and the recruitment domain A' are capable of interacting, such that the fusion of one of the first fusion and the second fusion or a portion thereof is capable of being recruited to the vicinity of the other fusion.

2. The complex according to claim 1, wherein the first fusion or the second fusion comprises a nucleic acid binding domain.

3. The complex according to claim 1 or 2, wherein the first fusion comprises a DNA methylation domain, a nucleic acid binding domain and at least one recruitment domain A, and the second fusion comprises a transcriptional repressor domain and at least one recruitment domain A'.

4. The complex according to any one of claims 1-3, wherein the first fusion comprises, in order from N-terminus to C-terminus, a DNA methylation domain, a nucleic acid binding domain, and a recruitment domain A.

5. The complex according to any one of claims 1-4, wherein the second fusion comprises, in order from N-terminus to C-terminus, a transcriptional repressor domain and a recruitment domain A', or comprises, in order from N-terminus to C-terminus, a recruitment domain A' and a transcriptional repressor domain.

6. The complex according to claim 1 or 2, wherein the first fusion comprises a transcriptional repressor domain, a nucleic acid binding domain and at least one recruitment domain A, and the second fusion comprises a DNA methylation domain and at least one recruitment domain A'.

7. The complex according to any one of claims 1, 2 and 6, wherein the first fusion comprises, in order from N-terminus to C-terminus, a recruitment domain A, a nucleic acid binding domain and a transcriptional repressor domain.

8. The complex according to any one of claims 1, 2, 6 and 7, wherein the second fusion comprises, in order from N-terminus to C-terminus, a DNA methylation domain and a recruitment domain A', or comprises, in order from N-terminus to C-terminus, a recruitment domain A' and a DNA methylation domain.

9. The complex according to any one of claims 1-8, **characterized in that**:
1) the first fusion comprises, in order from N-terminus to C-terminus, a DNA methylation domain, a nucleic acid binding domain, and a recruitment domain A, and the second fusion comprises, in order from N-terminus to C-terminus, a transcriptional repressor domain and a recruitment domain A'; or
2) the first fusion comprises, in order from N-terminus to C-terminus, a DNA methylation domain, a nucleic acid binding domain, and a recruitment domain A, and the second fusion comprises, in order from N-terminus to C-terminus, a recruitment domain A' and a transcriptional repressor domain; or
3) the first fusion comprises, in order from N-terminus to C-terminus, a recruitment domain A, a nucleic acid binding domain and a transcriptional repressor domain, and the second fusion comprises, in order from N-terminus to C-terminus, a DNA methylation domain and a recruitment domain A'; or
4) the first fusion comprises, in order from N-terminus to C-terminus, a recruitment domain A, a nucleic acid binding domain and a transcriptional repressor domain, and the second fusion comprises, in order from N-terminus to C-terminus, a recruitment domain A' and a DNA methylation domain.

10. The complex according to any one of claims 2-4, 6, 7 and 9, wherein the nucleic acid binding domain is a DNA binding domain.

11. The complex according to claim 10, wherein the DNA binding domain is selected from: a TALE domain, a zinc finger domain, a tetR domain, a meganuclease, a Cas protein, an Argonaute (Ago) protein, and a homolog, a modified form or a variant thereof.

12. The complex according to claim 10 or 11, wherein the DNA binding domain is capable of binding to a target sequence of a target locus.

13. The complex according to any one of claims 10-12, wherein the DNA binding domain is capable of binding to a guide RNA.

14. The complex according to claim 13, wherein the guide RNA is capable of specifically recognizing and hybridizing to the target sequence of the target locus.

15. The complex according to any one of claims 10-14, wherein the DNA binding domain is a Cas protein, and the Cas protein is a class II Cas nuclease.

16. The complex according to claim 15, wherein the Cas protein is selected from a class II type II Cas nuclease and a class II type V Cas nuclease.

17. The complex according to claim 15 or 16, wherein the Cas protein is a Cas9 protein or a Cas12 protein.

18. The complex according to any one of claims 15-17, wherein the Cas protein is a dead Cas9 (dCas9) protein or a dead Cas12 (dCas12) protein.

19. The complex according to any one of claims 10-14, wherein the DNA binding domain comprises an amino acid sequence as set forth in any one of SEQ ID Nos: 1-9, 343 and 344.

20. The complex according to any one of claims 1-19, wherein the recruitment domain A is selected from any one of one of the following two sets of domains, and the recruitment domain A' is selected from any one of the other of the following two sets of domains:
1) a general control nonderepressible protein 4 (GCN4), a GFP11 fragment derived from split green fluorescent protein (GFP), or a GVKESLV polypeptide; and
2) a single chain antibody (scFv), a GFP1-10 fragment derived from split green fluorescent protein (GFP), or a PDZ protein domain.

21. The complex according to claim 20, wherein:
1) the domain of one of the recruitment domain A and the recruitment domain A' is a GCN4, and wherein the other domain is a scFv; or
2) the domain of one of the recruitment domain A and the recruitment domain A' is a GFP11 fragment, and wherein the other domain is a GFP1-10; or
3) the domain of one of the recruitment domain A and the recruitment domain A' is a GVKESLV, and wherein the other domain is a PDZ protein domain.

22. The complex according to any one of claims 1-21, wherein the DNA methylation domain comprises at least one DNA methyltransferase or a functionally active fragment thereof.

23. The complex according to claim 22, wherein the DNA methyltransferase is selected from DNMT3A, DNMT3B, DNMT3c, DNMT1, DNMT2 and DNMT3L.

24. The complex according to any one of claims 1-23, wherein the DNA methylation domain comprises at least one DNMT3A and at least one DNMT3L.

25. The complex according to claim 22 or 23, wherein the DNA methyltransferase comprises an amino acid sequence as set forth in any one of SEQ ID Nos: 19-24.

26. The complex according to any one of claims 1-25, wherein the DNA methylation domain comprises a DNMT3A-DNMT3L domain or a DNMT3L-DNMT3A domain; wherein, - indicates that the domains at both ends thereof are directly or indirectly linked in order from N-terminus to C-terminus.

27. The complex according to any one of claims 1-26, wherein the transcriptional repressor is selected from one or more of the following domains: KRAB, ZIM3, ZNF680, ZNF554, ZNF264, ZNF582, ZNF324, ZNF669, ZNF354A, ZNF82, ZNF595, ZNF419, ZNF566, ZIM2, EHMT2, SUV39H1, ZFPM1, TRIM28, EZH2, MXD1, SID, LSD1, HP1a, HDAC3, HDAC1, PRMT1, SETDB1, hSIRT1, ZNF436, ZNF257, ZNF675, ZNF490, ZNF320, ZNF331, ZNF816, ZNF41, ZNF189, ZNF528, ZNF543, ZNF140, ZNF610, ZNF350, ZNF8, ZNF30, ZNF98, ZNF677, ZNF596, ZNF214, ZNF37A, ZNF34, ZNF250, ZNF547, ZNF273, ZFP82, ZNF224, ZNF33A, ZNF45, ZNF175, ZNF184, ZFP28-1, ZFP28-2, ZNF18, ZNF213, ZNF394, ZFP1, ZFP14, ZNF416, ZNF557, ZNF729, ZNF254, ZNF764, ZNF785, ZNF10, CBX5, RYBP, YAF2, MGA, CBX1, SCMH1, MPP8, SUMO3, HERC2, BIN1, PCGF2, TOX, FOXA1, FOXA2, IRF2BP1, IRF2BP2, IRF2BPL IRF-2BP1_2 N-terminal domain, HOXA13, HOXB13, HOXC13, HOXA11, HOXC11, HOXC10, HOXA10, HOXB9, HOXA9, ZFP28, ZN334, ZN568, ZN37A, ZN181, ZN510, ZN862, ZN140, ZN208, ZN248, ZN571, ZN699, ZN726, ZIK1, ZNF2, Z705F, ZNF14, ZN471, ZN624, ZNF84, ZNF7, ZN891, ZN337, Z705G, ZN529, ZN729, ZN419, Z705A, ZN302, ZN486, ZN621, ZN688, ZN33A, ZN554, ZN878, ZN772, ZN224, ZN184, ZN544, ZNF57, ZN283, ZN549, ZN211, ZN615, ZN253, ZN226, ZN730, Z585A, ZN732, ZN681, ZN667, ZN649, ZN470, ZN484, ZN431, ZN382, ZN254, ZN124, ZN607, ZN317, ZN620, ZN141, ZN584, ZN540, ZN75D, ZN555, ZN658, ZN684, RBAK, ZN829, ZN582, ZN112, ZN716, HKR1, ZN350, ZN480, ZN416, ZNF92, ZN100, ZN736, ZNF74, ZN443, ZN195, ZN530, ZN782, ZN791, ZN331, Z354C, ZN157, ZN727, ZN550, ZN793, ZN235, ZN724, ZN573, ZN577, ZN789, ZN718, ZN300, ZN383, ZN429, ZN677, ZN850, ZN454, ZN257, ZN264, ZN485, ZN737, ZNF44, ZN596, ZN565, ZN543, ZFP69, SUMO1, ZNF12, ZN169, ZN433, ZN175, ZN347, ZNF25, ZN519, Z585B, ZN517, ZN846, ZN230, ZNF66, ZN713, ZN816, ZN426, ZN674, ZN627, ZNF20, Z587B, ZN316, ZN233, ZN611, ZN556, ZN234, ZN560, ZNF77, ZN682, ZN614, ZN785, ZN445, ZFP30, ZN225, ZN551, ZN610, ZN528, ZN284, ZN418, ZN490, ZN805, Z780B, ZN763, ZN285, ZNF85, ZN223, ZNF90, ZN557, ZN425, ZN229, ZN606, ZN155, ZN222, ZN442, ZNF91, ZN135, ZN778, ZN534, ZN586, ZN567, ZN440, ZN583, ZN441, ZNF43, ZN589, ZN563, ZN561, ZN136, ZN630, ZN527, ZN333, Z324B, ZN786, ZN709, ZN792, ZN599, ZN613, ZF69B, ZN799, ZN569, ZN564, ZN546, ZFP92, ZN723, ZN439, ZFP57, ZNF19, ZN404, ZN274, CBX3, ZN250, ZN570, ZN675, ZN695, ZN548, ZN132, ZN738, ZN420, ZN626, ZN559, ZN460, ZN268, ZN304, ZN605, ZN844, SUMO5, ZN101, ZN783, ZN417, ZN182, ZN823, ZN177, ZN197, ZN717, ZN669, ZN256, ZN251, CBX4, CDY2, CDYL2, ZN562, ZN461, Z324A, ZN766, ID2, ZN214, CBX7, ID1, CREM, SCX, ASCL1, ZN764, SCML2, TWST1, CREB1, TERF1, ID3, CBX8, GSX1, NKX22, ATF1, TWST2, ZNF17, TOX3, TOX4, ZMYM3, I2BP1, RHXF1, SSX2, I2BPL, ZN680, TRI68, HXA13, PHC3, TCF24, HXB13, HEY1, PHC2, ZNF81, FIGLA, SAM11, KMT2B, HEY2, JDP2, HXC13, ASCL4, HHEX, GSX2, ETV7, ASCL3, PHC1, OTP, I2BP2, VGLL2, HXA11, PDLI4, ASCL2, CDX4, ZN860, LMBL4, PDIP3, NKX25, CEBPB, ISL1, CDX2, PROP1, SIN3B, SMBT1, HXC11, HXC10, PRS6A, VSX1, NKX23, MTG16, HMX3, HMX1, KIF22, CSTF2, CEBPE, DLX2, PPARG, PRIC1, UNC4, BARX2, ALX3, TCF15, TERA, VSX2, HXD12, CDX1, TCF23, ALX1, HXA10, RX, CXXC5, SCML1, NFIL3, DLX6, MTG8, CEBPD, SEC13, FIP1, ALX4, LHX3, PRIC2, MAGI3, NELL1, PRRX1, MTG8R, RAX2, DLX3, DLX1, NKX26, NAB1, SAMD7, PITX3, WDR5, MEOX2, NAB2, DHX8, CBX6, EMX2, CPSF6, HXC12, KDM4B, LMBL3, PHX2A, EMX1, NC2B, DLX4, SRY, ZN777, ZN398, GATA3, BSH, SF3B4, TEAD1, TEAD3, RGAP1, PHF1, GATA2, FOXO3, ZN212, IRX4, ZBED6, LHX4, SIN3A, RBBP7, NKX61, R51A1, MB3L1, DLX5, NOTC1, TERF2, ZN282, RGS12, ZN840, SPI2B, PAX7, NKX62, ASXL2, FOXO1, GATA1, ZMYM5, LRP1, MIXL1, SGT1, LMCD1, CEBPA, SOX14, WTIP, PRP19, NKX11, RBBP4, DMRT2, SMCA2, and functionally active fragments thereof.

28. The complex according to any one of claims 1-27, wherein the transcriptional repressor domain comprises an amino acid sequence as set forth in any one of SEQ ID NOs: 25-50.

29. The complex according to any one of claims 1-28, wherein:
1) the fusion of one of the first fusion and the second fusion comprises a DNA methylation domain-dCas9 or dCas12 or TALE-n×GCN4, and wherein the other fusion comprises a transcriptional repressor domain-scFv; or
2) the fusion of one of the first fusion and the second fusion comprises a DNA methylation domain-dCas9 or dCas12 or TALE-scFv, and wherein the other fusion comprises a transcriptional repressor domain-GCN4; or
3) the fusion of one of the first fusion and the second fusion comprises a DNA methylation domain-dCas9 or dCas12 or TALE-n×GFP11, and wherein the other fusion comprises a transcriptional repressor domain-GFP1-10; or
4) the fusion of one of the first fusion and the second fusion comprises a DNA methylation domain-dCas9 or dCas12 or TALE-GFP1-10, and wherein the other fusion comprises a transcriptional repressor domain-GFP11; or
5) the fusion of one of the first fusion and the second fusion comprises a DNA methylation domain-dCas9 or dCas12 or TALE-n×GCN4, and wherein the other fusion comprises a scFv-transcriptional repressor domain; or
6) the fusion of one of the first fusion and the second fusion comprises a DNA methylation domain-dCas9 or dCas12 or TALE-scFv, and wherein the other fusion comprises a GCN4-transcriptional repressor domain; or
7) the fusion of one of the first fusion and the second fusion comprises a DNA methylation domain-dCas9 or dCas12 or TALE-n×GFP11, and wherein the other fusion comprises a GFP1-10-transcriptional repressor domain; or
8) the fusion of one of the first fusion and the second fusion comprises a DNA methylation domain-dCas9 or dCas12 or TALE-GFP1-10, and wherein the other fusion comprises a GFP11-transcriptional repressor domain;
wherein - indicates that the domains at both ends thereof are directly or indirectly linked in order from N-terminus to C-terminus; n×GCN4 or n×GFP11 denotes respectively n copies of GCN4 or n copies of GFP11 linked by a linker sequence, and n is any integer selected from 1 to 20.

30. The complex according to any one of claims 1-29, wherein the first fusion and/or the second fusion comprises an amino acid sequence as set forth in any one of SEQ ID NOs: 51-76, 78-82, 85-93, 103-105, 110-115, 123, 124, 361 and 362.

31. The complex according to any one of claims 1-30, comprising an amino acid sequence as set forth in any one of SEQ ID NOs: 133-142, 153, 154, 158-163, 168, 345 and 346.

32. The complex according to any one of claims 1-28, wherein:
1) the fusion of one of the first fusion and the second fusion comprises a n×GCN4-dCas9 or dCas12 or TALE-transcriptional repressor domain, and wherein the other fusion comprises a DNA methylation domain-scFv; or
2) the fusion of one of the first fusion and the second fusion comprises a scFv-dCas9 or dCas12 or TALE-transcriptional repressor domain, and wherein the other fusion comprises a DNA methylation domain-GCN4; or
3) the fusion of one of the first fusion and the second fusion comprises a n×GFP11-dCas9 or dCas12 or TALE-transcriptional repressor domain, and wherein the other fusion comprises a DNA methylation domain-GFP1-10; or
4) the fusion of one of the first fusion and the second fusion comprises a GFP1-10-dCas9 or dCas12 or TALE-transcriptional repressor domain, and wherein the other fusion comprises a DNA methylation domain-GFP11; or
5) the fusion of one of the first fusion and the second fusion comprises a n×GCN4-dCas9 or dCas12 or TALE-transcriptional repressor domain, and wherein the other fusion comprises a scFv-DNA methylation domain; or
6) the fusion of one of the first fusion and the second fusion comprises a scFv-dCas9 or dCas12 or TALE-transcriptional repressor domain, and wherein the other fusion comprises a GCN4-DNA methylation domain; or
7) the fusion of one of the first fusion and the second fusion comprises a n×GFP11-dCas9 or dCas12 or TALE-transcriptional repressor domain, and wherein the other fusion comprises a GFP1-10-DNA methylation domain; or
8) the fusion of one of the first fusion and the second fusion comprises a GFP1-10-dCas9 or dCas12 or TALE-transcriptional repressor domain, and wherein the other fusion comprises a GFP11-DNA methylation domain;
wherein - indicates that the domains at both ends thereof are directly or indirectly linked in order from N-terminus to C-terminus; n×GCN4 or n×GFP11 denotes respectively n copies of GCN4 or n copies of GFP11 linked by a linker sequence, and n is any integer selected from 1 to 20.

33. The complex according to any one of claims 1-28 and 32, wherein the first fusion and/or the second fusion comprises an amino acid sequence as set forth in any one of SEQ ID NOs: 83, 84, 94-102, 106-109, 116-122, 363 and 364.

34. The complex according to any one of claims 1-28, 32 and 33, comprising an amino acid sequence as set forth in any one of SEQ ID NOs: 143-152, 155-157, 164-167, 347 and 348.

35. The complex according to any one of claims 1-34, further comprising a nuclear localization signal and/or a label domain.

36. A nucleic acid encoding the complex of any one of claims 1-35.

37. The nucleic acid according to claim 36, which is a recombinant vector.

38. The recombinant vector according to claim 37, further comprising a non-coding region.

39. The non-coding region according to claim 38, which is selected from an intron, a regulatory element, a promoter, an enhancer, a termination sequence, as well as a 5' untranslated region and a 3' untranslated region.

40. The nucleic acid according to any one of claims 36-39, comprising a first nucleic acid fragment encoding the first fusion and a second nucleic acid fragment encoding the second fusion.

41. The nucleic acid according to claim 40, wherein the first nucleic acid fragment and the second nucleic acid fragment are linked by a nucleic acid fragment encoding a cleavage peptide.

42. The nucleic acid according to claim 41, wherein the cleavage peptide is a 2A peptide and/or an IRES.

43. The nucleic acid according to claim 42, wherein the 2A peptide is selected from P2A, T2A, E2A and F2A.

44. The nucleic acid according to any one of claims 36-43, comprising a nucleic acid sequence as set forth in any one of SEQ ID NOs: 169-335 and 349-360.

45. A delivery vector comprising the complex of any one of claims 1-35 and/or the nucleic acid of any one of claims 36-44, and optionally a liposome and/or a lipid nanoparticle.

46. A composition comprising the complex of any one of claims 1-35, the nucleic acid of any one of claims 36-44, and/or the delivery vector of claim 45.

47. A cell comprising the complex of any one of claims 1-35, the nucleic acid of any one of claims 36-44, the delivery vector of claim 45, and/or the composition of claim 46.

48. A kit comprising the complex of any one of claims 1-35, the nucleic acid of any one of claims 36-44, the delivery vector of claim 45, the composition of claim 46, and/or the cell of claim 47.

49. A method for regulating target gene expression, comprising administering the complex of any one of claims 1-35, the nucleic acid of any one of claims 36-44, the delivery vector of claim 45, the composition of claim 46, the cell of claim 47, and/or the kit of claim 48.

50. The method according to claim 49, comprising introducing the complex, the nucleic acid, the delivery vector, the composition, the cell, and/or the kit into a cell containing the target gene.

51. The method according to claim 49, comprising contacting the complex, the nucleic acid, the delivery vector, and/or the composition with a regulatory element near and/or of the target gene.

52. The method according to claim 51, wherein the regulatory element comprises a core promoter, a proximal promoter, a distal enhancer, a silencer, an insulator element, a boundary element, and/or a locus control region.

53. Use of the complex of any one of claims 1-35, the nucleic acid of any one of claims 36-44, the delivery vector of claim 45, the composition of claim 46, the cell of claim 47, and/or the kit of claim 48 in the manufacture of a medicament for treating or alleviating a disease or a disorder thereof associated with abnormal target gene expression and/or abnormal target gene activity.
